# EUROPEAN PATENT APPLICATION

(11) **EP 4 706 683 A2**
(43) Date of publication of application: **11.03.2026**
(21) Application number: 25212849.1
(22) Date of filing: 01.09.2021
(51) Int. Cl.: A61K 47/02

(54) **CHEWABLE FORMULATIONS**

(30) Priority: 03.09.2020 EP 20305978
(62) Divisional of application: 21773481.3
(71) Applicant: Elanco Tiergesundheit AG, 4058 Basel (CH)
(72) Inventor: Chevreau, Clément Maxime, Basel (CH); Grenier, Pascal, Basel (CH); Reitz, Claudia, Basel (CH)
(74) Representative: Greaves Brewster LLP

(57) **Abstract**

Soft chewable dosage forms including an active ingredient, and a carbonate compound as a disintegrant.

## Description

### TECHNICAL FIELD

The present disclosure relates to medicinal chemistry, pharmacology, and veterinary and human medicine. More particularly, the present disclosure relates to chewable compositions, which can be used to deliver one or more agents which are active against parasites or animal diseases.

### BACKGROUND

In the field of medicine, oral administration of medicaments is desirable because it can often be carried out without the involvement of a clinician or veterinarian. Chewable dosage forms are particularly useful to promote human and animal acceptance of a drug product. However, some chewable pharmaceutical products fail to have desirable physicochemical attributes, such as suitable disintegration and active ingredient dissolution. Therefore, while soft chewable formulations are known, there remains a need for soft chewable dosage forms with improved physicochemical attributes.

### BRIEF DESCRIPTION OF THE DRAWINGS

FIG. 1 shows dissolution of lotilaner, milbemycin oxime, and praziquantel from an exemplary chewable composition.
FIG. 2 shows dissolution of lotilaner, moxidectin, and praziquantel from an exemplary chewable composition.
FIG. 3 shows dissolution of lotilaner, moxidectin, praziquantel, and pyrantel pamoate from an exemplary chewable composition.

### DETAILED DESCRIPTION

The present disclosure provides chewable compositions having improved properties as compared to existing compositions. The disclosed soft chewable dosage forms include at least one active ingredient, and a carbonate or bicarbonate compound as a disintegrant. Inclusion of a carbonate or bicarbonate compound unexpectedly promotes effective dosage form disintegration and therapeutically useful dissolution of active ingredients.

Conventional compressed tablets show disintegration when exposed to a dissolution medium. It is common to add disintegrating agents to a tablet formulation in order to enhance drug release. Disintegrants expand and dissolve when wet, and ensure that when the tablet is in contact with physiological media, it rapidly breaks down into smaller fragments, facilitating dissolution. Immediate drug release, caused by fast disintegration of the dosage form tends to be more robust towards changes of the physical properties of the tablet. Most disintegrants are only effective after dry compression, for example during tablet manufacturing.

As soft chewable dosage forms generally are neither produced via compression nor processed under dry conditions, it is challenging to identify an effective disintegrating agent for this type of dosage form. Soft chewable dosage forms are matricial. Final product properties, drug release for example, are strongly determined by the physical properties of the matrix. During exposure to liquid dissolution medium, matricial dosage forms tend to erode over time, characterized by continuous degradation of the surface leading to continuous reduction of the matrix size. Speed of erosion can be influenced by different matrix properties, such as surface porosity, texture, and wettability. Therefore, there is a risk that changes in matrix properties, caused for example by the manufacturing procedure or by ageing over storage, may impact matrix erosion speed and therefore also drug release.

The disclosed soft chewable dosage forms, through a disintegration mechanism facilitated by a carbonate or bicarbonate compound provide benefits over existing soft chewable drug products, such as rapid release, improved drug exposure, and drug release less sensitive to changes in the drug product physical properties. The soft chewable dosage forms are storage stable and robust to manufacturing processes, as well as changes in active ingredients and excipients.

Applicability of sodium bicarbonate as a disintegrant is demonstrated herein for multiple soft chewable dosage forms including different types of active ingredients and excipients. Without wishing to be bound by theory, it is believed the disintegration effect of the carbonate or bicarbonate compound is triggered by the production of carbon dioxide after dosage form contact with acidic dissolution medium, promoting rapid break down of the dosage form into smaller fragments, facilitating dissolution.

Through development of presently disclosed dosage forms, it was discovered that certain variables can effect disintegrant efficiency of the carbonate or bicarbonate compound. These variables include water content in the formulation; lipophilic content in the formulation (e.g., contributed from fat powder); content of carbonate or bicarbonate disintegrant in the formulation; and particle size of the carbonate or bicarbonate compound. Based on the present disclosure, the skilled artisan can readily manage these variables without undue experimentation to produce chewable dosage forms with suitable disintegration and active ingredient dissolution under physiological conditions.

In a preferred embodiment, the present disclosure provides soft chewable dosage forms including a combination of (i) an isoxazoline class parasiticide (e.g., fluralaner, afoxolaner, sarolaner, or lotilaner), (ii) a macrocyclic lactone class parasiticide (e.g., milbemycin oxime, moxidectin, doramectin, or selamectin), and (iii) a pyrazinoisoquinoline class parasiticide (e.g., praziquantel) and/or a tetrahydropyrimidine class parasiticide (e.g., pyrantel pamoate, pyrantel tartrate, morantel tartrate, or oxantel embonate). The present disclosure provides soft chewable dosage forms with the aforementioned actives exhibiting effective dosage form disintegration and therapeutically useful dissolution of these active ingredients. Also provided are soft chewable dosage forms with these actives that are palatable, stable, and processable.

### 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. All publications, patent applications, patents and other references mentioned herein are incorporated by reference in their entirety. The materials, methods, and examples disclosed herein are illustrative only and not intended to be limiting.

The term, "administering to a subject" includes but is not limited to cutaneous, subcutaneous, intramuscular, mucosal, submucosal, transdermal, oral or intranasal administration. Administration could include injection or topical administration.

The term, "chewable" refers to a solid form, which can be taken by mouth and crushed into smaller pieces before swallowing.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "an" and "the" include plural references unless the context clearly dictates otherwise. The present disclosure also contemplates other embodiments "comprising," "consisting of" and "consisting essentially of," the embodiments or elements presented herein, whether explicitly set forth or not.

The term "salt" refers to salts of veterinary or pharmaceutically acceptable organic acids and bases or inorganic acids and bases. Such salts are well known in the art and include those described in Journal of Pharmaceutical Science, 66, 2-19 (1977).

The skilled artisan will appreciate that certain of the compounds of the present invention exist as isomers. All stereoisomers of the compounds of the invention, including geometric isomers, enantiomers, and diastereomers, in any ratio, are contemplated to be within the scope of the present invention.

The skilled artisan will also appreciate that certain of the compounds of the present invention exist as tautomers. All tautomeric forms the compounds of the invention are contemplated to be within the scope of the present invention.

Compounds of the invention also include all isotopic variations, in which at least one atom of the predominant atom mass is replaced by an atom having the same atomic number, but an atomic mass different from the predominant atomic mass. Use of isotopic variations (e.g., deuterium, ²H) may afford greater metabolic stability. Additionally, certain isotopic variations of the compounds of the invention may incorporate a radioactive isotope (e.g., tritium, ³H, or ¹⁴C), which may be useful in drug and/or substrate tissue distribution studies. Substitution with positron emitting isotopes, such as ¹¹C, ¹⁸F, ¹⁵O and ¹³N, may be useful in Positron Emission Topography (PET) studies.

The terms "compounds of the invention" and "a compound of the invention" and "compounds of the present invention" and a like include the embodiment of formula (I) and the other more particular embodiments encompassed by formula (I) described herein and the exemplified compounds described herein and a salt of each of these embodiments.

The terms "treating", "to treat", "treated", or "treatment", include without limitation restraining, slowing, stopping or reversing the progression or severity of an existing symptom or disease.

The terms "control", "controlling" or "controlled" refers to include without limitation decreasing, reducing, or ameliorating the risk of a symptom, disorder, condition, or disease, and protecting an animal from a symptom, disorder, condition, or disease. Controlling may refer to therapeutic, prophylactic, or preventative administration. For example, a larvae or immature heartworm infection would be controlled by acting on the larvae or immature parasite preventing the infection from progressing to an infection by mature parasites.

The term "preventing" refers to the avoidance of a symptom or disease developing in an animal.

The terms "subject" and "patient" refers includes humans and non-human mammalian animals, such as dogs, cats, mice, rats, guinea pigs, rabbits, ferrets, cows, horses, sheep, goats, and pigs. It is understood that a more particular subject is a human. Also, a more particular subject are mammalian pets or companion animals, such as dogs and cats and also mice, guinea pigs, ferrets, and rabbits.

The term "effective amount" refers to an amount which gives the desired benefit to the subject, and includes administration for treatment or control. The amount will vary from one individual subject to another and will depend upon a number of factors, including the overall physical condition of the subject and the severity of the underlying cause of the condition to be treated, concomitant treatments, and the amount of compound of the invention used to maintain desired response at a beneficial level.

An effective amount can be readily determined by the attending diagnostician, as one skilled in the art, by the use of known techniques and by observing results obtained under analogous circumstances. In determining the effective amount, the dose, a number of factors are considered by the attending diagnostician, including, but not limited to: the species of patient; its size, age, and general health; the specific condition, disorder, infection, or disease involved; the degree of or involvement or the severity of the condition, disorder, or disease, the response of the individual patient; the particular compound administered; the mode of administration; the bioavailability characteristics of the preparation administered; the dose regimen selected; the use of concomitant medication; and other relevant circumstances. An effective amount of the present disclosure, the active ingredient treatment dosage, may range from, for example, 0.5 mg to 100 mg. Specific amounts can be determined by the skilled person. Although these dosages are based on a subject having a mass of about 1 kg to about 20 kg, the diagnostician will be able to determine the appropriate dose for a subject whose mass falls outside of this weight range. An effective amount of the present disclosure, the active ingredient treatment dosage, may range from, for example, 0.1 mg to 10 mg/kg of the subject. The dosing regimen is expected to be daily, weekly, or monthly administration.

The phrases "oral bioavailability" and "bioavailability upon oral administration" as used herein refer to the systemic availability (i.e., blood/plasma levels) of a given amount of active administered to a patient.

The term "clearance" as used herein refers to the removal of a substance from the blood, e.g., by renal excretion, expressed in terms of the volume flow of blood or plasma that would contain the amount of substance removed per unit time.

The term "half-life" as used herein refers to the period of time required for one-half of an amount of a substance to be lost through biological processes.

The term "bioavailability" as used herein refers to the physiological availability of a given amount of a drug, as distinct from its chemical potency. The term may also refer to the proportion of the administered dose which is absorbed into the bloodstream.

The term "animal" is used herein to include all vertebrate animals, including humans, companion animals, and livestock animals. It also includes an individual animal in all stages of development, including embryonic and fetal stages. Companion animals include, but are not limited to, dogs and cats. Livestock animals include, but are not limited to, cattle, camelids, pigs, sheep, goats and horses.

The term "parasite" as used herein refers to a pest which lives in or on the host animal and benefits by deriving nutrients at the host animal's expense. The term encompasses all stages in the lifecycle of the pest. An "endoparasite" is a parasite which lives in the host animal. An "ectoparasite" is a parasite which lives on the host animal.

The term "wt/wt" or "w/w" designates weight/weight. The term "% wt/wt" represents the percentage by weight of an ingredient in the recipe of a composition.

The term "w/v" designates weight/volume.

The term "mg/kg" designates milligrams per kilogram of body weight of the subject (e.g., a companion animal, such as a dog or cat).

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

### 2. Formulations

Soft chewable dosage forms of the present disclosure include an active ingredient, and a carbonate or bicarbonate compound. The soft chewable dosage forms optionally include one or more acceptable excipients. The term "acceptable excipient" refers to those typically used in preparing veterinary and pharmaceutical compositions and preferably are pure and non-toxic in the amounts used. They generally are a solid, semi-solid, or liquid material, which in the aggregate can serve as a vehicle or medium for the active ingredient. Some examples of acceptable excipients are found in Remington's Pharmaceutical Sciences and the Handbook of Pharmaceutical Excipients and include diluents, vehicles, carriers, ointment bases, binders, disintegrants, lubricants, glidants, sweetening agents, flavoring agents, gel bases, sustained release matrices, stabilizing agents, preservatives, solvents, suspending agents, buffers, emulsifiers, dyes, propellants, coating agents, and others. It should be understood that in certain embodiments, an excipient can serve multiple functions depending on, for example, the quantity used, the composition makeup, and the manufacture process.

### Active Ingredients

In certain embodiments, the active ingredients include those active against parasites or pests. Such exemplary active ingredients include, but are not limited to, afoxolaner, fluralaner, lotilaner, sarolaner, albendazole, cambendazole, fenbendazole, flubendazole, mebendazole, oxfendazole, parabendazole, tiabendazole, triclabendazole, amitraz, demiditraz, clorsulon, closantel, oxyclonazide, rafoxanide, cyphenothrin, flumethrin, permethrin, cyromazine, derquantel, diamphenetide, dicyclanil, dinotefuran, imidacloprid, nitenpyram, thiamethoxam, abamectin, doramectin, emamectin, eprinomectin, ivermectin, moxidectin, selamectin, milbemycin oxime, emodepside, epsiprantel, fipronil, fluazuron, fluhexafon, indoxacarb, levamisol, lufenuron, metaflumizone, methoprene, monepantel, morantel, niclosamide, nitroscanate, nitroxynil, novaluron, oxantel, praziquantel, pyrantel, pyriprole, pyriproxyfen, sisapronil, spinosad, spinetoram and triflumezopyrim, or a salt of any of the foregoing.

The active agent may act against ecto- and/or endo-parasites. In one embodiment, the chewable composition comprises a compound active against ectoparasites. In another embodiment, the chewable composition comprises a compound active against endoparasites. In a further embodiment, the chewable composition comprises a combination of at least one compound active against ectoparasites and at least one compound active against endoparasites.

The active agent may be an acaricide and may be, for example, selected from: acaricides such as abamectin, doramectin, eprinomectin, ivermectin, milbemectin, nikkomycins, selamectin, tetranactin, and thuringiensin; bridged diphenyl acaricides such as azobenzene, benzoximate, benzyl benzoate, bromopropylate, chlorbenside, chlorfenethol, chlorfenson, chlorfensulphide, chlorobenzilate, chloropropylate, dicofol, diphenyl sulfone, dofenapyn, fenson, fentrifanil, fluorbenside, proclonol, tetradifon, and tetrasul; carbamate acaricides such as benomyl, carbanolate, carbaryl, carbofuran, fenothiocarb, methiocarb, metolcarb, promacyl, and propoxur; oxime carbamate acaricides such as aldicarb, butocarboxim, oxamyl, thiocarboxime, and thiofanox; dinitrophenol acaricides such as binapacryl, dinex, dinobuton, dinocap, dinocap-4, dinocap-6, dinocton, dinopenton, dinosulfon, dinoterbon, and DNOC (4,6-dinitro-o-cresol); formamidine acaricides such as amitraz, chlordimeform, chloromebuform, formetanate, and formparanate; mite growth regulators such as clofentezine, dofenapyn, fluazuron, flubenzimine, flucycloxuron, flufenoxuron, and hexythiazox; organochlorine acaricides such as bromocyclen, camphechlor, dienochlor, and endosulfan; organotin acaricides such as azocyclotin, cyhexatin, and fenbutatin oxide; pyrazole acaricides such as acetoprole, fipronil, tebufenpyrad, and vaniliprole; pyrethroid acaricides including: pyrethroid ester acaricides like acrinathrin, bifenthrin, cyhalothrin, cypermethrin, alpha-cypermethrin, fenpropathrin, fenvalerate, flucythrinate, flumethrin, fluvalinate, tau-fluvalinate, and permethrin, and pyrethroid ether acaricides like halfenprox; quinoxaline acaricides such as chinomethionat and thioquinox; sulfite ester acaricides such as propargite; tetronic acid acaricides such as spirodiclofen; and form unclassified acaricides such acequinocyl, amidoflumet, arsenous oxide, chloromethiuron, closantel, crotamiton, diafenthiuron, dichlofluanid, disulfiram, fenazaflor, fenazaquin, fenpyroximate, fluacrypyrim, fluenetil, mesulfen, MNAF (2-fluoro-N-methyl-N-1-naphthylacetamide), nifluridide, pyridaben, pyrimidifen, sulfiram, sulfluramid, sulfur and triarathene.

The active agent may be an insecticide, acting either as an adulticide or an insect growth regulator (IGR) and may, for example, be selected from different chemical classes such as chlorinated hydrocarbons, organophosphates, carbamates, pyrethroids, formamidines, borates, phenylpyrazoles, and macrocyclic lactones. Representatives of adulticides include imidacloprid, fenthion, fipronil, allethrin, resmethrin, fenvalerate, permetrin and malathion. Representatives of IGRs include benzoylphenylureas such as diflubenzuron, lufenuron, noviflumuron, hexaflumuron, triflumuron, and teflubenzuron or substances like fenoxycarb, pyriproxifen, methoprene, kinoprene, hydroprene, cyromazine, buprofezin and pymetrozine. Insecticides include those of the neonicotinoid class, for example acetamiprid, clothianidin, dinotefuran, imidacloprid, nitenpyram, thiacloprid and thiamethoxam.

In certain embodiments, the soft chewable dosage form includes an isoxazoline class parasiticide. The isoxazoline class parasiticide may have the formula: 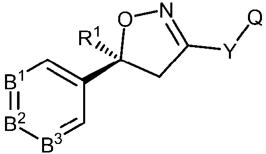 wherein B¹, B², B³, are each independently C-R or N; each R is independently H, halogen, cyano, -NO₂, alkyl, haloalkyl, alkoxy, haloalkoxy, alkylthio, haloalkylthio, alkylsulfinyl, haloalkylsulfinyl, alkylsulfonyl, haloalkylsulfonyl, alkylamino, dialkylamino or alkoxycarbonyl; R¹ is C₁-C₃alkyl or C₁-C₃haloalkyl; Y is an optionally substituted phenylene, naphthylene, indanylene, a 5- or 6-membered heteroarylene or an 8-10-membered fused heterobicyclylene, wherein the optional substituents are selected from the group consisting of halogen, alkyl, haloalkyl, cycloalkyl, halocycloalkyl, alkoxy, haloalkoxy, alkylthio, haloalkylthio, alkylsulfinyl, haloalkylsulfinyl, alkylsulfonyl, haloalkylsulfonyl, alkylamino, dialkylamino, -CN or -NO₂ and NH₂-C(=S)-; Q is T-NR²R³, the group (-CH₂-)(-CH₂-)N-R³, OH, NH₂, alkoxy, haloalkoxy, alkylamino, haloalkylamino, dialkylamino, halodialkylamino, thiol, alkylthio, haloalkylthio, alkylsulfinyl, haloalkylsulfinyl, alkylsulfonyl, haloalkylsulfonyl, or an optionally substituted 5- or 6-membered carbocyclyl, heterocyclyl or heteroaryl ring; T is (CH₂)ₙ, CH(CH₃), CH(CN), C(=O) or C(=S); R² is H, alkyl, alkenyl, alkynyl, cycloalkyl, alkylcycloalkyl, cycloalkylalkyl, alkylcarbonyl or alkoxycarbonyl; R³ is H, OR⁷, NR⁸R⁹ or Q¹; or alkyl, haloalkyl, alkenyl, haloalkenyl, alkynyl, haloalkynyl, cycloalkyl, alkylcycloalkyl, cycloalkylalkyl, alkylcarbonyl, alkoxycarbonyl, aminocarbonyl, alkylaminocarbonyl or dialkylaminocarbonyl, each optionally substituted with one or more substituents independently selected from R⁴; or R² and R³ are taken together with the nitrogen to which they are attached to form a ring containing 2 to 6 atoms of carbon and optionally one additional atom selected from the group consisting of N, S and O, said ring optionally substituted with 1 to 4 substituents independently selected from the group consisting of alkyl, halogen, -CN, -NO₂ and alkoxy; each R⁴ is independently halogen; alkyl, cycloalkyl, alkoxy, alkylthio, haloalkylthio, alkylsulfinyl, haloalkylsulfinyl, alkylsulfonyl, haloalkylsulfonyl, alkylamino, haloalkylamino, dialkylamino, dihaloalkylamino, cycloalkylamino, alkylcarbonyl, alkoxycarbonyl, alkylaminocarbonyl, dialkylaminocarbonyl, haloalkylcarbonyl, haloalkoxycarbonyl, haloalkylaminocarbonyl, dihaloalkylaminocarbonyl, hydroxy, -NH₂, -CN or -NO₂; or Q²; each R⁵ is independently halogen, alkoxy, haloalkoxy, alkylthio, haloalkylthio, alkylsulfinyl, haloalkylsulfinyl, alkylsulfonyl, haloalkylsulfonyl, alkylamino, dialkylamino, alkoxycarbonyl, -CN or -NO₂; each R⁶ is independently halogen, alkyl, haloalkyl, cycloalkyl, halocycloalkyl, alkoxy, haloalkoxy, alkylthio, haloalkylthio, alkylsulfinyl, haloalkylsulfinyl, alkylsulfonyl, haloalkylsulfonyl, alkylamino, dialkylamino, -CN, -NO₂, phenyl or pyridinyl; R⁷ is H; or alkyl, alkenyl, alkynyl, cycloalkyl, alkylcycloalkyl or cycloalkylalkyl, each optionally substituted with one of more halogen; R⁸ is H, alkyl, alkenyl, alkynyl, cycloalkyl, alkylcycloalkyl, cycloalkylalkyl, alkylcarbonyl or alkoxycarbonyl; R⁹ is H; Q³; or alkyl, alkenyl, alkynyl, cycloalkyl, alkylcycloalkyl or cycloalkylalkyl, each optionally substituted with one or more substituents independently selected from R⁴; or R⁸ and R⁹ are taken together with the nitrogen to which they are attached to form a ring containing 2 to 6 atoms of carbon and optionally one additional atom selected from the group consisting of N, S and O, said ring optionally substituted with 1 to 4 substituents independently selected from the group consisting of alkyl, halogen, -CN, -NO₂ and alkoxy; Q¹ is a phenyl ring, a 5- or 6-membered heterocyclic ring, or an 8-, 9- or 10-membered fused bicyclic ring system optionally containing one to three heteroatoms selected from up to 1 O, up to 1 S and up to 3 N, each ring or ring system optionally substituted with one or more substituents independently selected from R⁵; Q² is independently a phenyl ring or a 5- or 6-membered heterocyclic ring, each ring optionally substituted with one or more substituents independently selected from R⁶; Q³ is a phenyl ring or a 5- or 6-membered heterocyclic ring, each ring optionally substituted with one or more substituents independently selected from R⁶; and n is 1, 2 or 3; wherein the asterisk represents that the carbon atom is a quaternary carbon atom. Exemplary isoxazoline class parasiticides include afoxolaner, fluralaner, lotilaner, and sarolaner.

In certain embodiments, the soft chewable dosage form includes an anthelminitic. The anthelmintic may be selected from endo-parasiticides and endecticides including macrocyclic lactones, benzimidazoles, pro-benzimidazoles, imidazothiazoles, tetrahydropyrimidines, organophosphates, piperazines, salicylanilides and cyclic depsipeptides. Exemplary anthelmintics include milbemycins (e.g., milbemycin oxime), avermectins (e.g., ivermectin, selamectin, doramectin, eprinomectin, moxidectin, and abamectin), benzimidazoles (e.g., fenbendazole, albendazole, and triclabendazole), salicylanilides (e.g., closantel and oxyclozanide), substituted phenols (e.g., nitroxynil), tetrahydropyrimidines (e.g., pyrantel, morantel, oxantel), imidazothiazoles (e.g., levamisole), cyclooctadepsipeptide (e.g., emodepside), and pyrazinoisoquinolines (e.g., praziquantel).

In certain embodiments, the soft chewable dosage form includes a macrocyclic lactone class anthelminitic. Macrocyclic lactones include avermectins and derivatives thereof and milbemycins and derivatives thereof. Representatives of macrocyclic lactones include ivermectin, doramectin, moxidectin, selamectin, emamectin, eprinomectin, milbemectin, abamectin, milbemycin oxime, nemadectin, and derivatives thereof, in free form or in the form of a physiologically acceptable salt.

Benzimidazoles, benzimidazole carbamate and pro-benzimidazoles include compounds such as thiabendazole, mebendazole, fenbendazole, oxfendazole, oxibendazole, albendazole, luxabendazole, netobimin, parbendazole, flubendazole, cyclobendazole, febantel and thiophanate. Imidazothiazoles include compounds such as tetramisole and levamisole. Tetrahydropyrimidines include compounds such as morantel and pyrantel. Organophosphates include compounds such as dichlorvos, haloxon and trichlorfon. Piperazines include compounds such as piperazine and derivatives thereof, for example diethylcarbamozine. Salicylanilides include compounds such as closantel, tribromsalan, dibromsalan, oxyclozanide, clioxanide, rafoxanide, brotianide and bromoxanide. Cyclic depsipeptides include compounds consisting of amino acids and hydroxycarboxylic acids as ring structural units and 6 to 30 ring atoms, such as PF1022A and emodepside. Examples of other anthelmintics include clorsulon, oxamniquines, praziquantel and monepantel.

In certain embodiments, the soft chewable dosage form includes an insecticide or acaricide. Exemplary insecticides and acaricides include, for example, acephate, acetamiprid, acetoprole, amitraz, amidoflumet, avermectin, azadirachtin, azinphos-methyl, bifenthrin, bifenazate, buprofezin, bistrifluron, buprofezin, carbofuran, cartap, chlorfenapyr, chlorfluazuron, chlorantraniliprole), chlorpyrifos, chlorpyrifos-methyl, chromafenozide, clothianidin, cyflumetofen, cyfluthiin, β-cyfluthrin, cyhalothrin, γ-cyhalothrin λ-cyhalothrin, cypermethrin, cyromazine, deltamethrin, diafenthiuron, diazinon, dieldrin, diflubenzuron, dimefluthrin, dimethoate, dinotefuran, diofenolan, emamectin, endosulfan, esfenvalerate, ethiprole, fenothiocarb, fenoxycarb, fenpropathrin, fenvalerate, fipronil, flonicamid, flubendiamide, flucythrinate, tau-fluvalinate, flufenerim, flufenoxuron, fonophos, halofenozide, hexaflumuron, hydramethylnon, imidacloprid, indoxacarb, isofenphos, lufenuron, malathion, metaflumizone, metaldehyde, methamidophos, methidathion, methomyl, methoprene, methoxychlor, metofluthrin, monocrotophos, methoxyfenozide, monocrotophos, nitenpyram, nithiazine, novaluron, noviflumuron, óxamyl, parathion, parathiori-methyl, permethrin, phorate, phosalone, phosmet; phosphamidori, pirimicarb, profenofos, profluthrin, protrifenbute, pymetrozine, pyrafluprole, pyrethrin, pyridalyl, pyrifluquinazon, pyriprole, pyriproxyfen, rotenone, ryanodine, spinetoram, spinosad, spirodiclofen, spiromesifen, spirotetramat, sulprofos, tebufenozide, teflubenzuron, tefluthrin, terbufos, tetrachlorvinphos, thiacloprid, thiamethoxam, thiodicarb, thiosultap-sodium, tolfenpyrad, tralomethrin, triazamate, trichlorfon, and triflumuron.

The active agent may also be an antimicrobial active ingredient, for example, various penicillins, tetracyclines, sulfonamides, cephalosporins, cephamycins, aminoglucosids, trimethoprim, dimetridazoles, erythromycin, framycetin, fruazolidone, various pleuromutilins such as thiamulin, valnemulin, various macrolides, streptomycin and substances acting against protozoa, e.g. clopidol, salinomycin, monensin, halofuginone, narasin and robenidine.

In certain embodiments, the soft chewable dosage form includes one or more of the following substances: adrenergic agent; adrenocortical steroid; adrenocortical suppressant; aldosterone; alkylating agent; antagonist; amino acid; anabolic; analeptic; analgesic; analgesic agonists and antagonists; anesthetic; anorexogenic; anti-acne agent; anti-adrenergic; anti-allergic; anti-alopecia agent; anti-amebic; anti-anemic; anti-anginal; antiangiogenic, anti-anxiety; anti-arthritic; anti-asthmatic; anti-atherosclerotic; antibacterial; antibiotic; anticancer; anticholinergic; anticoagulant; anticonvulsant; antidepressant; antidiabetic; antidiarrheal; antidiuretic; anti-dyskinetic; anti-emetic; anti-epileptic; antifibrinolytic; antifungal; anti-hemorrhagic; antihistamine; anti-hypercalcemic, anti-hypercholesterolaemic; anti-hyperlipidaemic; anti-hypertensive; anti-hypertriglyceridemic; anti-hypotensive; anti-infective; anti-inflammatory; anti-ischemic; antimicrobial; antimigraine; antimitotic; antimycotic; anti-nauseant; anti-neoplastic; anti-neutropenic; anti-obesity agent; anti-osteoporotic, antiparasitic; antiproliferative; antipsychotic; antiretroviral; anti-resorptives; anti-rheumatic; anti-seborrheic; antisecretory; antispasmodic; antisclerotic; antithrombotic; antitumor; anti-ulcerative; antiviral; appetite suppressant; bisphosphonate; blood glucose regulator; bronchodilator; cardiovascular agent; central nervous system agent; contraceptive; cholinergic; concentration aid; depressant; diagnostic aid; diuretic; DNA-containing agent, dopaminergic agent; estrogen receptor agonist; fertility agent; fibrinolytic; fluorescent agent; free oxygen radical scavenger; gastric acid suppressant; gastrointestinal motility effector; glucocorticoid; glutamatergic agent; hair growth stimulant; hemostatic; histamine H2 receptor antagonist; hormone; hypo cholesterolemic; hypoglycemic; hypolipidemic; hypotensive; imaging agent; immunizing agent; immunomodulator; immunostimulant; immunosuppressant; interleukin, keratolytic; LHRH agonist; mood regulator; mucolytic; mydriatic; nasal decongestant; neuromuscular blocking agent; neuroprotective; NMDA antagonist; non-hormonal sterol derivative; nootropic agent; opioids; parasympathomimetic agent; plasminogen activator; platelet activating factor antagonist; platelet aggregation inhibitor; platinum-containing agent, psychotropic; radioactive agent; raf antagonist, RNA-containing agent, scabicide; sclerosing agent; sedative; sedative-hypnotic; selective adenosine A1 antagonist; selective estrogen receptor modulator, serotonin antagonist; serotonin inhibitor; serotonin receptor antagonist; steroid; stimulant; thrombic agent; thyroid hormone; thyroid inhibitor; thyromimetic; tranquilizer; vasoconstrictor; vasodilator; wound healing agent; xanthine oxidase inhibitor; and the like.

In certain embodiments, the soft chewable dosage form includes one or more of the following substances: Abacavir, Abacavir sulfate, abatacept, Acarbose, Acetaminophen, Aciclovir, Adalimumab, Adapalene, Alendronate, Alendronate sodium, Alfuzosin, aliskiren, allopurinol, alvimopan, ambrisentan, Aminocaproic acid, Amitriptyline hydrochloride, amlodipine, amlodipine besylate, amoxicillin, amoxicilline, Amphetamine, Anastrozole, Aripiprazole, armodafinil, Atazanavir, atenolol, Atomoxetine, atorvastatin calcium, atorvastatin, Atropine sulfate, Azelastine, azithromycin, Balsalazide, Benazepril, bendamustine hydrochloride, Benzepril hydrochloride, bevacizumab, Bicalutamide, Bimatoprost, Bisoprolol, Bisoprolol fumarate, Bosentan, Botulin toxin, Budesonide, Buformin, Buprenorphine, Bupropion, bupropion hydrobromide, Bupropion Hydrochloride, Cabergoline, Calcipotriol, calcitriol, candesartan cilexetil, Capecitabine, Captopril, carbidopa, carisoprodol, Carvedilol, Caspofungin, Cefdinir, Cefoperazone, Cefotiam, cefprozil, Cefuroxime, Celecoxib, cephalaxin, Certolizumab Pegol, Cetirizine, Cetrizine hydrochloride, Cetuximab, Chlorpromazine hydrochloride, Chlorpheniramine maleate, ciclesonide, Cilastatin, cimetidine, Cinacalcet, Ciprofloxacin, citalopram hydrobromide, Clarithromycin, Clindamycin, Clindamycin, clindamycin hydrochloride, Clomipramine hydrochloride, Clonidine hydrochloride, clopidogrel, Clopidogrel bisulfate, Cloxacillin Sodium, Co-Amoxiclav, Codeine phosphate, Colchicines, Colesevelam, cyclobenzaprine hydrochloride, Cyclophosphamide, Cyclosporine, darbepoetin alfa, Darifenacin, DCRM 197 protein, Desloratadine, desloratidine, Desmopressin sulfate, Desoximetasone, dexamethasone, Diclofenac, Diethylcarbamazine citrate, difluprednate, diphenhydramine, Dipyridamole, DL-methionine, Docetaxel, Donepezil, doripenem, Dorzolamide, Doxazosin, doxazosin mesylate, doxycydine, Drospirenone, Duloxetine, Dutasteride, eculizumab, Efavirenz, Emtricitabine, Enalapril, enalapril maleate, Enoxaparin Sodium, Eprosartan, Erlotinib, Erythromycin, Erythropoetin, Escitalopram, esomeprazole, estradiol, Estrogen, Eszopiclone, etanercept, Ethembutol hydrochloride, Ethosuximide, ethynl estradiol, etonogestrel, etoricoxib, etravirine, Exenatide, Ezetimibe, Ezetimibe, Factor VII, famotidine, Famotidine, Fenofibrate, Fenofibrate, Fentanyl, Fentanyl citrate, Ferrous sulfate, Fexofenadine, fexofenadine hydrochloride, Filgrastim, Finasteride, fluconazole, Fluoxetine hydrochloride, Fluticasone, Fluvastatin, folic acid, Follitropin alfa, Follitropin beta, Formoterol, Fosinopril sodium, Gabapentin, Gabapentin, Gemcitabine, glargine insulin, Glatiramer, glimepride, Goserelin, histrelin acetate, Human growth hormone, Hydralazine hydrochloride, Hydrocodone bitartrate, Hydroxyurea, Hydroxyzine hydrochloride, Ibandronate, Imatinib, Imiglucerase, Imipenem, imiquimod, Indinavir sulfate, infliximab, Interferon beta-1a, Ipratropium, Irbesartan, Irinotecan, Isoniazid, Isosorbide moninitrate, ixabepilone, ketamine, ketoconazole, Ketorolac, Lactobionate, Lamivudine, Lamivudine, Lamotrigine, lanreotide acetate, Lansoprazole, lapatinib, laropiprant, Latanoprost, Letrozole, Leuprolide, Levalbuterol, Levamisole hydrochloride, Levetiracetam, levocetirizine dihydrochloride, levodopa, Levofloxacin, levonorgestrel, Levothyroxine, levothyroxine sodium, Lidocaine, Linezolid, Lisdexamfetamine Dimesylate, Lisinopril, Lispro insulin, Lopinavir, Loratadine, lorazepam, Losartan potassium, maraviroc, Marinol, meclizine hydrochloride, Meloxicam, Memantine, Meropenem, metaxalone, metformin, Metformin Hydrochloride, methadone, methoxy polyethylene glycol-epoetin beta, Methylphenidate, Methylphenidate hydrochloride, Metoprolol, Metoprolol tartrate, metronidazole, Metronidazole, miglitol, Minocycline, Minocycline hydrochloride, mirtazepine, Modafinil, Mometasone, montelukast, Montelukast sodium, Morphine, Moxifloxacin, Mycophenolate mofetil, Naloxone, Naproxen sodium, natalizumab, Neostigmine bromide, Niacin, Nicotinamide, Nifedipine, Nifurtimox, nilotinib hydrochloride monohydrate, nitrofurantoin, Nortriptyline hydrochloride, nystatin, olanzapine, Olanzepine, Olmesartan, olmesartan medoxomil, olopatadine hydrochloride, Omalizumab, Omega-3 acid ethyl esters, Omeprazole, Ondansetron, Opioids, Opoid Antagonists, Orlistat, Oseltamivir, Oxaliplatin, Oxcarbazepine, Oxybytynin chloride, oxycodone hydrochloride, Paclitaxel, Palivizumab, Pantoprazole, paracetamol, Paroxetine, paroxetine hydrochloride, Pegylated interferon alfa-2a, Pemetrexed, Penicillamine, Penicillin V potassium, Phenformin, Phenyloin sodium, Pioglitazone, Piperacillin, Potassium chloride, Pramipexole, Pravastatin, Pravastatin sodium, prednisolone quetiapine fumerate, Pregabalin, Primaquine phosphate, Progesterone, Promethazine, Promethazine hydrochloride, Proponolol hydrochloride, Propoxyphene hydrochloride, pseudoephedrine, Pseudophedrine hydrochloride, Pyridostigmine bromide, Pyridoxine hydrochloride, Quetiapine, quetiapine fumarate, Quinapril hydrochloride, Rabeprazole, raloxifene, raltegravir, Ramipril, Ranitidine, Ranitidine hydrochloride, Recombinant factor VIII, retapamulin, Rimonabant, Risedronate, Risedronate sodium, risperidone, Ritonavir, rituximab, Rivastigmine, rivastigmine tartrate, Rizatriptan, Ropinirole, rosiglitazone, Rosiglitazone maleate, Rosuvastatin, Rotavirus vaccine, rotigotine, Salbutamol, Salbutamol sulfate, salmeterol, sapropterin dihydrochloride, Sertraline, sertraline hydrochloride, Sevelamer, Sevoflurane, Sildenafil, sildenafil citrate, simvastatin, Simvastatin, Sitagliptin, Sodium valproate, Solifenacin, Somatostatin, Somatropin, Stavudine, Sulfomethoxazole, Sumatriptan, Sumatriptan succinate, Tacrolimus, Tadalafil, tamoxifen citrate, Tamsulosin, tamsulosin hydrochloride, Tegaserod, Telmisartan, temazepam, Temozolomide, temsirolimus, Tenofovir, Terazosin Hydrochloride, Terbinafine, Teriparatide, testosterone, Tetracycline hydrochloride, Thalidomide, thymopentin, Timolol meleate, Tiotropium, tipranavir, Tolterodine, tolterodine tartrate, topiramate, topotecan, Tramadol, Tramodol hydrochloride, trastuzumab, trazodone hydrochloride, trimethoprim, Valaciclovir, Valacyclovir hydrochloride, Valproate semisodium, valsartan, Vancomycin, Vardenafil, Varenicline, venlafaxine, Venlafaxine hydrochloride, Verapamil Hydrochloride, vildagliptin, Voglibose, Voriconazole, Wafarin sodium acetylsalicylic acid, Zaleplon, Zidovudine, Ziprasidone, Zoledronate, Zolpidem, or pharmaceutically acceptable salts thereof; 16-alpha fluoroestradiol, 17-alpha dihydroequilenin, 17-alpha estradiol, 17-beta estradiol, 17-hydroxyprogesterone, 1-dodecpyrrolidinone, 22-oxacalcitriol, 3-isobutyl-gammabutyric acid, 6-fluoroursodeoxycholic acid, 7-methoxytacrine, Abacavir, Abacavir sulfate, Abamectin, abanoquil, abatacept, abecarnil, abiraterone, Ablukast, Ablukast Sodium, Acadesine, acamprosate, Acarbose, Acebutolol, Acecamide Hydrochloride, Aceclidine, aceclofenae, Acedapsone, Acedapsone, Aceglutamide Aluminum, Acemannan, Acetaminophen, Acetazolamide, Acetohexamide, Acetohydroxamic Acid, acetomepregenol, Acetophenazine Maleate, Acetosulfone Sodium, Acetylcholine Chloride, Acetylcysteine, acetyl-L-carnitine, acetylmethadol, Aciclovir, Acifran, acipimox, acitemate, Acitretin, Acivicin, Aclarubicin, aclatonium, Acodazole Hydrochloride, aconiazide, Acrisorcin, Acrivastine, Acronine, Actisomide, Actodigin, Acyclovir, acylfulvene, Adatanserin Hydrochloride, adafenoxate, Adalimumab, Adapalene, adatanserin, adecypenol, adecypenol, Adefovir, adelmidrol, ademetionine, Adenosine, Adinazolam, Adipheinine Hydrochloride, adiposin, Adozelesin, adrafinil, Adrenalone, Aiclometasone Dipropionate, airbutamine, alacepril, Alamecin, Alanine, Alaproclate, alaptide, Albendazole, albolabrin, Albuterol, Alclofenae, Alcloxa, aldecalmycin, Aldesleukin, Aldioxa, Aletamine Hydrochloride, Alendronate, Alendronate Sodium, alendronic acid, alentemol, Alentemol Hydrobromide, Aleuronium Chloride, Alexidine, alfacalcidol, Alfentanil Hydrochloride, alfuzosin, Algestone Acetonide, alglucerase, Aliflurane, alinastine, Alipamide, aliskiren, Allantoin, Allobarbital, Allopurinol, Alonimid, alosetron, Alosetron Hydrochloride, Alovudine, Alpertine, alpha-idosone, Alpidem, Alprazolam, Alprenolol Hydrochloride, Alprenoxime Hydrochloride, Alprostadil, Alrestatin Sodium, Altanserin Tartrate, Alteplase, Althiazide, Altretamine, altromycin B, Alverinc Citrate, alvimopan, Alvircept Sudotox, Amadinone Acetate, Amantadine Hydrochloride, ambamustine, Ambomycin, ambrisentan, Ambruticin, Ambuphylline, Ambuside, Amcinafal, Amcinonide, Amdinocillin, Amdinocillin Pivoxil, Amedalin Hydrochloride, amelometasone, Ameltolide, Amesergide, Ametantrone Acetate, amezinium metilsulfate, amfebutamone, Amfenac Sodium, Amfiutizole, Amicycline, Amidephrine Mesylate, amidox, Amifloxacin, amifostine, Amilcacin, Amiloride Hydrochloride, Aminacrine Hydrochloride, Aminobenzoate Potassium, Aminobenzoate Sodium, Aminocaproic Acid, Aminoglutethimide, Aminohippurate Sodium, aminolevulinic acid, Aminophylline, A minorex, Aminosalicylate sodium, Aminosalicylic acid, Amiodarone, Amiprilose Hydrochloride, Amiquinsin Hydrochloride, amisulpride, Amitraz, Amitriptyline Hydrochloride, Amlexanox, amlodipine, amlodipine besylate, Amobarbital Sodium, Amodiaquine, Amodiaquine Hydrochloride, Amorolfine, Amoxapine, Amoxicillin, Amphecloral, Amphetamine, Amphetamine Sulfate, Amphomycin, Amphoterin B, Ampicillin, ampiroxieam, Ampyzine Sulfate, Amquinate, Amrinone, amrubicin, Amsacrine, Amylase, amylin, amythiamicin, Anagestone Acetate, anagrelide, Anakinra, ananain, anaritide, Anaritide Acetate, Anastrozole, Anazolene Sodium, Ancrod, andrographolide, Androstenedione, Angiotensin Amide, Anidoxime, Anileridine, Anilopam Hydrochloride, Aniracetam, Anirolac, Anisotropine Methylbromide, Anistreplase, Anitrazafen, anordrin, antagonist D, antagonist G, antarelix, Antazoline Phosphate, Anthelmycin, Anthralin, Anthramyciantiandrogen, antiestrogen, antineoplaston, Antipyrine, antisense oligonucleotides, apadoline, apafant, Apalcillin Sodium, apaxifylline, Apazone, aphidicolin glycinate, Apixifylline, Apomorphine Hydrochloride, apraclonidine, Apraclonidine Hydrochloride, Apramycin, Aprindine, Aprindine Hydrochloride, aprosulate sodium, Aprotinin, Aptazapine Maleate, aptiganel, apurinic acid, apurinic acid, aranidipine, Aranotin, Arbaprostil, arbekicin, arbidol, Arbutamine Hydrochloride, Arclofenin, Ardeparin Sodium, argatroban, Arginine, Argipressin Tannate, Arildone, Aripiprazole, armodafinil, arotinolol, Arpinocid, Arteflene, Artilide Fumarate, asimadoline, aspalatone, Asparaginase, Aspartic Acid, Aspartocin, asperfuran, Aspirin, aspoxicillin, Asprelin, Astemizole, Astromicin Sulfate, asulacrine, atamestane, Atazanavir, Atenolol, atevirdine, Atipamezole, Atiprosin Maleate, Atolide, Atomoxetine, atorvastatin, Atorvastatin Calcium, Atosiban, Atovaquone, atpenin B, Atracurium Besylate, atrimustine, atrinositol, Atropine, Atropine sulfate, Auranofin, aureobasidin A, Aurothioglucose, Avilamycin, Avoparcin, Avridine, Axid, axinastatin 1, axinastatin 2, axinastatin 3, Azabon, Azacitidinie, Azaclorzine Hydrochloride, Azaconazole, azadirachtine, Azalanstat Dihydrochloride, Azaloxan Fumarate, Azanator Maleate, Azanidazole, Azaperone, Azaribine, Azaserine, azasetron, Azatadine Maleate, Azathioprine, Azathioprine Sodium, azatoxin, azatyrosine, azelaic acid, Azelastine, azelnidipine, Azepindole, Azetepa, azimilide, Azithromycin, Azlocillin, Azolimine, Azosemide, Azotomycin, Aztreonam, Azumolene Sodium, Bacampicillin Hydrochloride, baccatin III, Bacitracin, Baclofen, bacoside A, bacoside B, bactobolamine, balanol, balazipone, balhimycin, balofloxacin, balsalazide, Bambermycins, bambuterol, Bamethan Sulfate, Bamifylline Hydrochloride, Bamidazole, baohuoside 1, Barmastine, barnidipine, Basic, Basifungin, Batanopride Hydrochloride, batebulast, Batelapine Maleate, Batimastat, beau vericin, Becanthone Hydrochloride, becaplermin, becliconazole, Beclomethasone Dipropionate, befloxatone, Beinserazide, Belfosdil, Belladonna, Beloxamide, Bemesetron, Bemitradine, Bemoradan, Benapryzine Hydrochloride, Benazepril, Benazepril Hydrochloride, Benazeprilat, Benda calol Mesylate, bendamustine hydrochloride, Bendazac, Bendroflumethiazide, benflumetol, benidipine, Benorterone, Benoxaprofen, Benoxaprofen, Benoxinate Hydrochloride, Benperidol, Bentazepam, Bentiromide, Benurestat, Benzbromarone, Benzepril hydrochloride, Benzethonium Chloride, Benzetimide Hydrochloride, Benzilonium Bromide, Benzindopyrine Hydrochloride, benzisoxazole, Benzocaine, benzochlorins, Benzoctamine Hydrochloride, Benzodepa, benzoidazoxan, Benzonatate, Benzoyl Peroxide, benzoylstaurosporine, Benzquinamide, Benzthiazide, benztropine, Benztropine Mesylate, Benzydamine Hydrochloride, Benzylpenicilloyl Polylysine, bepridil, Bepridil Hydrochloride, Beractant, Beraprost, Berefrine, berlafenone, bertosamil, Berythromycin, besipirdine, betaalethine, betaclamycin B, Betamethasone, betamipron, betaxolol, Betaxolol Hydrochloride, Bethanechol Chloride, Bethanidine Sulfate, betulinic acid, bevacizumab, bevantolol, Bevantolol Hydrochloride, Bezafibrate, Bialamicol Hydrochloride, Biapenem, Bicalutamide, Bicifadine Hydrochloride, Biclodil Hydrochloride, Bidisomide, bifemelane, Bifonazole, bimakalim, Bimatoprost, bimithil, Bindarit, Biniramycin, binospirone, bioxalomycin, Bipenamol Hydrochloride, Biperiden, Biphenamine Hydrochloride, biriperone, bisantrene, bisaramil, bisaziridinylspermine, bis-benzimidzole A, bis-benzimidazole B, bisnafide, Bisobrin Lactate, Bisoprolol, Bisoprolol fumarate, Bispyrithione Magsulfex, bistramide D, bistramide K, bistratene A, Bithionolate Sodium, Bitolterol Mesylate, Bivalirudin, Bizelesin, Bleomycin Sulfate, boldine, Bolandiol Dipropionate, Bolasterone, Boldenone Undecylenate, Bolenol, Bolmantalate, bopindolol, Bosentan, Botulin toxin, Boxidine, brefeldin, breflate, Brequinar Sodium, Bretazenil, Bretylium Tosylate, Brifentanil Hydrochloride, brimonidine, Brinolase, Brocresine, Brocrinat, Brofoxine, Bromadoline Maleate, Bromazepam, Bromchlorenone, Bromelain, bromfenac, Brominidione, Bromocriptine, Bromodiphenhydramine Hydrochloride, Bromoxanide, Bromperidol, Bromperidol Decanoate, Brompheniramine Maleate, Broperamole, Bropirimine, Brotizolam, Bucamide Maleate, bucindolol, Buclizine Hydrochloride, Bucromar one, Budesonide, budipine, budotitane, Buformin, Bumetanide, Bunaprolast, bunazosin, Bunolol Hydrochloride, Bupicomide, Bupivacaine Hydrochloride, Buprenorphine, Buprenorphine Hydrochloride, Bupropion, bupropion hydrobromide, Bupropion Hydrochloride, Buramate, Buserelin Acetate, Buspirone Hydrochloride, Busulfan, Butabarbital, Butacetin, Butaclamol Hydrochloride, Butalbital, Butamben, Butamirate Citrate, Butaperazine, Butaprost, Butedronate Tetrasodium, butenafine, Buterizine, buthioninc sulfoximine, Butikacin, Butilfenin, Butirosin Sulfate, Butixirate, butixocort propionate, Butoconazole Nitrate, Butonate, Butopamine, Butoprozine Hydrochloride, Butorphanol, Butoxamine Hydrochloride, Butriptyline Hydrochloride, Cabergoline, Cactinomycin, Cadexomer Iodine, Caffeine, calanolide A, Calcifediol, Calcipotriene, calcipotriol, Calcitonin, Calcitriol, Calcium Undecylenate, calphostin C, Calusterone, Cambendazole, Cammonam Sodium, camonagrel, canary pox IL-2, candesartan, candesartan cilexetil, Candicidin, candoxatril, candoxatrilat, Caniglibose, Canrenoate Potassium, Canrenone, capecitabine, Capobenate Sodium, Capobenic Acid, Capreomycin Sulfate, capromab, capsaicin, Captopril, Capuride, Car bocysteine, Caracemide, Carbachol, Carbadox, Carbamazepine, Carbamide Peroxide, Carbantel Lauryl Sulfate, Carbaspirin Calcium, Carbazeran, carbazomycin C, Carbenicillin Potassium, Carbenoxolone Sodium, Carbetimer, carbetocin, Carbidopa, Carbidopa-Levodopa, Carbinoxamine Maleate, Carbiphene Hydrochloride, Carbocloral, Carbol-Fuchsin, Carboplatin, Carboprost, carbovir, carboxamide-amino-triazo-1e, carboxyamidotriazole, carboxymethylated beta-1,3-glucan, Carbuterol Hydrochloride, CaRest M3, Carfentanil Citrate, Carisoprodol, Carmantadine, Carmustine, CARN 700, Carnidazole, Caroxazone, carperitide, Carphenazine Maleate, Carprofen, Carsatrin Succinate, Cartazolate, carteolol, Carteolol Hydrochloride, Carubicin Hydrochloride, carvedilol, carvotroline, Carvotroline Hydrochloride, carzelesin, Caspofungin, castanospermine, caurumonam, cebaracetam, cecropin B, Cedefingol, Cefaclor, Cefadroxil, Cefamandole, Cefaparole, Cefatrizine, Cefazaflur Sodium, Cefazolin, cefcapene pivoxil, cefdaloxime pentexil tosilate, Cefdinir, cefditoren pivoxil, Cefepime, cefetamet, Cefetecol, cefixime, cefluprenam, Cefinenoxime Hydrochloride, Cefinetazole, cefminlox, cefodizime, Cefonicid Sodium, Cefoperazone, Cefoperazone Sodium, Ceforanide, cefoselis, Cefotaxime Sodium, Cefotetan, cefotiam, Cefoxitin, cefozopran, cefpimizole, Cefpiramide, cefpirome, cefpodoxime proxetil, cefprozil, Cefroxadine, cefsulodin, Ceftazidime, cefteram, ceftibuten, Ceftizoxime Sodium, ceftriaxooe, Cefuroxime, celastrol, Celecoxib, celikalim, celiprolol, cepacidiine A, Cephacetrile Sodium, Cephalexin, Cephaloglycin, Cephaloridine, Cephalothin Sodium, Cephapirin Sodium, Cephradine, cericlamine, cerivastatin, Ceruletide, Ceronapril, Certolizumab Pegol, certoparin sodium, Cetaben Sodium, Cetalkonium Chloride, Cetamolol Hydrochloride, Cethuperazone, cetiedil, cetirizine, Cetophenicol, Cetraxate Hydrochloride, Cetrizine hydrochloride, cetrorelix, Cetuximab, Cetylpyridinium Chloride, Chenodiol, Chlophedianol Hydrochloride, Chloral Betaine, Chlorambucil, Chloramphenicol, Chlordantoin, Chlordiazepoxide, Chlorhexidine Gluconate, chlorins, Chlormadinone Acetate, chloroorienticin A, Chloroprocaine Hydrochloride, Chloropropamide, Chloroquine, chloroquinoxaline sulfonamide, Chlorothiazide, Chlorotrianisene, Chloroxine, Chloroxylenol, Chlorpheniramine Maleate, Chlorphenesin Carbamate, Chlorpheniramine maleate, Chlorpromazine, Chlorpromazine hydrochloride, Chlorpropamide, Chlorprothixene, Chlortetracycline Bisulfate, Chlorthalidone, Chlorzoxazone, Cholestyramine Resin, Chromonar Hydrochloride, cibenzoline, cicaprost, Ciclafrine Hydrochloride, Ciclazindol, ciclesonide, cicletanine, Ciclopirox, Cicloprofen, cicloprolol, Cidofovir, Cidoxepin Hydrochloride, Cifenline, Ciglitazone, Ciladopa Hydrochloride, cilansetron, Cilastatin, Cilastatin Sodium, Cilazapril, cilnidipine, Cilobamine Mesylate, cilobradine, Cilofungin, cilostazol, Cimaterol, Cimetidine, cimetropium bromide, Cinacalcet, Cinalukast, Cinanserin Hydrochloride, Cinepazet Maleate, Cinflumide, Cingestol, cinitapride, Cinnamedrine, Cinnarizine, cinolazepam, Cinoxacin, Cinperene, Cinromide, Cintazone, Cintriamide, Cioteronel, Cipamfylline, Ciprefadol Succinate, Ciprocinonide, Ciprofibrate, Ciprofloxacin, ciprostene, Ciramadol, Cirolemycin, Cisplatin, cisapride, cisatracurium besilate, Cisconazole, cis-porphyrin, cistinexine, citalopram, citalopram hydrobromide, Citenamide, citicoline, citreamicin alpha, cladribine, Clamoxyquin Hydrochloride, Clarithromycin, clausenamide, Clavulanate Potassium, Clazolam, Clazolimine, clebopride, Clemastine, Clentiazem Maleate, Clidinium Bromide, clinafloxacin, Clindamycin, clindamycin hydrochloride, Clioquinol, Clioxanide, Cliprofen, clobazam, Clobetasol Propionate, Clobetasone Butyrate, Clocortolone Acetate, Clodanolene, Clodazon Hydrochloride, clodronic acid, Clofazimine, Clofibrate, Clofilium Phosphate, Cloge stone Acetate, Clomacran Phosphate, Clomegestone Acetate, Clometherone, clomethiazole, clomifeneanalogues, Clominorex, Clomiphene, Clomipramine Hydrochloride, Clonazepam, Clonidine, Clonidine hydrochloride, Clonitrate, Clonixeril, Clonixin, Clopamide, Clopenthixol, Cloperidone Hydrochloride, clopidogrel, Clopidogrel bisulfate, Clopimozide, Clopipazan Mesylate, Clopirac, Cloprednol, Cloprostenol Sodium, Clorazepate Dipotassium, Clorethate, Clorexolone, Cloroperone Hydrochloride, Clorprenaline Hydrochloride, Clorsulon, Clortemine Hydrochloride, Closantel, Closiramine Aceturate, Clothiapine, Clothixamide Maleate Cloticasone Propionate, Clotrimazole, Cloxacillin Benzathine, Cloxacillin Sodium, Cloxyquin, Clozapine, Co-Amoxiclav, Cocaine, Coccidioidin, Codeine, Codeine phosphate, Codoxime, Colchicine, Colesevelam, colestimide, Colestipol Hydrochloride, Colestolone, Colforsin, Colfosceril Palmitate, Colistimethate Sodium, Colistin Sulfate, collismycin A, collismycin B, Colterol Mesylate, combretastatin A4, complestatin, conagenin, Conorphone Hydrochloride, contignasterol, contortrostatin, Cormethasone Acetate, Corticorelin Ovine Tnflutate, Corticotropin, Cortisone Acetate, Cortivazol, Cortodoxone, cosalane, costatolide, Cosyntropin, cotinine, Coumadin, Coumermycin, crambescidin, Crilvastatin, crisnatol, Cromitrile Sodium, Cromolyn Sodium, Crotamiton, cryptophycin, cucumariosid, Cuprimyxin, curacin A, curdlan sulfate, curiosin, Cyclacillin, Cyclazocine, cyclazosin, Cyclindole, Cycliramine Maleate, Cyclizine, Cyclobendazole, cyclobenzaprine, cyclobenzaprine hydrochloride, cyclobut A, cyclobut G, cyclocapron, Cycloguanil Pamoate, Cycloheximide, cyclopentanthraquinones, Cyclopenthiazide, Cyclopentolate Hydrochloride, Cyclophenazine Hydrochloride, Cyclophosphamide, cycloplatam, Cyclopropane, Cycloserine, cyclosin, Cyclosporine, cyclothialidine, Cyclothiazide, cyclothiazomycin, Cyheptamide, cypemycin, Cyponamine Hydrochloride, Cyprazepam, Cyproheptadine Hydrochloride, Cyprolidol Hydrochloride, cyproterone, Cyproximide, Cysteamine, Cysteine Hydrochloride, Cystine, Cytarabine, Cytarabine Hydrochloride, cytarabine ocfosfate, cytochalasin B, cytostatin, Dacarbazine, dacliximab, dactimicin, Dactinomycin, daidzein, Daledalin Tosylate, dalfopristin, Dalteparin Sodium, Daltroban, Dalvastatin, danaparoid, Danazol, Dantrolene, daphlnodorin A, dapiprazole, dapitant, Dapoxetine Hydrochloride, Dapsone, Daptomycin, darbepoetin alfa, Darglitazone Sodium, darifenacin, darlucin A, Darodipine, darsidomine, Daunorubicin Hydrochloride, Dazadrol Maleate, Dazepinil Hydrochloride, Dazmegrel, Dazopride Fumarate, Dazoxiben Hydrochloride, DCRM 197 protein, Debrisoquin Sulfate, Decitabine, deferiprone, deflazacort, Dehydrocholic Acid, dehydrodidemnin B, Dehydroepiandrosterone, delapril, Delapril Hydrochloride, Delavirdine Mesylate, delequamine, delfaprazine, Delmadinone Acetate, delmopinol, delphinidin, Demecarium Bromide, Demeclocycline, Demecycline, Demoxepam, Denofungin, deoxypyridinoline, Depakote, deprodone, Deprostil, depsidomycin, deramciclane, dermatan sulfate, Desciclovir, Descinolone Acetonide, Desfurane, Desipramine Hydrochloride, desirudin, Deslanoside, Desloratadine, deslorelin, desmopressin, Desmopressin sulfate, desogestrel, Desonide, Desoximetasone, desoxoamiodarone, Desoxy-corticosterone Acetate, detajmium bitartrate, Deterenol Hydrochloride, Detirelix Acetate, Devazepide, Dexamethasone, Dexamisole, Dexbrompheniramine Maleate, Dexchlorpheniramine Maleate, Dexclamol Hydrochloride, Dexetimide, Dexfenfluramine Hydrochloride, dexifosfamide, Deximafen, dexketoprofen, dexloxiglumide, Dexmedetomidine, Dexormaplatin, Dexoxadrol Hydrochloride, Dexpanthenol, Dexpemedolac, Dexpropranolol Hydrochloride, Dexrazoxane, dexsotalol, dextrin 2-sulphate, Dextroamphetamine, Dextromethorphan, Dextrorphan Hydrochloride, Dextrothyroxine Sodium, dexverapamil, Dezaguanine, dezinamide, dezocine, Diacetolol Hydrochloride, Diamocaine Cyclamate, Diapamide, Diatrizoate Meglumine, Diatrizoic Acid, Diaveridine, Diazepam, Diaziquone, Diazoxide, Dibenzepin Hydrochloride, Dibenzothiophene, Dibucaine, Dichliorvos, Dichloralphenazone, Dichlorphenamide, Dicirenone, Diclofenac, Diclofenac Sodium, Dicloxacillin, dicranin, Dicumarol, Dicyclomine Hydrochloride, Didanosine, didemnin B, didox, Dienestrol, dienogest, Diethylcarbamazine Citrate, diethylhomospermine, diethylnorspermine, Diethylpropion Hydrochloride, Diethylstilbestrol, Difenoximide Hydrochloride, Difenoxin, Diflorasone Diacetate, Difloxacin Hydrochloride, Difluanine Hydrochloride, Diflucortolone, Diflumidone Sodium, Diflunisal, Difluprednate, Diftalone, Digitalis, Digitoxin, Digoxin, Dihexyverine Hydrochloride, dihydrexidine, dihydro-5-azacytidine, Dihydrocodeine Bitartrate, Dihydroergotamine Mesylate, Dihydroestosterone, Dihydrostreptomycin Sulfate, Dihydrotachysterol, Dilantin, Dilevalol Hydrochloride, Diltiazem Hydrochloride, Dimefadane, Dimefline Hydrochloride, Dimenhydrinate, Dimercaprol, Dimethadione, Dimethindene Maleate, Dimethisterone, Dimethyl Sulfoxide, dimethylhomospermine, dimethylprostaglandin A1, dimiracetam, Dimoxamine Hydrochloride, Dinoprost, Dinoprostone, Dioxadrol Hydrochloride, dioxamycin, diphenhydramine, Diphenhydramine Citrate, Diphenidol, Diphenoxylate Hydrochloride, diphenylspiromustine, Dipivefin Hydrochloride, Dipivefrin, dipliencyprone, diprafenone, dipropylnorspermine, Dipyridamole, Dipyrithione, Dipyrone, dirithromycin, discodermolide, Disobutamide, Disofenin, Disopyramide, Disoxaril, disulfuram, Ditekiren, Divalproex Sodium, Dizocilpine Maleate, DL-methionine, Dobutamine, docarpamine, Docebenone, Docetaxel, Doconazole, docosanol, dofetilide, dolasetron, Donepezil, doripenem, Dorzolamide, Doxazosin, doxazosin mesylate, doxycydine, Drospirenone, Duloxetine, Dutasteride, Ebastine, ebiratide, ebrotidine, ebselen, ecabapide, ecabet, ecadotril, ecdisteron, echicetin, echistatin, Echothiophate Iodide, Eclanamine Maleate, Eclazolast, ecomustine, Econazole, ecteinascidin 722, eculizumab, edaravone, Edatrexate, edelfosine, Edifolone Acetate, edobacomab, Edoxudine, edrecolomab, Edrophonium Chloride, edroxyprogesteone Acetate, Efavirenz, efegatran, eflornithine, efonidipine, egualcen, Elantrine, eleatonin, elemene, eletriptan, elgodipine, eliprodil, Elsamitrucin, eltenae, Elucaine, emailcalim, emedastine, Emetine Hydrochloride, emiglitate, Emilium Tosylate, emitefur, emoctakin, Emtricitabine, Enadoline Hydrochloride, Enailciren, enalapril, enalapril maleate, enazadrem, Encyprate, Endralazine Mesylate, Endrysone, Enflurane, englitazone, Enilconazole, Enisoprost, Enlimomab, Enloplatin, Enofelast, Enolicam Sodium, Enoxacin, enoxacin, enoxaparin sodium, Enoxaparin Sodium, Enoximone, Enpiroline Phosphate, Enprofylline, Enpromate, entacapone, enterostatin, Enviradene, Enviroxime, Ephedrine, Epicillin, Epimestrol, Epinephrine, Epinephryl Borate, Epipropidine, Epirizole, epirubicin, Epitetracycline Hydrochloride, Epithiazide, Epoetin Alfa, Epoetin Beta, Epoprostenol, Epoprostenol Sodium, epoxymexrenone, epristeride, Eprosartan, eptastigmine, equilenin, Equilin, Erbulozole, erdosteine, Ergoloid Mesylates, Ergonovine Maleate, Ergotamine Tartrate, Erlotinib, ersentilide, Ersofermin, erythritol, Erythrityl Tetranitrate, Erythromycin, Erythropoetin, Escitalopram, Esmolol Hydrochloride, esomeprazole, Esorubicin Hydrochloride, Esproquin Hydrochloride, Estazolam, Estradiol, Estramustine, Estrazinol Hydrobromide, Estriol, Estrofurate, Estrogen, Estrone, Estropipate, esuprone, Eszopiclone, Etafedrine Hydrochloride, etanercept, Etanidazole, etanterol, Etarotene, Etazolate Hydrochloride, Eterobarb, ethacizin, Ethacrynate Sodium, Ethacrynic Acid, Ethambutol Hydrochloride, Ethamivan, Ethanolamine Oleate, Ethehlorvynol, Ethembutol hydrochloride, Ethinyl estradiol, Ethiodized Oil, Ethionamide, Ethonam Nitrate, Ethopropazine Hydrochloride, Ethosuximide, Ethosuximide, Ethotoin, Ethoxazene Hydrochloride, Ethybenztropine, Ethyl Chloride, Ethyl Dibunate, Ethylestrenol, Ethyndiol, Ethynerone, ethyn1 estradiol, Ethynodiol Diacetate, Etibendazole, Etidocaine, Etidronate Disodium, Etidronic Acid, Etifenin, Etintidine Hydrochloride, etizolam, Etodolac, Etofenamate, Etoformin Hydrochloride, Etomidate, Etonogestrel, Etoperidone Hydrochloride, Etoposide, Etoprine, etoricoxib, Etoxadrol Hydrochloride, Etozolin, etrabamine, etravirine, Etretinate, Etryptamine Acetate, Eucatropine Hydrochloride, Eugenol, Euprocin Hydrochloride, eveminomicin, Exametazime, examorelin, Exaprolol Hydrochloride, exemestane, Exenatide, Ezetimibe, Ezetimibe, Factor VII, fadrozole, faeriefungin, Famciclovir, Famotidine, Fampridine, fantofarone, Fantridone Hydrochloride, faropenem, fasidotril, fasudil, fazarabine, fedotozine, Felbamate, felbamate, Felbinac, Felodipine, Felypressin, Fenalamide, Fenamole, Fenbendazole, Fenbufen, Fencibutirol, Fenclofenac, Fenclonine, Fenclorac, Fendosal, Fenestrel, Fenethylline Hydrochloride, Fenfluramine Hydrochloride, Fengabine, Fenimide, Fenisorex, Fenmetozole Hydrochloride, Fenmetramide, Fenobam, Fenoctimine Sulfate, Fenofibrate, fenoldopam, Fenoprofen, Fenoterol, Fenpipalone, Fenprinast Hydrochloride, Fenprostalene, Fenquizone, fenretinide, fenspiride, fentanyl, Fentanyl Citrate, Fentiazac, Fenticlor, fenticonazole, Fenyripol Hydrochloride, fepradinol, ferpifosate sodium, ferristene, ferrixan, Ferrous Sulfate, Ferumoxides, ferumoxsil, Fetoxylate Hydrochloride, Fexofenadine, fexofenadine hydrochloride, Fezolamine Fumarate, Fiacitabine, Fialuridine, fibmoxef, Fibrinogen, Filgrastim, Filipin, Finasteride, fiorfenicol, fiorifenine, fiosatidil, fiumecinol, fiunarizine, fiuparoxan, fiupirtine, fiurithromycin, fiutrimazole, fiuvastatin, fluvoxamine, Flavodilol Maleate, flavopiridol, Flavoxate Hydrochloride, Flazalone, flecamide, flerobuterol, Fleroxacin, flesinoxan, Flestolol Sulfate, Fletazepam, flezelastine, flobufen, Floctafenine, Flordipine, Flosequinan, Floxacillin, Floxuridine, fluasterone, Fluazacort, Flubanilate Hydrochloride, Flubendazole, Flucindole, Flucloronide, Fluconazole, Flucytosine, Fludalanine, Fludarabine Phosphate, Fludazonium Chloride, Fludeoxyglucose, Fludorex, Fludrocortisone Acetate, Flufenamic Acid, Flufenisal, Flumazenil, Flumequine, Flumeridone, Flumethasone, Flumetramide, Flumezapine, Fluminorex, Flumizole, Flumoxonide, Flunidazole, Flunisolide, Flunitrazepam, Flunixin, fluocalcitriol, Fluocinolone Acetonide, Fluocinonide, Fluocortin Butyl, Fluocortolone, Fluorescein, fluorodaunorunicin hydrochloride, Fluorodopa, Fluorometholone, Fluorouracil, Fluotracen Hydrochloride, Fluoxetine, Fluoxetine hydrochloride, Fluoxymesterone, Fluperamide, Fluperolone Acetate, Fluphenazine Decanoate, Fluprednisolone, Fluproquazone, Fluprostenol Sodium, Fluquazone, Fluradoline Hydrochloride, Flurandrenolide, Flurazepam Hydrochloride, Flurbiprofen, Fluretofen, Fluorocitabine, Fluorofamide, Fluorogestone Acetate, Fluorothyl, Fluoroxene, Fluspiperone, Fluspirilene, Fluticasone, Fluticasone Propionate, Flutroline, Fluvastatin, Fluvastatin Sodium, Fluzinamide, Folic Acid, Follicle regulatory protein, Folliculostatin, Follitropin alfa, Follitropin beta, Fomepizole, Fonazine Mesylate, forasartan, forfenimex, forfenirmex, formestane, Formocortal, formoterol, Fosarilate, Fosazepam, Foscarnet Sodium, fosfomycin, Fosfonet Sodium, fosinopril, Fosinopril sodium, Fosinoprilat, fosphenyloin, Fosquidone, Fostedil, fostriecin, fotemustine, Fuchsin, Fumoxicillin, Fungimycin, Furaprofen, Furazolidone, Furazolium Chloride, Furegrelate Sodium, Furobufen, Furodazole, Furosemide, Fusidate Sodium, Fusidic Acid, Gabapentin, Gadobenate Dimeglumine, gadobenic acid, gadobutrol, Gadodiamide, gadolinium texaphyrin, Gadopentetate Dimegiumine, gadoteric acid, Gadoteridol, Gadoversetamide, galantamine, galdansetron, Galdansetron Hydrochloride, Gallamine Triethiodide, gallium nitrate, gallopamil, galocitabine, Gamfexine, gamolenic acid, Ganciclovir, ganirelix, Gemcadiol, Gemcitabine, Gemeprost, Gemfibrozil, Gentamicin Sulfate, Gentian Violet, gepirone, Gestaclone, Gestodene, Gestonorone Caproate, Gestrinone, Gevotroline Hydrochloride, girisopam, glargine insulin, glaspimod, Glatiramer, glaucocalyxin A, Glemanserin, Gliamilide, Glibornuride, Glicetanile Sodium, Glifiumide, Glimepiride, Glipizide, Gloximonam, Glucagon, glutapyrone, Glutethimide, Glyburide, glycopine, glycopril, Glycopyrrolate, Glyhexamide, Glymidine Sodium, Glyoctamide, Glyparamide, Gold Au-198, Gonadoctrinins, Gonadorelin, Gonadotropins, Goserelin, Gramicidin, Granisetron, grapiprant, grepafloxacin, Griseofulvin, Guaiapate, Guaithylline, Guanabenz, Guanabenz Acetate, Guanadrel Sulfate, Guancydine, Guanethidine Monosulfate, Guanfacine Hydrochloride, Guanisoquin Sulfate, Guanoclor Sulfate, Guanoctine Hydrochloride, Guanoxabenz, Guanoxan Sulfate, Guanoxyfen Sulfate, Gusperimus Trihydrochloride, Halazepam, Halcinonide, halichondrin B, Halobetasol Propionate, halofantrine, Halofantrine Hydrochloride, Halofenate, Halofuginone Hydrobromide, halomon, Halopemide, Haloperidol, halopredone, Haloprogesterone, Haloprogin, Halothane, Halquinols, Hamycin, hatomamicin, hatomarubigin A, hatomarubigin B, hatomarubigin C, hatomarubigin D, Heparin Sodium, hepsulfam, heregulin, Hetacillin, Heterooium Bromide, Hexachlorophene Hydrogen Peroxide, Hexafluorenium Bromide, hexamethylene bisacetamide, Hexedine, Hexobendine, Hexoprenaline Sulfate, Hexylresorcinol, Histamine Phosphate, Histidine, Histoplasmin, Histrelin, histrelin acetate, Homatropine Hydrobromide, Hoquizil Hydrochloride, Human chorionic gonadotropin, Human growth hormone, Hycanthone, Hydralazine Hydrochloride, Hydralazine Polistirex, Hydrochlorothiazide, Hydrocodone Bitartrate, Hydrocortisone, Hydroflumethiazide, Hydromorphone Hydrochloride, Hydroxyamphetamine Hydrobromide, Hydroxychloroquine Sulfate, Hydroxyphenamate, Hydroxyprogesterone Caproate, Hydroxyurea, Hydroxyzine Hydrochloride, Hymecromone, Hyoscyamine, hypericin, Ibafloxacin, Ibandronate, ibogaine, Ibopam, ibudilast, Ibufenac, Ibuprofen, Ibutilide Fumarate, Icatibant Acetate, Ichthammol, Icotidine, idarubicin, idoxifene, Idoxuridine, idramantone, Ifetroban, Ifosfamide, Ilepeimide, illimaquinone, ilmofosin, ilomastat, Ilonidap, iloperidone, iloprost, Imafen Hydrochloride, Imatinib, Imazodan Hydrochloride, imidapril, imidazenil, imidazoacridone, Imidecyl Iodine, Imidocarb Hydrochloride, Imidoline Hydrochloride, Imidurea, Imiglucerase, Imiloxan Hydrochloride, Imipenem, Imipramine Hydrochloride, imiquimod, Impromidine Hydrochloride, Indacrinone, Indapamide, Indecamide Hydrochloride, Indeloxazine Hydrochloride, Indigotindisulfonate Sodium, indinavir, Indinavir sulfate, Indocyanine Green, Indolapril Hydrochloride, Indolidan, indometacin, Indomethacin Sodium, Indoprofen, indoramin, Indorenate Hydrochloride, Indoxole, Indriline Hydrochloride, infliximab, inocoterone, inogatran, inolimomab, Inositol Niacinate, Insulin, Interferon beta-1a, Intrazole, Intriptyline Hydrochloride, iobenguane, Iobenzamic Acid, iobitridol, Iodine, iodoamiloride, iododoxorubicin, iofratol, iomeprol, iopentol, iopromide, iopyrol, iotriside, ioxilan, ipazilide, ipenoxazone, ipidacrine, Ipodate Calcium, ipomeanol, Ipratropium, Ipratropium Bromide, ipriflavone, Iprindole, Iprofenin, Ipronidazole, Iproplatin, Iproxamine Hydrochloride, ipsapirone, irbesartan, irinotecan, irloxacin, iroplact, irsogladin, Irtemazole, isalsteine, Isamoxole, isbogrel, Isepamicin, isobengazole, Isobutamben, Isocarboxazid, Isoconazole, Isoetharine, isofloxythepin, Isoflupredone Acetate, Isoflurane, Isofluorophate, isohomohalicondrin B, Isoleucine, Isomazole Hydrochloride, Isomylamine Hydrochloride, Isoniazid, Isopropamide Iodide, Isopropyl Alcohol, isopropyl unoprostone, Isoproterenol Hydrochloride, Isosorbide, Isosorbide Mononitrate, Isotiquimide, Isotretinoin, Isoxepac, Isoxicam, Isoxsuprine Hydrochloride, isradipine, itameline, itasetron, Itazigrel, itopride, Itraconazole, Ivermectin, ixabepilone, jasplakinolide, Jemefloxacin, Jesopitron, Josamycin, kahalalide F, Kalafungin, Kanamycin Sulfate, ketamine, Ketanserin, Ketazocine, Ketazolam, Kethoxal, Ketipramine Fumarate, Ketoconazole, Ketoprofen, Ketorfanol, ketorolac, Ketotifen Fumarate, Kitasamycin, Labetalol Hydrochloride, Lacidipine, lacidipine, lactitol, lactivicin, Lactobionate, laennec, lafutidine, 1-alphahydroxyvitamin D2, lamellarin-N triacetate, lamifiban, Lamivudine, Lamotrigine, lanoconazole, Lanoxin, lanperisone, lanreotide, lanreotide acetate, Lansoprazole, lapatinib, laropiprant, latanoprost, lateritin, laurocapram, Lauryl Isoquinolinium Bromide, Lavoltidine Succinate, lazabemide, Lecimibide, leinamycin, lemildipine, leminoprazole, lenercept, Leniquinsin, lenograstim, Lenperone, lentinan sulfate, leptin, leptolstatin, lercanidipine, Lergotrile, lerisetron, Letimide Hydrochloride, letrazuril, letrozole, Leucine, leucomyzin, leuprolide, Leuprolide Acetate, leuprorelin, Levalbuterol, Levamfetamine Succinate, levamisole, Levdobutamine Lactobionate, Leveromakalim, levetiracetam, Leveycloserine, levobetaxolol, levobunolol, levobupivacaine, levocabastine, levocarnitine, levocetirizine, levocetirizine dihydrochloride, Levodopa, levodropropizine, levofloxacin, Levofuraltadone, Levoleucovorin Calcium, Levomethadyl Acetate, Levomethadyl Acetate Hydrochloride, levomoprolol, Levonantradol Hydrochloride, Levonordefrin, Levonorgestrel, Levopropoxyphene Napsylate, Levopropylcillin Potassium, levormeloxifene, Levorphanol Tartrate, levosimendan, levosulpiride, Levothyroxine, Levothyroxine Sodium, Levoxadrol Hydrochloride, Lexipafant, Lexithromycin, liarozole, Libenzapril, Lidamidine Hydrochloride, Lidocaine, Lidofenin, Lidoflazine, Lifarizin, Lifibrate, Lifibrol, Linarotene, Lincomycin, Linezolid, Linogliride, Linopirdine, linotroban, linsidomine, lintitript, lintopride, Liothyronine 1-125, liothyronine sodium, Liotrix, lirexapride, Lisdexamfetamine Dimesylate, lisinopril, Lispro insulin, lissoclinamide, Lixazinone Sulfate, lobaplatin, Lobenzarit Sodium, Lobucavir, locarmate Meglumine, locarmic Acid, locetamic Acid, lodamide, Lodelaben, lodipamide Meglumine, lodixanol, Iodoantipyrine I-131, Iodocholesterol I-131, Iodohippurate Sodium 1-131, Iodopyracet 1-125, Iodoquinol, Iodoxamate Meglumine, lodoxamide, Iodoxamie Acid, Lofemizole Hydrochloride, Lofentanil Oxalate, Lofepramine Hydrochloride, lofetamine Hydrochloride I-123, Lofexidine Hydrochloride, loglicic Acid, loglucol, loglucomide, loglycamic Acid, logulamide, lohexyl, lombricine, Lomefloxacin, lomerizine, lomethin I-125, Lometraline Hydrochloride, lometrexol, Lomofungin, Lomoxicam, Lomustine, Lonapalene, lonazolac, lonidamine, lopamidol, lopanoic Acid, Loperamide Hydrochloride, lophendylate, Lopinavir, loprocemic Acid, lopronic Acid, lopydol, lopydone, loracarbef, Lorajmine Hydrochloride, loratadine, Lorazepam, Lorbamate, Lorcamide Hydrochloride, Loreclezole, Loreinadol, lorglumide, Lormetazepam, Lomoxicam, lornoxicam, Lortalamine, Lorzafone, losartan, Losartan potassium, losefamic Acid, loseric Acid, losigamone, losoxantrone, losulamide Meglumine, Losulazine Hydrochloride, losumetic Acid, lotasul, loteprednol, lotetric Acid, lothalamate Sodium, lothalamic Acid, lotrolan, lotroxic Acid, lovastatin, loversol, loviride, loxagiate Sodium, loxaglate Meglumine, loxaglic Acid, Loxapine, Loxoribine, loxotrizoic Acid, lubeluzole, Lucanthone Hydrochloride, Lufironil, Lurosetron Mesylate, lurtotecan, lutetium, Lutrelin Acetate, luzindole, Lyapolate Sodium, Lycetamine, lydicamycin, Lydimycin, Lynestrenol, Lypressin, Lysine, lysofylline, lysostaphin, Maduramicin, Mafenide, magainin 2 amide, Magnesium Salicylate, Magnesium Sulfate, magnolol, maitansine, Malethamer, mallotoaponin, mallotochromene, Malotilate, malotilate, mangafodipir, manidipine, maniwamycin A, Mannitol, mannostatin A, manumycin E, manumycin F, mapinastine, Maprotiline, maraviroc, marimastat, Marinol, Masoprocol, maspin, massetolide, Maytansine, Mazapertine Succiniate, Mazindol, Mebendazole, Mebeverine Hydrochloride, Mebrofenin, Mebutamate, Mecamylamine Hydrochloride, Mechlorethamine Hydrochloride, meclizine hydrochloride, Meclocycline, Meclofenamate Sodium, Mecloqualone, Meclorisone Dibutyrate, Medazepam Hydrochloride, Medorinone, Medrogestone, Medroxalol, Medroxyprogesterone, Medrysone, Meelizine Hydrochloride, Mefenamic Acid, Mefenidil, Mefenorex Hydrochloride, Mefexamide, Mefloquine Hydrochloride, Mefruside, Megalomicin Potassium Phosphate, Megestrol Acetate, Meglumine, Meglutol, Melengestrol Acetate, Meloxicam, Melphalan, Memantine, Memotine Hydrochloride, Menabitan Hydrochloride, Menoctone, menogaril, Menotropins, Meobentine Sulfate, Mepartricin, Mepenzolate Bromide, Meperidine Hydrochloride, Mephentermine Sulfate, Mephenyloin, Mephobarbital, Mepivacaine Hydrochloride, Meprobamate, Meptazinol Hydrochloride, Mequidox, Meralein Sodium, merbarone, Mercaptopurine, Mercufenol Chloride, Merisoprol, Meropenem, Mesalamine, Meseclazone, Mesoridazine, Mesterolone, Mestranol, Mesuprine Hydrochloride, Metalol Hydrochloride, Metaproterenol Polistirex, Metaraminol Bitartrate, Metaxalone, Meteneprost, meterelin, Metformin, Methacholine Chloride, Methacycline, methadone, Methadyl Acetate, Methalthiazide, Methamphetamine Hydrochloride, Methaqualone, Methazolamide, Methdilazine, Methenamine, Methenolone Acetate, Methetoin, Methicillin Sodium, Methimazole, methioninase, Methionine, Methisazone, Methixene Hydrochloride, Methocarbamol, Methohexital Sodium, Methopholine, Methotrexate, Methotrimeprazine, methoxatone, methoxy polyethylene glycol-epoetin beta, Methoxyflurane, Methsuximide, Methyclothiazide, Methyl Palmoxirate, Methylatropine Nitrate, Methylbenzethonium Chloride, Methyldopa, Methyldopate Hydrochloride, Methylene Blue, Methylergonovine Maleate, methylhistamine, methylinosine monophosphate, Methylphenidate, Methylprednisolone, Methyltestosterone, Methynodiol Diacelate, Methysergide, Methysergide Maleate, Metiamide, Metiapine, Metioprim, metipamide, Metipranolol, Metizoline Hydrochloride, Metkephamid Acetate, metoclopramide, Metocurine Iodide, Metogest, Metolazone, Metopimazine, Metoprine, Metoprolol, Metoprolol tartrate, Metouizine, metrifonate, Metrizamide, Metrizoate Sodium, Metronidazole, Meturedepa, Metyrapone, Metyrosine, Mexiletine Hydrochloride, Mexrenoate Potassium, Mezlocillin, Mianserin Hydrochloride, mibefradil, Mibefradil Dihydrochloride, Mibolerone, michellamine B, Miconazole, microcolin A, Midaflur, Midazolam Hydrochloride, midodrine, mifepristone, Mifobate, miglitol, milacemide, milameline, mildronate, Milenperone, Milipertine, milnacipran, Milrinone, miltefosine, Mimbane Hydrochloride, minaprine, Minaxolone, Minocromil, Minocycline, Minocycline hydrochloride, Minoxidil, Mioflazine Hydrochloride, miokamycin, mipragoside, mirfentanil, mirimostim, Mirincamycin Hydrochloride, Mirisetron Maleate, Mirtazapine, Misonidazole, Misoprostol, Mitindomide, Mitocarcin, Mitocromin, Mitogillin, mitoguazone, mitolactol, Mitomalcin, Mitomycin, mitonafide, Mitosper, Mitotane, mitoxantrone, mivacurium chloride, mivazerol, mixanpril, Mixidine, mizolastine, mizoribine, Moclobemide, modafinil, Modaline Sulfate, Modecamide, moexipril, mofarotene, Mofegiline Hydrochloride, mofezolac, molgramostim, Molinazone, Molindone Hydrochloride, Molsidomine, mometasone, Monatepil Maleate, Monensin, Monoctanoin, montelukast, Montelukast Sodium, montirelin, mopidamol, moracizine, Morantel Tartrate, Moricizine, Morniflumate, Morphine, Morrhuate Sodium, mosapramine, mosapride, motilide, Motretinide, Moxalactam Disodium, Moxazocine, Moxifloxacin, moxiraprine, Moxnidazole, moxonidine, Mumps Skin Test Antigen, Muzolimine, mycaperoxide B, Mycophenolate mofetil, Mycophenolic Acid, myriaporone, Nabazenil, Nabilone, Nabitan Hydrochloride, Naboctate Hydrochloride, Nabumetone, N-acetyldinaline, Nadide, nadifloxacin, Nadolol, nadroparin calcium, nafadotride, nafamostat, nafarelin, Nafcillin Sodium, Nafenopin, Nafimidone Hydrochloride, Naflocort, Nafomine Malate, Nafoxidine Hydrochloride, Nafronyl Oxalate, Naftifine Hydrochloride, naftopidil, naglivan, nagrestip, Nalbuphine Hydrochloride, Nalidixate Sodium, Nalidixic Acid, nalmefene, Nalmexone Hydrochloride, naloxone, Naltrexone, Namoxyrate, Nandrolone Phenpropionate, Nantradol Hydrochloride, Napactadine Hydrochloride, napadisilate, Napamezole Hydrochloride, napaviin, Naphazoline Hydrochloride, naphterpin, Naproxen, Naproxen sodium, Naproxol, napsagatran, Naranol Hydrochloride, Narasin, naratriptan, nartograstim, nasaruplase, natalizumab, Natamycin, nateplase, Naxagolide Hydrochloride, Nebivolol, Nebramycin, nedaplatin, Nedocromil, Nefazodone Hydrochloride, Neflumozide Hydrochloride, Nefopam Hydrochloride, Nelezaprine Maleate, Nemazoline Hydrochloride, nemorubicin, Neomycin Palmitate, Neostigmine Bromide, neridronic acid, Netilmicin Sulfate, Neutramycin, Nevirapin Nexeridine Hydrochloride, Niacin, Nibroxane, Nicardipine Hydrochloride, Nicergoline, Niclosamide, Nicorandil, Nicotinamide, Nicotinyl Alcohol, Nifedipine, Nifirmerone, Nifluridide, Nifuradene, Nifuraldezone, Nifuratel, Nifuratrone, Nifurdazil, Nifurimide, Nifurpirinol, Nifurquinazol, Nifurthiazole, Nifurtimox, nilotinib, nilotinib hydrochloride monohydrate, nilutamide, Nilvadipine, Nimazone, Nimodipine, niperotidine, niravoline, Niridazole, nisamycin, Nisbuterol Mesylate, nisin, Nisobamate, Nisoldipine, Nisoxetin Nisterime Acetate, Nitarsone, nitazoxanide, nitecapone, Nitrafudam Hydrochloride, Nitralamine Hydrochloride, Nitramisole Hydrochloride, Nitrazepam, Nitrendipine, Nitrocydine, Nitrodan, Nitrofurantoin, Nitrofurazone, Nitroglycerin, Nitromersol, Nitromide, Nitromifene Citrate, Nitrous Oxide, nitroxide antioxidant, nitrullyn, Nivazol, Nivimedone Sodium, Nizatidine, Noberastine, Nocodazole, Nogalamycin, Nolinium Bromide, Nomifensine Maleate, Noracymethadol Hydrochloride, Norbolethone, Norepinephrine Bitartrate, Norethindrone, Norethynodrel, Norfurane, Norfloxacin, Norgestimate, Norgestomet, Norgestrel, Nortriptyline Hydrochloride, Noscapine, Novobiocin Nylestriol, Nystatin, Obidoxime Chloride, Ocaperidone, Ocfentanil Hydrochloride, Ocinaplon, Octanoic Acid, Octazamide, Octenidine Hydrochloride, Octodrine, Octreotide, Octriptyline Phosphate, Ofloxacin, Ofornine, okicenone, Olanzepine, Olmesartan, olmesartan medoxomil, olopatadine, olopatadine hydrochloride, olprinone, olsalazine, Olsalazine Sodium, Olvanil, Omalizumab, Omega-3 acid ethyl esters, omeprazole, onapristone, ondansetron, Ontazolast, Oocyte Opipramol Hydrochloride, oracin, Orconazole Nitrate, Orgotein, Orlislat, Ormaplatin, Ormetoprim, Ornidazole, Orpanoxin, Orphenadrine Citrate, osaterone, Oseltamivir, otenzepad, Oxacillin Sodium, Oxagrelate, oxaliplatin, Oxamarin Hydrochloride, oxamisole, Oxamniquine, oxandrolone, Oxantel Pamoate, Oxaprotiline Hydrochloride, Oxaprozin, Oxarbazole, Oxatomide, oxaunomycin, Oxazepam, oxcarbazepine, Oxendolone, Oxethazaine, Oxetorone Fumarate, Oxfendazole, Oxfenicine, Oxibendazole, oxiconazole, Oxidopamine, Oxidronic Acid, Oxifungin Hydrochloride, Oxilorphan, Oximonam, Oximonam Sodium, Oxiperomide, oxiracetam, Oxiramide, Oxisuran, Oxmetidine Hydrochloride, oxodipine, Oxogestone Phenopropionate, Oxolinic Acid, Oxprenolol Hydrochloride, Oxtriphylline, Oxybutynin Chloride, Oxychlorosene, Oxycodone, oxycodone hydrochloride, Oxymetazoline Hydrochloride, oxymetholone, Oxymorphone Hydrochloride, Oxypertine, Oxyphenbutazone, Oxypurinol, Oxytetracycline, Oxytocin, ozagrel, Ozlinone, Paclitaxel, palauamine, Paldimycin, palinavir, Palivizumab, palmitoylrhizoxin, Palmoxirate Sodium, pamaqueside, Pamatolol Sulfate, pamicogrel, Pamidronate Disodium, pamidronic acid, Panadiplon, panamesine, panaxytriol, Pancopride, Pancuronium Bromide, panipenem, pannorin, panomifene, pantethine, pantoprazole, Papaverine Hydrochloride, parabactin, paracetamol, Parachlorophenol, Paraldehyde, Paramethasone Acetate, Paranyline Hydrochloride, Parapenzolate Bromide, Pararosaniline Pamoate, Parbendazole, Parconazole Hydrochloride, Paregoric, Pareptide Sulfate, Pargyline Hydrochloride, parnaparin sodium, Paromomycin Sulfate, Paroxetine, paroxetine hydrochloride, parthenolide, Partricin, Paulomycin, pazelliptine, Pazinaclone, Pazoxide, pazufloxacin, pefloxacin, pegaspargase, Pegorgotein, Pegylated interferon alfa-2a, Pelanserin Hydrochloride, peldesine, Peliomycipelretin, Pelrinone Hydrochloride, Pemedolac, Pemerid Nitrate, Pemetrexed, pemirolast, Pemoline, Penamecillin, Penbutolol Sulfate, Penciclovir, Penfluridol, Penicillamine, Penicillin G Benzathine, Penicillin G Potassium, Penicillin G Procaine, Penicillin G Sodium, Penicillin V Hydrabamine, Penicillin V Benzathine, Penicillin V Potassium, Pentabamate, Pentaerythritol Tetranitrate, pentafuside, pentamidine, pentamorphone, Pentamustine, Pentapiperium Methylsulfate, Pentazocine, Pentetic Acid, Pentiapine Maleate, pentigetide, Pentisomicin, Pentizidone Sodium, Pentobarbital, Pentomone, Pentopril, pentosan, pentostatin, Pentoxifylline, Pentrinitrol, pentrozole, Peplomycin Sulfate, Pepstatin, perflubron, perfofamide, Perfosfamide, pergolide, Perhexyline Maleate, perillyl alcohol, Perindopril, perindoprilat, Perlapin Permethrin, perospirone, Perphenazine, Phenacemide, phenaridine, phenazinomycin, Phenazopyridine Hydrochloride, Phenbutazone Sodium Glycerate, Phencarbamide, Phencyclidine Hydrochloride, Phendimetrazine Tartrate, Phenelzine Sulfate, Phenformin, Phenmetrazine Hydrochloride, Phenobarbital, Phenoxybenzamine Hydrochloride, Phenprocoumon, phenserine, phensuccinal, Phensuximide, Phentermine, Phentermine Hydrochloride, phentolamine mesilate, Phentoxifylline, Phenyl Aminosalicylate, phenylacetate, Phenylalanine, phenylalanylketoconazole, Phenylbutazone, Phenylephrine Hydrochloride, Phenylpropanolamine Hydrochloride, Phenylpropanolamine Polistirex, Phenyramidol Hydrochloride, Phenyloin, Phenyloin sodium, Physostigmine, picenadol, picibanil, Picotrin Diolamine, picroliv, picumeterol, pidotimod, Pifarnine, Pilocarpine, pilsicamide, pimagedine, Pimetine Hydrochloride, pimilprost, Pimobendan, Pimozide, Pinacidil, Pinadoline, Pindolol, pinnenol, pinocebrin, Pinoxepin Hydrochloride, pioglitazone, Pipamperone, Pipazethate, pipecuronium bromide, Piperacetazine, Piperacillin, Piperacillin Sodium, Piperamide Maleate, piperazine, Pipobroman, Piposulfan, Pipotiazine Palmitate, Pipoxolan Hydrochloride, Piprozolin, Piquindone Hydrochloride, Piquizi1 Hydrochloride, Piracetam, Pirandamine Hydrochloride, pirarubicin, Pirazmonam Sodium, Pirazolac, Pirbenicillin Sodium, Pirbuterol Acetate, Pirenperone, Pirenzepine Hydrochloride, piretanide, Pirfenidone, Piridicillin Sodium, Piridronate Sodium, Piriprost, piritrexim, Pirlimycin Hydrochloride, pirlindole, pirmagrel, Pirmenol Hydrochloride, Pirnabine, Piroctone, Pirodavir, pirodomast, Pirogliride Tartrate, Pirolate, Pirolazamide, Piroxantrone Hydrochloride, Piroxicam, Piroximone, Pirprofen, Pirquinozol, Pirsidomine, Pivampicillin Hydrochloride, Pivopril, Pizotyline, placetin A, Plicamycin, Plomestane, Pobilukast Edamine, Podofilox, Poisonoak Extract, Poldine Methylsulfate, Poliglusam, Polignate Sodium, Polymyxin B Sulfate, Polythiazide, Ponalrestat, Porfimer Sodium, Porfiromycin, Potassium Chloride, Potassium Iodide, Potassium Permanganate, Povidone-Iodine, Practolol, Pralidoxime Chloride, Pramipexole, Pramiracetam Hydrochloride, Pramoxine Hydrochloride, Pranolium Chloride, Pravadoline Maleate, Pravastatin, Pravastatin sodium, Prazepam, Prazosin, Prazosin Hydrochloride, Prednazate, Prednicarbate, Prednimustine, Prednisolone, prednisolone quetiapine fumerate, Prednisone, Prednival, Pregabalin, Pregnenolone Succiniate, Prenalterol Hydrochloride, Prenylamine, Pridefine Hydrochloride, Prifelone, Prilocalne Hydrochloride, Prilosec, Primaquine Phosphate, Primidolol, Primidone, Prinivil, Prinomide Tromethamine, Prinoxodan, pritosufloxacin, Prizidilol Hydrochloride, Proadifen Hydrochloride, Probenecid, Probicromil Calcium, Probucol, Procainamide Hydrochloride, Procaine Hydrochloride, Procarbazine Hydrochloride, Procaterol Hydrochloride, Prochlorperazine, Procinonide, Proclonol, Procyclidine Hydrochloride, Prodilidine Hydrochloride, Prodolic Acid, Profadol Hydrochloride, Progabide, Progesterone, Proglumide, Proinsulin (human), Proline, Prolintane Hydrochloride, Promazine Hydrochloride, Promethazine, Promethazine hydrochloride, Propafenone Hydrochloride, propagermanium, Propanidid, Propantheline Bromide, Proparacaine Hydrochloride, Propatyl Nitrate, propentofylline, Propenzolate Hydrochloride, Propikacin, Propiomazine, Propionic Acid, propionylcarnitine, propiram, propiram, propiverine, Propofol, Proponolol hydrochloride, Propoxycaine Hydrochloride, Propoxyphene Hydrochloride, Propranolol Hydrochloride, Propulsid, propylbis-acridone, Propylhexedrine, Propyliodone, Propylthiouracil, Proquazone, Prorenoate Potassium, Proroxan Hydrochloride, Proscillaridin, Prostalene, prostratin, Protamine Sulfate, protegrin, Protirelin, Protriptyline Hydrochloride, Proxazole, Proxazole Citrate, Proxicromil, Proxorphan Tartrate, prulifloxacin, pseudoephedrine, Pseudophedrine hydrochloride, Puromycin, Pyrabrom, Pyrantel Pamoate, Pyrazinamide, Pyrazofurin, pyrazoloacridine, Pyridostigmine Bromide, Pyridoxine hydrochloride, Pyrilamine Maleate, Pyrimethamine, Pyrinoline, Pyrithione Sodium, Pyrithione Zinc, Pyrovalerone Hydrochloride, Pyroxamine Maleate, Pyrrocaine, Pyrroliphene Hydrochloride, Pyrrolnitrin, Pyrvinium Pamoate, Quadazocine Mesylate, Quazepam, Quazinone, Quazodine, Quazolast, quetiapine, quetiapine fumarate, quiflapon, quinagolide, Quinaldine Blue, quinapril, Quinapril hydrochloride, Quinazosin Hydrochloride, Quinbolone, Quinctolate, Quindecamine Acetate, Quindonium Bromide, Quinelorane Hydrochloride, Quinestrol, Quinfamide, Quingestanol Acetate, Quingestrone, Quinidine Gluconate, Quinielorane Hydrochloride, Quinine Sulfate, Quinpirole Hydrochloride, Quinterenol Sulfate, Quinuclium Bromide, Quinupristin, Quipazine Maleate, Rabeprazole, Rabeprazole Sodium, Racephenicol, Racepinephrine, Rafoxanide, Ralitoline, raloxifene, raltegravir, raltitrexed, ramatroban, Ramipril, Ramoplanin, ramosetron, ranelic acid, Ranimycin, Ranitidine, Ranitidine hydrochloride, ranolazine, Rauwolfia Serpentina, recainam, Recainam Hydrochloride, Reclazepam, Recombinant factor VIII, regavirumab, Regramostim, Relaxin, Relomycin, Remacemide Hydrochloride, Remifentanil Hydrochloride, Remiprostol, Remoxipride, Repirinast, Repromicin, Reproterol Hydrochloride, Reserpine, resinferatoxin, Resorcinol, retapamulin, retelliptine demethylated, reticulon, reviparin sodium, revizinone, rhenium etidronate, rhizoxin, RI retinamide, Ribaminol, Ribavirin, Riboprine, ricasetron, Ridogrel, Rifabutin, Rifametane, Rifamexil, Rifamide, Rifampin, Rifapentine, Rifaximin, rilopirox, Riluzole, rimantadine, Rimcazole Hydrochloride, Rimexolone, Rimiterol Hydrobromide, Rimonabant, rimoprogin, riodipine, Rioprostil, Ripazepam, ripisartan, Risedronate, Risedronate Sodium, risedronic acid, Risocaine, Risotilide Hydrochloride, rispenzepine, Risperdal, Risperidone, Ritanserin, ritipenem, Ritodrine, Ritolukast, ritonavir, rituximab, rivastigmine, rivastigmine tartrate, Rizatriptan, rizatriptan benzoate, Rocastine Hydrochloride, Rocuronium Bromide, Rodocaine, Roflurane, Rogletimide, rohitukine, rokitamycin, Roletamicide, Rolgamidine, Rolicyprine, Rolipram, Rolitetracycline, Rolodine, Romazarit, romurtide, Ronidazole, Ropinirole, Ropitoin Hydrochloride, ropivacaine, Ropizine, roquinimex, Rosaramicin, rosiglitazone, Rosiglitazone maleate, Rosoxacin, Rosuvastatin, Rotavirus vaccine, rotigotine, Rotoxamine, roxaitidine, Roxarsone, roxindole, roxithromycin, rubiginone B1, ruboxyl, rufloxacin, rupatidine, Rutamycin, ruzadolane, Sabeluzole, safingol, safironil, saintopin, salbutamol, Salbutamol sulfate, Salcolex, Salethamide Maleate, Salicyl Alcohol, Salicylamide, Salicylate Meglumine, Salicylic Acid, Salmeterol, Salnacediin, Salsalate, sameridine, sampatrilat, Sancycline, sanfetrinem, Sanguinarium Chloride, Saperconazole, saprisartan, sapropterin, sapropterin dihydrochloride, saquinavir, Sarafloxacin Hydrochloride, Saralasin Acetate, sarcophytol A, sargramostim, Sarmoxicillin, Sarpicillin, sarpogrelate, saruplase, saterinone, satigrel, satumomab pendetide, Scopafungin, Scopolamine Hydrobromide, Scrazaipine Hydrochloride, Secalciferol, Secobarbital, Seelzone, segiline, Seglitide Acetate, Selegiline Hydrochloride, Selenium Sulfide, Selenomethionine Se-75, Selfotel, sematilide, semduramicin, semotiadil, semustine, Sepazonium Chloride, Seperidol Hydrochloride, Seprilose, Seproxetine Hydrochloride, Seractide Acetate, Sergolexole Maleate, Serine, Sermetacin, Sermorelin Acetate, sertaconazole, sertindole, sertraline, sertraline hydrochloride, S-ethynyluracil, setiptiline, Setoperone, Sevelamer, sevirumab, sevoflurane, sezolamide, Sibopirdine, Sibutramine Hydrochloride, Silandrone, Sildenafil, sildenafil citrate, silipide, silteplase, Silver Nitrate, simendan, Simtrazene, Simvastatin, Sincalide, Sinefungin, sinitrodil, sinnabidol, sipatrigine, sirolimus, Sisomicin, Sitagliptin, Sitogluside, sizofuran, sobuzoxane, Sodium Amylosulfate, Sodium Iodide I-123, Sodium Nitroprusside, Sodium Oxybate, sodium phenylacetate, Sodium Salicylate, Sodium valproate, Solifenacin, solverol, Solypertine Tartrate, Somalapor, Somantadine Hydrochloride, somatomedin B, somatomedin C, Somatostatin, somatrem, somatropin, Somenopor, Somidobove, Sorbinil, Sorivudine, sotalol, Soterenol Hydrochloride, Sparfioxacin, Sparfosate Sodium, sparfosic acid, Sparsomy, Sparteine Sulfate, Spectinomycin Hydrochloride, spicamycin D, Spiperone, Spiradoline Mesylate, Spiramycin, Spirapril Hydrochloride, Spiraprilat, Spirogermanium Hydrochloride, Spiromustine, Spironolactone, Spiroplatin, Spiroxasone, splenopentin, spongistatin, Sprodiamide, squalamine, Stallimycin Hydrochloride, Stannous Pyrophosphate, Stannous Sulfur Colloid, Stanozolol, Statolon, staurosporine, stavudine, Steffimycin, Stenbolone Acetate, stepronin, Stilbazium Iodide, Stilonium Iodide, stipiamide, Stiripentol, stobadine, Streptomycin Sulfate, Streptonicozid, Streptonigrin, Streptozocin, Strontium Chloride Sr-89, succibun, Succimer, Succinylcholine Chloride, Sucralfate, Sucrosofate Potassium, Sudoxicam, Sufentanil, Sufotidine, Sulazepam, Sulbactam Pivoxil, Sulconazole Nitrate, Sulfabenz, Sulfabenzamide, Sulfacetamide, Sulfacytine, Sulfadiazine, Sulfadoxine, Sulfalene, Sulfamerazine, Sulfameter, Sulfamethazine, Sulfamethizole, Sulfamethoxazole, Sulfamonomethoxine, Sulfamoxole, Sulfanilate Zinc, Sulfanitran, sulfasalazine, Sulfasomizole, Sulfazamet, Sulfinalol Hydrochloride, sulfinosine, Sulfinpyrazone, Sulfisoxazole, Sulfomethoxazole, Sulfomyxin, Sulfonterol Hydrochloride, sulfoxamine, Sulinldac, Sulmarin, Sulnidazole, Suloctidil, Sulofenur, sulopenem, Suloxifen Oxalate, Sulpiride, Sulprostone, sultamicillin, Sulthiame, sultopride, sulukast, Sumarotene, sumatriptan, Sumatriptan succinate, Suncillin Sodium, Suproclone, Suprofen, suradista, suramin, Surfomer, Suricamide Maleate, Suritozole, Suronacrine Maleate, Suxemerid Sulfate, swainsonine, symakalim, Symclosene, Symetine Hydrochloride, Taciamine Hydrochloride, Tacrine Hydrochloride, Tacrolimus, Tadalafil, Talampicillin Hydrochloride, Taleranol, Talisomycin, tallimustine, Talmetacin, Talniflumate, Talopram Hydrochloride, Talosalate, Tametraline Hydrochloride, Tamoxifen, tamoxifen citrate, Tampramine Fumarate, Tamsulosin, Tamsulosin Hydrochloride, Tandamine Hydrochloride, tandospirone, tapgen, taprostene, Tasosartan, tauromustine, Taxane, Taxoid, Tazadolene Succinate, tazanolast, tazarotene, Tazifylline Hydrochloride, Tazobactam, Tazofelone, Tazolol Hydrochloride, Tebufelone, Tebuquine, Teclozan, Tecogalan Sodium, Teecleukin, Teflurane, Tegafur, Tegaserod, Tegretol, Teicoplanin, telenzepine, tellurapyrylium, telmesteine, telmisartan, Teloxantrone Hydrochloride, Teludipine Hydrochloride, Temafloxacin Hydrochloride, Tematropium Methyl sulfate, Temazepam, Temelastine, temocapril, Temocillin, temoporfin, temozolomide, temsirolimus, Tenidap, Teniposide, Tenofovir, tenosal, tenoxicam, tepirindole, Tepoxalin, Teprotide, terazosin, Terazosin Hydrochloride, Terbinafine, Terbutaline Sulfate, Terconazole, terfenadine, terfiavoxate, terguride, Teriparatide, Teriparatide Acetate, terlakiren, terlipressin, terodiline, Teroxalene Hydrochloride, Teroxirone, tertatolol, Tesicam, Tesimide, Testolactone, Testosterone, Tetracaine, tetrachlorodecaoxide, Tetracycline, Tetracycline hydrochloride, Tetrahydrozoline Hydrochloride, Tetramisole Hydrochloride, Tetrazolast Meglumine, tetrazomine, Tetrofosmin, Tetroquinone, Tetroxoprim, Tetrydamine, thaliblastine, Thalidomide, Theofibrate, Theophylline, Thiabendazole, Thiamiprine, Thiamphenicol, Thiamylal, Thiazesim Hydrochloride, Thiazinamium Chloride, Thiethylperazine, Thiithixene, Thimerfonate Sodium, Thimerosal, thiocoraline, thiofedrine, Thioguanine, thiomarinol, Thiopental Sodium, thioperamide, Thioridazine, Thiotepa, Thiphenamil Hydrochloride, Thiphencillin Potassium, Thiram, Thozalinone, Threonine, Thrombin, thrombopoietin, thymalfasin, thymopentin, thymotrinan, Thyromedan Hydrochloride, Thyroxine, Tiacrilast, Tiacrilast Sodium, tiagabine, Tiamenidine, tianeptine, tiapafant, Tiapamil Hydrochloride, Tiaramide Hydrochloride, Tiazofurin, Tibenelast Sodium, Tibolone, Tibric Acid, Ticabesone Propionate, Ticarbodine, Ticarcillin Cresyl Sodium, Ticlatone, ticlopidine, Ticrynafen, tienoxolol, Tifurac Sodium, Tigemonam Dicholine, Tigestol, Tiletamine Hydrochloride, Tilidine Hydrochloride, tilisolol, tilnoprofenarbamel, Tilorone Hydrochloride, Tiludronate Disodium, tiludronic acid, Timefurone, Timobesone Acetate, Timolol, Timolol meleate, Tinabinol, Timidazole, Tinzaparin Sodium, Tioconazole, Tiodazosin, Tiodonium Chloride, Tioperidone Hydrochloride, Tiopinac, Tiospirone Hydrochloride, Tiotidine, Tiotropium, tiotropium bromide, Tioxidazole, Tipentosin Hydrochloride, tipranavir, Tipredane, Tiprenolol Hydrochloride, Tiprinast Meglumine, Tipropidil Hydrochloride, Tiqueside, Tiquinamide Hydrochloride, tirandalydigin, Tirapazamine, tirilazad, tirofiban, tiropramide, titanocene dichloride, Tixanox, Tixocortol Pivalate, Tizanidine Hydrochloride, Tnmethobenzamide Hydrochloride, Tobramycin, Tocamide, Tocamphyl, Tofenacin Hydrochloride, Tolamolol, Tolazamide, Tolazoline Hydrochloride, Tolbutamide, Tolcapone, Tolciclate, Tolfamide, Tolgabide, Tolimidone, Tolindate, Tolmetin, Tolnaftate, Tolpovidone, Tolpyrramide, Tolrestat, Tolterodine, tolterodine tartrate, Tomelukast, Tomoxetine Hydrochloride, Tonazocine Mesylate, Topiramate, topotecan, Topotecan Hydrochloride, topsentin, Topterone, Toquizine, torasemide, toremifene, Torsemide, Tosifen, Tosufloxacin, totipotent stem cell factor (TSCF), Tracazolate, trafermin, Tralonide, Tramadol, Tramadol Hydrochloride, Tramazoline Hydrochloride, trandolapril, Tranexamic Acid, Tranilast, Transcamide, trastuzumab, traxanox, Trazodone Hydrochloride, Trebenzomine Hydrochloride, Trefentanil Hydrochloride, Treloxinate, Trepipam Maleate, Trestolone Acetate, tretinoin, Triacetin, triacetyluridine, Triafungin, Triamcinolone, Triampyzine Sulfate, Triamterene, Triazolam, Tribenoside, tricaprilin, Tricetamide, Trichlormethiazide, trichohyalin, triciribine, Tricitrates, Triclofenol piperazine, Triclofos Sodium, trientine, Trifenagrel, triflavin, Triflocin, Triflubazam, Triflumidate, Trifluoperazine Hydrochloride, Trifluperidol, Triflupromazine, Triflupromazine Hydrochloride, Trifluridine, Trihexyphenidyl Hydrochloride, Trilostane, Trimazosin Hydrochloride, trimegestone, Trimeprazine Tartrate, Trimethadione, Trimethaphan Camsylate, Trimethoprim, Trimetozine, Trimetrexate, Trimipramine, Trimoprostil, Trimoxamine Hydrochloride, Triolein, Trioxifene Mesylate, Tripamide, Tripelennamine Hydrochloride, Triprolidine Hydrochloride, Triptorelin, Trisulfapyrimidines, Troclosene Potassium, troglitazone, Trolamine, Troleandomycin, trombodipine, trometamol, Tropanserin Hydrochloride, Tropicamide, tropine, tropisetron, trospectomycin, trovafloxacin, trovirdine, Tryptophan, Tuberculin, Tubocurarine Chloride, Tubulozole Hydrochloride, tucarcsol, tulobuterol, turosteride, Tybamate, tylogenin, Tyropanoate Sodium, Tyrosine, Tyrothricin, tyrphostins, ubenimex, Uldazepam, Undecylenic Acid, Uracil Mustard, urapidil, Urea, Uredepa, uridine triphosphate, Urofollitropin, Urokinase, Ursodiol, valaciclovir, Valacyclovir hydrochloride, Valine, Valnoctamide, Valproate semisodium, Valproic Acid, valsartan, vamicamide, vanadeine, Vancomycin, vaminolol, Vapiprost Hydrochloride, Vapreotide, Vardenafil, Varenicline, variolin B, Vasopressin, Vecuronium Bromide, velaresol, Velnacrine Maleate, venlafaxine, Venlafaxine hydrochloride, Veradoline Hydrochloride, veramine, Verapamil Hydrochloride, verdins, Verilopam Hydrochloride, Verlukast, Verofylline, veroxan, verteporfin, Vesnarinone, vexibinol, Vidarabine, vigabatrin, vildagliptin, Viloxazine Hydrochloride, Vinblastine Sulfate, vinburnine citrate, Vincofos, vinconate, Vincristine Sulfate, Vindesine, Vindesine Sulfate, Vinepidine Sulfate, Vinglycinate Sulfate, Vinleurosine Sulfate, vinorelbine, vinpocetine, vintoperol, vinxaltine, Vinzolidine Sulfate, Viprostol, Virginiamycin, Viridofulvin, Viroxime, vitaxin, Voglibose, Volazocine, voriconazole, vorozole, voxergolide, Wafarin, Xamoterol, Xanomeline, Xanoxate Sodium, Xanthinol Niacinate, xemiloflban, Xenalipin, Xenbucin, Xilobam, ximoprofen, Xipamide, Xorphanol Mesylate, Xylamidine Tosylate, Xylazine Hydrochloride, Xylometazoline Hydrochloride, xylose, yangambin, zabicipril, zacopride, zafirlukast, Zalcitabine, Zaleplon, zalospirone, Zaltidine Hydrochloride, zaltoprofen, zanamivir, zankiren, zanoterone, Zantac, Zarirlukast, zatebradine, zatosetron, Zatosetron Maleate, zenarestat, Zenazocine Mesylate, Zeniplatin, Zeranol, Zidometacin, Zidovudine, zifrosilone, Zilantel, zilascorb, zileuton, Zimeldine Hydrochloride, Zinc Undecylenate, Zindotrine, Zinoconazole Hydrochloride, Zinostatin, Zinterol Hydrochloride, Zinviroxime, ziprasidone, Zobolt, Zofenopril Calcium, Zofenoprilat, Zolamine Hydrochloride, Zolazepam Hydrochloride, Zoledronate, Zolertine Hydrochloride, zolmitriptan, zolpidem, Zomepirac Sodium, Zometapine, Zoniclezole Hydrochloride, Zonisamide, zopiclone, Zopolrestat, Zorbamyciin, Zorubicin Hydrochloride, zotepine, Zucapsaicin, and pharmaceutically acceptable salts thereof.

In certain embodiments, the soft chewable dosage form can include active ingredient(s) ranging from 0.005% to 80% (wt/wt), based on total weight of the dosage form. In certain embodiments, the soft chewable dosage form include active ingredient(s) at 1% to 50% (wt/wt), 5% to 50% (wt/wt), 10% to 50% (wt/wt), 15% to 50% (wt/wt/), 20% to 50% (wt/wt), 25% to 50% (wt/wt), 30% to 50% (wt/wt), 35% to 50% (wt/wt), 40% to 50% (wt/wt), 45% to 50% (wt/wt), 1% to 40% (wt/wt), 1% to 35% (wt/wt), 1% to 30% (wt/wt), 1% to 25% (wt/wt), 1% to 20% (wt/wt), 1% to 15% (wt/wt), 1% to 10% (wt/wt), 1% to 5% (wt/wt), 10% to 40% (wt/wt), 10% to 30% (wt/wt), 10% to 25% (wt/wt), 10% to 20% (wt/wt), 10% to 15% (wt/wt), 15% to 30% (wt/wt), 15% to 25% (wt/wt), or 15% to 20% (wt/wt), based on total weight of the dosage form.

In certain embodiments, the soft chewable dosage forms can provide, independently for each active ingredient, a dosage of 0.001 mg to 1000 mg per kg of the subject, preferably 0.01 mg to 100 mg per kg of the subject, more preferably 0.1 mg to 50 mg per kg of the subject, and even more preferably 0.1 mg to 10 mg per kg of the subject.

In certain embodiments, the soft chewable dosage forms include, independently for each active ingredient, 0.01 mg to 1000 mg of active ingredient, preferably 0.1 to 500 mg of active ingredient, more preferably 0.5 mg to 100 mg of active ingredient.

In a preferred embodiment, the soft chewable dosage form includes a combination of active ingredients. In another preferred embodiment, the soft chewable dosage form includes a combination of an isoxazoline class parasiticide (e.g., fluralaner, afoxolaner, sarolaner, or lotilaner), a macrocyclic lactone class parasiticide (e.g., milbemycin oxime, moxidectin, doramectin, or selamectin), and a pyrazinoisoquinoline class parasiticide (e.g., praziquantel). In another preferred embodiment, the soft chewable dosage form includes a combination of an isoxazoline class parasiticide (e.g., fluralaner, afoxolaner, sarolaner, or lotilaner), a macrocyclic lactone class parasiticide (e.g., milbemycin oxime, moxidectin, doramectin, or selamectin), and a tetrahydropyrimidine class parasiticide (e.g., pyrantel pamoate, pyrantel tartrate, morantel tartrate, or oxantel embonate). In another preferred embodiment, the soft chewable dosage form includes a combination of an isoxazoline class parasiticide (e.g., fluralaner, afoxolaner, sarolaner, or lotilaner), a macrocyclic lactone class parasiticide (e.g., milbemycin oxime, moxidectin, or selamectin), a pyrazinoisoquinoline class parasiticide (e.g., praziquantel), and a tetrahydropyrimidine class parasiticide (e.g., pyrantel pamoate, pyrantel tartrate, morantel tartrate, or oxantel embonate).

In yet another preferred embodiment, the soft chewable dosage form includes a combination of lotilaner, moxidectin, and praziquantel. In yet another preferred embodiment, the soft chewable dosage form includes a combination of lotilaner, moxidectin, praziquantel, and pyrantel or a salt thereof. In yet another preferred embodiment, the soft chewable dosage form includes a combination of sarolaner, moxidectin, and pyrantel or a salt thereof (e.g., pyrantel pamoate). In yet another preferred embodiment, the soft chewable dosage form includes a combination of sarolaner and selamectin. In yet another preferred embodiment, the soft chewable dosage form includes a combination of fluralaner and moxidectin. In yet another preferred embodiment, the soft chewable dosage form includes a combination of afoxolaner and milbemycin oxime.

In yet another preferred embodiment, the soft chewable dosage form includes a combination of 0.5% to 25% (wt/wt) of an isoxazoline (e.g., lotilaner, sarolaner, fluralaner, or afoxolaner), 0.005% to 1% (wt/wt) of a macrocyclic lactone (e.g., moxidectin, milbemycin oxime, doramectin, or selamectin), and 1% to 10% (wt/wt) of a pyrazinoisoquinoline (e.g., praziquantel), based on total weight of the dosage form.

In yet another preferred embodiment, the soft chewable dosage form provides a dosage of 1 mg/kg to 100 mg/kg of an isoxazoline (e.g., lotilaner, sarolaner, fluralaner, or afoxolaner), 0.001 mg/kg to 15 mg/kg of a macrocyclic lactone (e.g., moxidectin, milbemycin oxime, doramectin, or selamectin), and 1 mg/kg to 100 mg/kg of a pyrazinoisoquinoline (e.g., praziquantel).

In yet another preferred embodiment, the soft chewable dosage form includes a combination of 0.5% to 25% (wt/wt) of an isoxazoline (e.g., lotilaner, sarolaner, fluralaner, or afoxolaner), 0.005% to 1% (wt/wt) of a macrocyclic lactone (e.g., moxidectin, milbemycin oxime, doramectin, or selamectin), and 5% to 30% (wt/wt) of a tetrahydropyrimidine (e.g., pyrantel pamoate, pyrantel tartrate, morantel tartrate, or oxantel embonate), based on total weight of the dosage form.

In yet another preferred embodiment, the soft chewable dosage form provides a dosage of 1 mg/kg to 100 mg/kg of an isoxazoline (e.g., lotilaner, sarolaner, fluralaner, or afoxolaner), 0.001 mg/kg to 15 mg/kg of a macrocyclic lactone (e.g., moxidectin, milbemycin oxime, doramectin, or selamectin), and 1 mg/kg to 100 mg/kg of a tetrahydropyrimidine (e.g., pyrantel pamoate, pyrantel tartrate, morantel tartrate, or oxantel embonate).

In yet another preferred embodiment, the soft chewable dosage form includes a combination of 0.5% to 25% (wt/wt) of an isoxazoline (e.g., lotilaner, sarolaner, fluralaner, or afoxolaner), 0.005% to 1% (wt/wt) of a macrocyclic lactone (e.g., moxidectin, milbemycin oxime, doramectin, or selamectin), 1% to 10% (wt/wt) of a pyrazinoisoquinoline (e.g., praziquantel), and 5% to 30% (wt/wt) of a tetrahydropyrimidine (e.g., pyrantel pamoate, pyrantel tartrate, morantel tartrate, or oxantel embonate), based on total weight of the dosage form.

In yet another preferred embodiment, the soft chewable dosage form provides a dosage of 1 mg/kg to 100 mg/kg of an isoxazoline (e.g., lotilaner, sarolaner, fluralaner, or afoxolaner), 0.001 mg/kg to 15 mg/kg of a macrocyclic lactone (e.g., moxidectin, milbemycin oxime, doramectin, or selamectin), 1 mg/kg to 100 mg/kg of a pyrazinoisoquinoline (e.g., praziquantel), and 1 mg/kg to 100 mg/kg of a tetrahydropyrimidine (e.g., pyrantel pamoate, pyrantel tartrate, morantel tartrate, or oxantel embonate).

In yet another preferred embodiment, the soft chewable dosage form includes a combination of 0.5% to 25% (wt/wt) an isoxazoline (e.g., lotilaner, sarolaner, fluralaner, or afoxolaner), 0.005% to 1% (wt/wt) milbemycin oxime, and 1% to 10% (wt/wt) praziquantel, based on total weight of the dosage form.

In yet another preferred embodiment, the soft chewable dosage form provides a dosage of 1 mg/kg to 100 mg/kg of an isoxazoline (e.g., lotilaner, sarolaner, fluralaner, or afoxolaner), 0.05 mg/kg to 3 mg/kg of milbemycin oxime, and 1 mg/kg to 30 mg/kg of praziquantel.

In yet another preferred embodiment, the soft chewable dosage form includes a combination of 0.5% to 25% (wt/wt) lotilaner, 0.005% to 1% (wt/wt) milbemycin oxime, and 1% to 10% (wt/wt) praziquantel, based on total weight of the dosage form.

In yet another preferred embodiment, the soft chewable dosage form provides a dosage of 1 mg/kg to 100 mg/kg of lotilaner, 0.05 mg/kg to 3 mg/kg of milbemycin oxime, and 1 mg/kg to 30 mg/kg of praziquantel.

In yet another preferred embodiment, the soft chewable dosage form includes 20-1,000 mg of lotilaner, 0.1-150 mg of milbemycin oxime, and 5-250 mg of praziquantel.

In yet another preferred embodiment, the soft chewable dosage form includes a combination of 0.5% to 25% (wt/wt) an isoxazoline (e.g., lotilaner, sarolaner, fluralaner, or afoxolaner), 0.005% to 1% (wt/wt) moxidectin, and 1% to 10% (wt/wt) praziquantel, based on total weight of the dosage form.

In yet another preferred embodiment, the soft chewable dosage form provides a dosage of 1 mg/kg to 100 mg/kg of an isoxazoline (e.g., lotilaner, sarolaner, fluralaner, or afoxolaner), 0.001 mg/kg to 5 mg/kg of moxidectin, and 1 mg/kg to 30 mg/kg of praziquantel.

In yet another preferred embodiment, the soft chewable dosage form includes a combination of 0.5% to 25% (wt/wt) lotilaner, 0.005% to 1% (wt/wt) moxidectin, and 1% to 10% (wt/wt) praziquantel, based on total weight of the dosage form. In yet another preferred embodiment, the soft chewable dosage form includes a combination of 14-16% (wt/wt) lotilaner, 0.025% to 0.045% (wt/wt) moxidectin, and 3-5% (wt/wt) praziquantel, based on total weight of the dosage form.

In yet another preferred embodiment, the soft chewable dosage form provides a dosage of 1 mg/kg to 100 mg/kg of lotilaner, 0.001 mg/kg to 5 mg/kg of moxidectin, and 1 mg/kg to 30 mg/kg of praziquantel. In yet another preferred embodiment, the soft chewable dosage form provides a dosage of 15 mg/kg to 45 mg/kg of lotilaner, 0.025 mg/kg to 0.50 mg/kg of moxidectin, and 1 mg/kg to 15 mg/kg of praziquantel. In yet another preferred embodiment, the soft chewable dosage form provides a dosage of 20 mg/kg to 40 mg/kg of lotilaner, 0.05 mg/kg to 0.10 mg/kg of moxidectin, and 5 mg/kg to 10 mg/kg of praziquantel. In yet another preferred embodiment, the compositions provide a dosage of 20 mg/kg to 40 mg/kg of lotilaner, 0.02 mg/kg to 0.04 mg/kg of moxidectin, and 5 mg/kg to 10 mg/kg of praziquantel.

In yet another preferred embodiment, the soft chewable dosage form includes 20-1,000 mg of lotilaner, 0.001-200 mg of moxidectin, and 5-250 mg of praziquantel. In yet another preferred embodiment, the soft chewable dosage form includes 55-57 mg of lotilaner, 0.1-0.2 mg of moxidectin, and 13-15 mg of praziquantel. In yet another preferred embodiment, the soft chewable dosage form includes 111-113 mg of lotilaner, 0.2-0.3 mg of moxidectin, and 28-30 mg of praziquantel. In yet another preferred embodiment, the soft chewable dosage form includes 224-226 mg of lotilaner, 0.5-0.6 mg of moxidectin, and 56-58 mg of praziquantel. In yet another preferred embodiment, the soft chewable dosage form includes 449-451 mg of lotilaner, 1.1-1.2 mg of moxidectin, and 113-115 mg of praziquantel. In yet another preferred embodiment, the soft chewable dosage form includes 899-901 mg of lotilaner, 2.2-2.3 mg of moxidectin, and 227-229 mg of praziquantel.

In yet another preferred embodiment, the soft chewable dosage form includes a combination of 0.5% to 25% (wt/wt) an isoxazoline (e.g., lotilaner, sarolaner, fluralaner, or afoxolaner), 0.005% to 1% (wt/wt) moxidectin, and 5% to 30% (wt/wt) pyrantel pamoate, based on total weight of the dosage form.

In yet another preferred embodiment, the soft chewable dosage form provides a dosage of 1 mg/kg to 100 mg/kg of an isoxazoline (e.g., lotilaner, sarolaner, fluralaner, or afoxolaner), 0.001 mg/kg to 5 mg/kg of moxidectin, and 1 mg/kg to 30 mg/kg of pyrantel pamoate.

In yet another preferred embodiment, the soft chewable dosage form includes a combination of 0.5% to 25% (wt/wt) of lotilaner, 0.005% to 1% (wt/wt) moxidectin, and 5% to 30% (wt/wt) pyrantel pamoate, based on total weight of the dosage form.

In yet another preferred embodiment, the soft chewable dosage form provides a dosage of 1 mg/kg to 100 mg/kg of lotilaner, 0.001 mg/kg to 5 mg/kg of moxidectin, and 1 mg/kg to 30 mg/kg of pyrantel pamoate.

In yet another preferred embodiment, the soft chewable dosage form includes 20-1,000 mg of lotilaner, 0.001-200 mg of moxidectin, and 5-250 mg of pyrantel pamoate.

In yet another preferred embodiment, the soft chewable dosage form includes a combination of 0.5% to 25% (wt/wt) of an isoxazoline (e.g., lotilaner, sarolaner, fluralaner, or afoxolaner), 0.005% to 1% (wt/wt) moxidectin, 1% to 10% (wt/wt) praziquantel, and 5% to 30% (wt/wt) pyrantel pamoate, based on total weight of the dosage form.

In yet another preferred embodiment, the soft chewable dosage form provides a dosage of 1 mg/kg to 100 mg/kg of an isoxazoline (e.g., lotilaner, sarolaner, fluralaner, or afoxolaner), 0.001 mg/kg to 5 mg/kg of moxidectin, 1 mg/kg to 30 mg/kg of praziquantel, and 1 mg/kg to 30 mg/kg of pyrantel pamoate.

In yet another preferred embodiment, the soft chewable dosage form includes a combination of 0.5% to 25% (wt/wt) lotilaner, 0.005% to 1% (wt/wt) moxidectin, 1% to 10% (wt/wt) praziquantel, and 5% to 30% (wt/wt) pyrantel pamoate, based on total weight of the dosage form.

In yet another preferred embodiment, the soft chewable dosage form provides a dosage of 1 mg/kg to 100 mg/kg of lotilaner, 0.001 mg/kg to 5 mg/kg of moxidectin, 1 mg/kg to 30 mg/kg of praziquantel, and 1 mg/kg to 30 mg/kg of pyrantel pamoate. In yet another preferred embodiment, the soft chewable dosage form provides a dosage of 15 mg/kg to 45 mg/kg of lotilaner, 0.01 mg/kg to 0.05 mg/kg of moxidectin, 1 mg/kg to 15 mg/kg of praziquantel, and 1 mg/kg to 15 mg/kg of pyrantel pamoate. In yet another preferred embodiment, the soft chewable dosage form provides a dosage of 20 mg/kg to 41 mg/kg of lotilaner, 0.02 mg/kg to 0.041 mg/kg of moxidectin, 5 mg/kg to 10.4 mg/kg of praziquantel, and 5 mg/kg to 10.4 mg/kg of pyrantel pamoate. In yet another preferred embodiment, the soft chewable dosage form provides a dosage of 20 mg/kg to 40 mg/kg of lotilaner, 0.02 mg/kg to 0.04 mg/kg of moxidectin, 5 mg/kg to 10 mg/kg of praziquantel, and 5 mg/kg to 10 mg/kg of pyrantel pamoate.

In yet another preferred embodiment, the soft chewable dosage form provides a dosage of 20.8-28.1 mg/kg of lotilaner, 0.021-0.028 mg/kg moxidectin, 5.28-7.13 mg/kg praziquantel, and 5.28-7.13 mg/kg pyrantel pamoate. In yet another preferred embodiment, the soft chewable dosage form provides a dosage of 20.8-40.2 mg/kg of lotilaner, 0.021-0.040 mg/kg moxidectin, 5.28-10.18 mg/kg praziquantel, and 5.28-10.18 mg/kg pyrantel pamoate. In yet another preferred embodiment, the soft chewable dosage form provides a dosage of 20.0-40.9 mg/kg of lotilaner, 0.020-0.041 mg/kg moxidectin, 5.07-10.36 mg/kg praziquantel, and 5.07-10.36 mg/kg pyrantel pamoate. In yet another preferred embodiment, the soft chewable dosage form provides a dosage of 20-39.8 mg/kg of lotilaner, 0.020-0.040 mg/kg moxidectin, 5.07-10.09 mg/kg praziquantel, and 5.07-10.09 mg/kg pyrantel pamoate. In yet another preferred embodiment, the soft chewable dosage form provides a dosage of 20.8-39.8 mg/kg of lotilaner, 0.020-0.040 mg/kg moxidectin, 5.07-10.09 mg/kg praziquantel, and 5.07-10.09 mg/kg pyrantel pamoate.

In yet another preferred embodiment, the soft chewable dosage form includes 20-1,000 mg of lotilaner, 0.001-200 mg of moxidectin, 5-250 mg of praziquantel, and 5-250 mg of pyrantel pamoate. In yet another preferred embodiment, the soft chewable dosage form includes 55-57 mg of lotilaner, 0.05-0.06 mg of moxidectin, 13-15 mg of praziquantel, and 13-15 mg of pyrantel pamoate. In yet another preferred embodiment, the soft chewable dosage form includes 111-113 mg of lotilaner, 0.1-0.2 mg of moxidectin, 28-30 mg of praziquantel, and 28-30 mg of pyrantel pamoate. In yet another preferred embodiment, the soft chewable dosage form includes 224-226 mg of lotilaner, 0.2-0.3 mg of moxidectin, 56-58 mg of praziquantel, and 56-58 mg of pyrantel pamoate. In yet another preferred embodiment, the soft chewable dosage form includes 449-451 mg of lotilaner, 0.4-0.5 mg of moxidectin, 113-115 mg of praziquantel, and 113-115 mg of pyrantel pamoate. In yet another preferred embodiment, the soft chewable dosage form includes 899-901 mg of lotilaner, 0.85-0.95 mg of moxidectin, 227-229 mg of praziquantel, and 227-229 mg of pyrantel pamoate.

### Protected Active Ingredients

In certain embodiments, one or more actives of the soft chewable dosage forms may be incorporated into the dosage form via an active-coated and/or active-containing substrate. Actives may be incorporated in the dosage forms via a substrate to, for example, taste-mask unpalatable ingredients or shield a labile active ingredient from other actives or excipients in the dosage form matrix.

In preferred embodiments, the substrate is a pellet. One or more actives of the soft chewable dosage forms can be incorporated into the dosage form via an active-coated pellet. Exemplary active-coated pellets include (i) a pellet (e.g., comprised of microcrystalline cellulose), (ii) a first coating over at least a portion of the pellet, said first coating comprising one or more actives (e.g., praziquantel), and optionally (iii) a second coating over at least a portion of the first coating, said second coating comprising a physiologically acceptable polymer (e.g., dimethylaminoethyl methacrylate-copolymer).

Suitable physiologically acceptable carrier materials for producing the pellets include but are not limited to cellulose, starch, saccharose, lactose or other different types of sugar. Exemplary pellets made of microcrystalline cellulose include those commercially available under the Celphere^{®} mark (e.g., Celphere^{®} CP203) or the Cellets^{®} mark (e.g., Cellets^{®} 100 (100 - 200 µm) or Cellets^{®} 200 (200 - 355 µm)).

In order to coat the pellets with active ingredient, actives can be dissolved in a solvent (e.g., water, alcohols, and mixtures thereof) optionally with additional components, and applied to the pellets by a spraying process. Further suitable solvents are known to those skilled in the art, readily volatile solvents are preferred. After the spraying procedure, volatile components may be removed (e.g. under vacuum). After the drying process, the pellets may be further sieved.

The active-coated pellets can be further coated with a secondary layer (e.g., a protective layer) comprising a physiologically acceptable polymer matrix. Suitable classes of polymer include, but are not limited to, shellac, a polymer on a cellulose, acrylic acid or methacrylic acid, maleic acid anhydride, polyvinyl pyrrolidone or polyvinyl alcohol basis. Other polymers may also be considered, e.g. polymers on a cellulose basis, e.g. produced from cellulose acetate phthalate or cellulose acetate-N,N-di-n-butylhydroxypropylether. The starting materials for polymers on an acrylic acid or methacrylic acid basis may be methacrylate/methacrylic acid copolymer, 2-methyl-5-vinylpyridine/methacrylate/methacrylic acid copolymer, methyl methacrylate/methacrylic acid copolymer, methyl methacrylate/methacrylic acid copolymer, methyl methacrylate/maleic acid anhydride copolymer, or methyl methacrylate/maleic acid anhydride copolymer.

Polymers on an acrylic acid or methacrylic acid basis are preferably used, e.g. polymerization products of acrylic acid and acrylic acid esters with a low content of quaternary ammonium groups, e.g. as commercially available under the names Eudragit^{®} E, L or S from the company Röhm, Darmstadt, Germany. Eudragit^{®} E is a cationic polymer of dimethylaminoethyl methacrylate and a neutral methacrylic acid ester. Eudragit^{®} and S are anionic copolymers of methacrylic acid and methacrylic acid methylester. Eudragit^{®}E 100 is a pH-dependent cationic polymer, which dissolves in the gastric juices at an acidic pH value of up to pH 5.0. Above pH 5.0, it is capable of swelling. In powder form, it is known and commercially available as Eudragit^{®} EPO. Eudragit^{®} EPO has the advantage that the process can be carried out in an aqueous medium and optionally without organic solvents.

Secondary coating of the active-coated pellet can be affected by dissolving the shellac or polymer in a solvent or vehicle, and spraying the solution or suspension onto the active-coated pellets. Volatile components (e.g., solvent) can be subsequently removed (e.g. under vacuum). After the drying process, the pellets may be further sieved.

Suitable solvents for dissolution of the secondary polymer are, for example, solvents which are relatively readily volatile, e.g. one or more of the following: methanol, ethanol, isopropanol, butanol, benzyl alcohol, ethylene glycol, propylene glycol, phenol, acetone, acetic acid, acetic acid anhydride, nitromethane, ethylene diamine, acetic acid cellosolve, e.g. an acetone-ethanol mixture, e.g. in a ratio of 1:1. Aqueous suspensions or solutions may be used, for example coating may be carried out with Eudragit^{®} EPO from an aqueous suspension. According to this process, safety aspects, environmental protection and economical advantages are optimally combined.

Optionally, one or more materials may be applied to the active-containing double-coated particles. For example, a hydrophilic fumed silica (e.g., Aerosil^{®} 200) may be applied to the double-coated particles.

In certain embodiments, when the soft chewable dosage forms include praziquantel as an active, it is incorporated into the dosage form via a double-coated pellet. The process for the production of such double-coated praziquantel pellets may be performed as follows: (a) neutral-tasting, physiologically acceptable, solid pellets, with a diameter of 100-1000 µm for example, preferably an average diameter of 200-250 µm, are coated with praziquantel, (b) praziquantel coated pellets obtained in (a) are further coated with a secondary layer comprising a physiologically acceptable polymer matrix.

In order to coat the pellets, praziquantel can be dissolved in a suitable solvent or vehicle (e.g., purified water, sodium dodecyl sulfate (SDS), polyvinylpyrrolidone), and applied to the pellets by a spraying process. After the spraying procedure, volatile components may be removed (e.g. under vacuum). After the drying process, the praziquantel-coated pellets may be sieved. The praziquantel-coated pellets can be further coated with a secondary coating (e.g., for taste-masking), comprising a physiologically acceptable polymer matrix. In order to apply the secondary coating to the praziquantel-coated pellets, the polymer (e.g., dimethylaminoethyl methacrylate-copolymer, also referred to as Eudragit^{®} EPO) can be dissolved or suspended in a suitable solvent or vehicle (e.g., water, SDS, dibutyl sebacate, and pourous silica gel (e.g., Syloid^{®} 244FP silica)), and applied to the pellets by a spraying process. After the spraying procedure, volatile components may be removed (e.g. under vacuum). After the drying process, the double-coated pellets may be sieved. Optionally, one or more materials may be applied to the praziquantel-containing double-coated particles. For example, a hydrophilic fumed silica (e.g., Aerosil^{®} 200) may be applied to the double-coated particles.

Such double-coated particles (e.g. first coated with praziquantel and then with the polymer matrix), may be further processed with one or more additional actives (e.g., isoxazoline class parasiticides, avermectins, or tetrahydropyrimidines) or with suitable pharmaceutically acceptable excipients (e.g., fillers, disintegrants, glidants and/or lubricants) to obtain a blend for further processing into the final soft chewable dosage form.

The amount of praziquantel in the pellets may be, for example, 10-50%, 20-40%, or 25-35%, based on weight of the final active-coated substrate. The amount of praziquantel in the pellets may be, for example, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, or 50%, based on weight of the final active-coated substrate. The amount of praziquantel in the pellets may be, for example, about 30%, based on weight of the final active-coated substrate.

According to one aspect, the particle size of all excipients may be adjusted to approximate the average diameter of the coated- or double-coated pellets, for example to a size of from 200 µm to 400 µm, from 200 µm and 350 µm, or from 200 µm to 250 µm to avoid segregation during downstream dosage form manufacture.

Preferably, where active-coated substrates (e.g., pellets) are incorporated into the chewable dosage form, the solid particles are embedded in the dosage form matrix with homogenous distribution and content uniformity. The disclosed methods of manufacture provide chewable dosage forms where such particles have homogeneous distribution and content uniformity.

### Carbonate or Bicarbonate Compound

The present chewable compositions comprise a carbonate or bicarbonate compound. The carbonate or bicarbonate compound may be selected from sodium hydrogen carbonate, sodium carbonate, sodium bicarbonate, potassium carbonate, potassium bicarbonate, sodium glycine carbonate, lysine carbonate, arginine carbonate, calcium carbonate, hydrogen carbonate, lithium carbonate, lithium bicarbonate, or a combination thereof. In a preferred embodiment, the carbonate or bicarbonate compound is sodium bicarbonate. In a preferred embodiment, the composition comprises 2.5 to 30% (wt/wt) of the carbonate or bicarbonate compound. In a further embodiment, the composition comprises 2.5 to 30% (wt/wt) sodium bicarbonate, preferably 5 to 30% (wt/wt) sodium bicarbonate, more preferably 5 to 27% (wt/wt) sodium bicarbonate.

In certain embodiments, the soft chewable dosage forms include 0.5% to 40% (wt/wt) a carbonate or bicarbonate compound, preferably sodium bicarbonate, based on total weight of the dosage form. In certain embodiments, the soft chewable dosage forms include 0.5% to 25% (wt/wt) a carbonate or bicarbonate compound, preferably sodium bicarbonate, based on total weight of the dosage form. Preferably, the soft chewable dosage forms include 3% to 25% (wt/wt), more preferably 3% to 15% (wt/wt), more preferably 3% to 10% (wt/wt), and even more preferably 3% to 5% (wt/wt) of a carbonate or bicarbonate compound, preferably sodium bicarbonate, based on total weight of the dosage form.

In certain embodiments, the soft chewable dosage forms include 0.5%-25%, 0.5%-24%, 0.5%-23%, 0.5%-22%, 0.5%-21%, 0.5%-20%, 0.5%-19%, 0.5%-18%, 0.5%-17%, 0.5%-16%, 0.5%-15%, 0.5%-14%, 0.5%-13%, 0.5%-12%, 0.5%-11%, 0.5%-10%, 0.5%-9%, 0.5%-8%, 0.5%-7%, 0.5%-6%, 0.5%-5%, 0.5%-4%, 0.5%-3%, 0.5%-2%, 0.5%-1%, 1%-25%, 1%-24%, 1%-23%, 1%-22%, 1%-21%, 1%-20%, 1%-19%, 1%-18%, 1%-17%, 1%-16%, 1%-15%, 1%-14%, 1%-13%, 1%-12%, 1%-11%, 1%-10%, 1%-9%, 1%-8%, 1%-7%, 1%-6%, 1%-5%, 1%-4%, 1%-3%, 1%-2%, 2%-25%, 2%-24%, 2%-23%, 2%-22%, 2%-21%, 2%-20%, 2%-19%, 2%-18%, 2%-17%, 2%-16%, 2%-15%, 2%-14%, 2%-13%, 2%-12%, 2%-11%, 2%-10%, 2%-9%, 2%-8%, 2%-7%, 2%-6%, 2%-5%, 2%-4%, 2%-3%, 3%-25%, 3%-24%, 3%-23%, 3%-22%, 3%-21%, 3%-20%, 3%-19%, 3%-18%, 3%-17%, 3%-16%, 3%-15%, 3%-14%, 3%-13%, 3%-12%, 3%-11%, 3%-10%, 3%-9%, 3%-8%, 3%-7%, 3%-6%, 3%-5%, 3%-4%, 4%-25%, 4%-24%, 4%-23%, 4%-22%, 4%-21%, 4%-20%, 4%-19%, 4%-18%, 4%-17%, 4%-16%, 4%-15%, 4%-14%, 4%-13%, 4%-12%, 4%-11%, 4%-10%, 4%-9%, 4%-8%, 4%-7%, 4%-6%, 4%-5%, 5%-25%, 5%-24%, 5%-23%, 5%-22%, 5%-21%, 5%-20%, 5%-19%, 5%-18%, 5%-17%, 5%-16%, 5%-15%, 5%-14%, 5%-13%, 5%-12%, 5%-11%, 5%-10%, 5%-9%, 5%-8%, 5%-7%, 5%-6%, 6%-25%, 6%-24%, 6%-23%, 6%-22%, 6%-21%, 6%-20%, 6%-19%, 6%-18%, 6%-17%, 6%-16%, 6%-15%, 6%-14%, 6%-13%, 6%-12%, 6%-11%, 6%-10%, 6%-9%, 6%-8%, 6%-7%, 7%-25%, 7%-24%, 7%-23%, 7%-22%, 7%-21%, 7%-20%, 7%-19%, 7%-18%, 7%-17%, 7%-16%, 7%-15%, 7%-14%, 7%-13%, 7%-12%, 7%-11%, 7%-10%, 7%-9%, 7%-8%, 8%-25%, 8%-24%, 8%-23%, 8%-22%, 8%-21%, 8%-20%, 8%-19%, 8%-18%, 8%-17%, 8%-16%, 8%-15%, 8%-14%, 8%-13%, 8%-12%, 8%-11%, 8%-10%, 8%-9%, 9%-25%, 9%-24%, 9%-23%, 9%-22%, 9%-21%, 9%-20%, 9%-19%, 9%-18%, 9%-17%, 9%-16%, 9%-15%, 9%-14%, 9%-13%, 9%-12%, 9%-11%, 9%-10%, 10%-25%, 10%-24%, 10%-23%, 10%-22%, 10%-21%, 10%-20%, 10%-19%, 10%-18%, 10%-17%, 10%-16%, 10%-15%, 10%-14%, 10%-13%, 10%-12%, 10%-11%, 11%-25%, 11%-24%, 11%-23%, 11%-22%, 11%-21%, 11%-20%, 11%-19%, 11%-18%, 11%-17%, 11%-16%, 11%-15%, 11%-14%, 11%-13%, 11%-12%, 12%-25%, 12%-24%, 12%-23%, 12%-22%, 12%-21%, 12%-20%, 12%-19%, 12%-18%, 12%-17%, 12%-16%, 12%-15%, 12%-14%, 12%-13%, 13%-25%, 13%-24%, 13%-23%, 13%-22%, 13%-21%, 13%-20%, 13%-19%, 13%-18%, 13%-17%, 13%-16%, 13%-15%, 13%-14%, 14%-25%, 14%-24%, 14%-23%, 14%-22%, 14%-21%, 14%-20%, 14%-19%, 14%-18%, 14%-17%, 14%-16%, 14%-15%, 15%-25%, 15%-24%, 15%-23%, 15%-22%, 15%-21%, 15%-20%, 15%-19%, 15%-18%, 15%-17%, 15%-16%, 16%-25%, 16%-24%, 16%-23%, 16%-22%, 16%-21%, 16%-20%, 16%-19%, 16%-18%, 16%-17%, 17%-25%, 17%-24%, 17%-23%, 17%-22%, 17%-21%, 17%-20%, 17%-19%, 17%-18%, 18%-25%, 18%-24%, 18%-23%, 18%-22%, 18%-21%, 18%-20%, 18%-19%, 19%-25%, 19%-24%, 19%-23%, 19%-22%, 19%-21%, 19%-20%, 20%-25%, 20%-24%, 20%-23%, 20%-22%, 20%-21%, 21%-25%, 21%-24%, 21%-23%, 21%-22%, 22%-25%, 22%-24%, 22%-23%, 23%-25%, 23%-24%, or 24%-25% of a carbonate or bicarbonate compound, preferably sodium bicarbonate, based on total weight of the dosage form.

In certain embodiments, the soft chewable dosage forms include 0.5%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, or 25% of a carbonate or bicarbonate compound, preferably sodium bicarbonate, based on total weight of the dosage form. Preferably, the soft chewable dosage forms include 3%, 4%, or 5% of a carbonate or bicarbonate compound, preferably sodium bicarbonate, based on total weight of the dosage form.

In certain embodiments, the soft chewable dosage forms include 10 mg to 500 mg of a carbonate or bicarbonate compound, preferably sodium bicarbonate.

In certain embodiments, the compositions of the present disclosure include a carbonate or bicarbonate compound at a select average particle size. While presence of the carbonate or bicarbonate compound promotes dosage form disintegration and active ingredient dissolution, it has been discovered that carbonate or bicarbonate material with a too large an average particle does not, in certain embodiments, promote sufficient dosage form disintegration and dissolution. Accordingly, in certain embodiments, the carbonate or bicarbonate compound, preferably sodium bicarbonate, used to prepare the chewable compositions has an average particle size of less than or equal to 500 µm, less than or equal to 450 µm, less than or equal to 400 µm, less than or equal to 350 µm, less than or equal to 300 µm, less than or equal to 250 µm, less than or equal to 200 µm, less than or equal to 150 µm, less than or equal to 100 µm, less than or equal to 50 µm. In certain embodiments, the carbonate or bicarbonate compound, preferably sodium bicarbonate, used to prepare the chewable compositions has an average particle size of 10-250 µm, 20-200 µm, 30-150 µm, or 40-100 µm. In certain embodiments, the carbonate or bicarbonate compound, preferably sodium bicarbonate, used to prepare the chewable compositions has an average particle size of about 10 µm, about 20 µm, about 30 µm, about 40 µm, about 50 µm, about 60 µm, about 70 µm, about 80 µm, about 90 µm, about 100 µm, about 110 µm, about 120 µm, about 130 µm, about 140 µm, about 150 µm, about 160 µm, about 170 µm, about 180 µm, about 190 µm, or about 200 µm.

### Flavorants

The present chewable compositions may comprise a flavouring agent to improve the palatability. In particular, the compositions may comprise a meat flavouring. Exemplary flavorants include, but are not limited to, Flavorpal^{®} flavourings, Desiccated Pork Liver Powder^{™}, Provesta^{®} 356, Provesta^{®} 400, PC-0125, Symtripal^{®} flavourings, for example Symtripal^{®} chicken cooked dry flavor, Symtripal^{®} meat chopped type dry flavor, Symtripal^{®} liver type dry flavor and Symtripal^{®} chicken skin and meat dry flavor. Meat or beef flavorings used in exemplary embodiments may be naturally derived or artificially formulated to have a meat or beef flavor. Natural flavorings are often made up of dried and pulverized or powdered meat which may be obtained from domesticated meat animals including cattle such as cows or bulls, pigs, deer, sheep, goats, poultry which may include turkey, chicken, duck and the like. Non-animal, often plant derived flavorings, include soy, peanuts, fruits, sweeteners, honey, sugar, maple syrup and fructose, parsley, celery, peppermint, spearmint, garlic, and the like.

In certain embodiments, the chewable compositions include natural flavorants. In certain embodiments, the chewable compositions include synthetic flavorants. In certain embodiment, the chewable compositions include a combination of natural and synthetic flavorants.

In exemplary embodiments, flavorants may optionally be included in the compositions, for example, in concentrations of 0.5% to 40% (wt/wt), based on total weight of the dosage form. In a preferred embodiment, the flavouring is Symtripal^{®} meat chopped type dry flavor or Symtripal^{®} liver type dry flavor. In a preferred embodiment, the composition comprises 0.5 to 20% (wt/wt) of a flavoring, preferably 0.5 to 10% (wt/wt) of a flavoring, more preferably 0.5 to 7%, based on total weight of the dosage form. In a further embodiment, the composition comprises 0.5 to 10% (wt/wt) Symtripal^{®} meat chopped type dry flavor or Symtripal^{®} liver type dry flavor, based on total weight of the dosage form.

### Humectants

Exemplary humectants include, but are not limited to, glycerol, propylene glycol, cetyl alcohol, glycerol monostearate, ammonium alginate, sodium lactate, sorbitol, triacetin, xylitol, invert sugar, polyhydric alcohols, polyethylene glycol, polyglycerol, xanthan gums, carageenans, alginates, cyclomethicone, sodium hyaluronate, sodium lactate, tracetin, triethanolamine, corn syrup, and mixtures thereof. In some embodiments, the humectant may comprise more than one oil including, but not limited to, fat or fats, both natural and synthetic. Oil employed as an ingredient in the soft chew may be a saturated or unsaturated liquid fatty acid, its glyceride derivatives or fatty acid derivatives of plant or animal origin or a mixture thereof. A source for typical animal fats or oils are fish oil, chicken fat, tallow, choice white grease, prime steam lard and mixtures thereof. However, other animal fats are also suitable for use in the soft chew. Suitable sources for vegetable fats or oils can be derived palm oil, palm hydrogenated oil, corn germ hydrogenated oil, castor hydrogenated oil, cotton-seed oil, soybean oil, olive oil, peanut oil, palm olein oil, coconut oil, cocoa butter, margarine, butter, shortening and palm stearin oil, and mixtures thereof. Additionally, a mixture of animal or vegetable oils or fats is suitable for use in the matrix.

In exemplary embodiments, humectants may optionally be included in the compositions, for example, in concentrations of about 1% to about 25% (wt/wt), about 1% to about 20% (wt/wt), about 1% to about 15% (wt/wt) or about 5% to about 15% (wt/wt), based on total weight of the dosage form. More typically, the compositions will contain about 1% to about 10% (wt/wt) humectant.

### Surfactants

The present chewable compositions may comprise a surfactant. A surfactant (also known as a surface-active agent) is a component which lowers surface tension at the interface between two liquids or between a liquid and solid. Suitable surfacants can be found in REMINGTON: THE SCIENCE AND PRACTICE OF PHARMACY (20th ed., Mack Publishing Co., 2001). Exemplary surfactants include, but are not limited to, glyceryl monooleate, polyoxyethylene sorbitan fatty acid esters, sorbitan esters including sorbitan monooleate (Span^{®} 20), polyvinyl alcohol, polysorbates including polysorbate 20 and polysorbate 80, d-α-tocopheryl polyethylene glycol 1000 succinate (TPGS), Vitamin E TPGS (D-α-Tocopheryl polyethylene glycol 1000 succinate), sodium lauryl sulfate (SLS), copolymers of ethylene oxide and propylene oxide (e.g. poloxamers such as Poloxamer 124, 188, 338, and 407, and LUTROL^{®} F87 and the like), polyethylene glycol castor oil derivatives including polyoxyl 35 castor oil (Cremophor^{®} EL), polyoxyl 40 hydrogenated castor oil (Cremophor^{®} RH 40), polyoxyl 60 hydrogenated castor oil (Cremophor^{®} RH60); propylene glycol monolaurate (LAUROGLYCOL^{®}); glyceride esters including glycerol caprylate/caprate (CAPMUL^{®} MCM), polyglycolized glycerides (GELUCIRE^{®}, such as Gelucire^{®} 44/14), PEG 300 caprylic/capric glycerides (Softigen^{®} 767), PEG 400 caprylic/capric glycerides (Labrasol^{®}), PEG 300 oleic glycerides (Labrafil^{®} M-1944CS), PEG 300 linoleic glycerides (Labrafil^{®} M-2125CS); polyethylene glycol stearates and polyethylene glycol hydroxy stearates including polyoxyl 8 stearate (PEG 400 monostearate), polyoxyl 40 stearate (PEG 1750 monostearate), PEG3350, and the like. Polyethylene glycol stearates (synonyms include macrogol stearates, polyoxylstearates, polyoxyethylene stearates, ethoxylated stearates; CAS No. 9004-99-3, 9005-08-7) are mixtures of mono- and distearate esters of mixed polyoxyethylene polymers. Polyethylene glycol hydroxystearate is a mixture of mono- and diesters of hydroxystearic acid with polyethylene glycols. One polyethylene glycol hydroxystearate that may be used in the compositions is polyethylene glycol 12-hydroxystearate. The compositions may include the surfactant polyethylene glycol 15 12-hydroxystearate (Solutol^{®} HS 15 from BASF), a mixture of mono- and diesters of 12-hydroxystearic acid with 15 moles of ethylene oxide. The compositions may include polyoxyl 35 castor oil (Cremophor^{®} EL) as a surfactant. The compositions may include polyoxyl 40 hydrogenated castor oil (Cremophor^{®} RH 40) or polyoxyl 60 hydrogenated castor oil (Cremophor^{®} RH60) as surfactants.

In exemplary embodiments, surfactants may optionally be included in the compositions, for example, in concentrations of about 0.1% to about 10% (wt/wt), about 1% to about 10% (wt/wt) or about 5% to about 10% (wt/wt), based on total weight of the dosage form. More typically, surfactants may be present at concentrations of about 0.1% to about 5% (wt/wt) or about 1 to about 5% (wt/wt). In a preferred embodiment the composition comprises 0.5 to 10% of a surfactant. In a preferred embodiment, the composition comprises sodium lauryl sulfate. In a further embodiment, the composition comprises 0.5 to 5% (w/w) sodium lauryl sulfate, more preferably about 1 to 2% (w/w) sodium lauryl sulfate.

### Solvents

Exemplary solvents include, but are not limited to, glycerol anhydrous (CAS 56-81-5), various grades of liquid polyethylene glycol (PEG) including PEG 200, PEG 300, PEG 400 and PEG 540; propylene carbonate; propylene glycol; triglycerides including, but not limited to caprylic/capric triglyceride, caprylic/capric/linoleic triglyceride (e.g. MIGLYOL^{®} 810 and 812), caprylic/capric/succinic triglyceride, propylene glycol dicaprylate/dicaprate, and the like; water, sorbitol solution, glycerol caprylate/caprate and polyglycolized glycerides (GELUCIRE^{®}), or a combination thereof. Further exemplary solvents include medium chain triglycerides (MCTs) and vegetable oils termed long-chain triglycerides (LCTs). Triglycerides of medium-chain length fatty acids, known as medium-chain triglycerides or MCTs, can be synthesized by esterifying glycerol with fatty acids of carbon chain lengths of C8 or C10. MCTs are usually commercially-available as a mixture of glycerol esters of C8 (octanoic acid or caprylic acid) and C10 (decanoic acid or capric acid) fatty acids, with small amounts (<1 % of each) of glycerol esters of C6 (hexanoic acid or caproic acid) and C12 (dodecanoic acid or lauric acid) fatty acids.

In exemplary embodiments, solvents may optionally be included in the compositions, for example, in concentrations of about 1 to about 50% (wt/wt), based on total weight of the dosage form. In other embodiments, the concentration of the solvents will be from about 1 to about 40% (wt/wt), about 1 to about 30% (wt/wt) or about 1 to about 20% (wt/wt). More typically, the solvents will be in the compositions at concentrations of about 5% to about 20% (wt/wt) or about 5% to about 15% (wt/wt).

In certain embodiments, the chewable compositions do not include water as contributed from addition as a solvent. It has been discovered that, in certain embodiments, addition of water to the formulation reduces the efficiency of the carbonate or bicarbonate disintegrant. Without wishing to be bound by theory, it is believed added water may react with carbonate or bicarbonate present in the chewable formulation, thereby reducing carbonate or bicarbonate compound content available for reaction with physiological media, and thus dosage form disintegration and dissolution.

### Fillers

The present chewable compositions may comprise a filler. Exemplary fillers include, but are not limited to, corn starch, pre-gelatinized corn starch, soy protein fines, corn cob, corn gluten meal, dibasic caclcium phosphate hydrous or anhydrous, microcrystalline cellulose (MCC), and lactose hydrous or anhydrous. The starch component may comprise starch from any source and may act as a binder in the soft chew. The starch component used in the compositions may be unmodified, or derivatized and/or pregelatinized. Starches that can serve as a base starch for derivatization include regular corn, waxy corn, potato, tapioca, rice, etc. Suitable types of derivatizing agents for the starch include, but are not limited to, ethylene oxide, propylene oxide, acetic anhydride, and succinic anhydride, and other food approved esters or ethers, introducing such chemicals alone or in combination with one another.

The present chewable compositions may comprise a hydrophilic filler. A "hydrophilic filler" is a hydrophilic component used to increase the mass of the composition. Suitable hydrophilic fillers include, but are not limited to, mannitol, lactose, sucrose, sorbitol and dextrose.

In exemplary embodiments, fillers may optionally be included in the compositions, for example, in concentrations of about 5% to about 80% (wt/wt), about 10% to about 70% (wt/wt), about 10% to about 60%, about 10% to about 50% (wt/wt), or about 10% to about 40% (wt/wt), based on total weight of the dosage form. More typically, the fillers may be present at concentrations of about 30% to about 70% (wt/wt), about 30% to about 60% (wt/wt), about 30% to about 50% (wt/wt), or about 35% to about 55% (wt/wt).

### Forming Agents

Exemplary forming agents include, but are not limited to, polyethylene glycol (PEG), microcrystalline wax, cetyl alcohol and polyvinylpyrrolidone (PVP). Depending upon the desired consistency of the composition, different molecular weight PEG may be utilized (e.g., higher or lower than PEG 8000, but preferably higher than 600).

In exemplary embodiments, forming agents may optionally be included in the compositions, for example, in concentrations of about 2% to about 30% wt/wt of the composition, based on total weight of the dosage form.

### Binders

The present chewable compositions may comprise a binder. A binder is a component which increases cohesiveness. Suitable binders can be found in REMINGTON: THE SCIENCE AND PRACTICE OF PHARMACY (20th ed., Mack Publishing Co., 2001).

Exemplary binders include, but are not limited to, polyvinylpyrrolidone (e.g. Povidone and Plasdone^{®} K-29/32), cross-linked polyvinylpyrrolidone (Crospovidone), polyethylene glycols of various grades including PEG 3350, PEG 4000, PEG 6000, PEG 8000 and even PEG 20,000, and the like; co-polymers of vinylpyrrolidone and vinyl acetate (e.g. Copovidone) such as the product sold by BASF by the tradename Kollidon^{®} VA 64 and the like; starch such as potato starch, tapioca starch or corn starch; molasses, corn syrup, honey, maple syrup and sugars of various types; hydroxypropyl cellulose (HPC), hydroxypropyl methylcellulose (HPMC), and alginate.

In exemplary embodiments, binders may optionally be included in the compositions, for example, in concentrations of about 1% to about 30% (wt/wt). More typically, the compositions will include binders at a concentration of about 1% to about 20% (wt/wt), about 1 to about 15% (wt/wt), about 1% to about 10% (wt/wt), about 5% to about 15% (wt/wt), about 5% to about 10% (wt/wt), or about 1% to about 5% (wt/wt), based on total weight of the dosage form.

### Antioxidants

The present chewable compositions may comprise an antioxidant. "Antioxidants" are components which serve as preservatives to increase the stability of active ingredients which are not stable if exposed to oxygen. Suitable antioxidants can be found in REMINGTON: THE SCIENCE AND PRACTICE OF PHARMACY (20th ed., Mack Publishing Co., 2001). Suitable antioxidants include, but are not limited to, Tenox^{®} 2; Tenox^{®} PG; Tenox^{®} s-1; BHA (2-t-butyl-4-methoxyphenol); BHT (2,6-di-t-butyl-4-methylphenol); sodium metabisulfite reducing agents; antoxidant synergists such as tocopherols (alpha, beta, or delta-tocopherol, tocopherol esters, alpha-tocopherol acetate), alkyl gallates, butylated hydroxyanisole, butylated hydroxytoluene, citric acid anhydrous and hydrous, edetic acid and its salts, lecithin, tartaric acid, ascorbic acid, ascrobyl palmitate, fumaric acid, malic acid, sodium ascorbate, sodium metabisulfate, n-propyl gallate, monothioglycerol, resveratrol, quercetin, benzoic acid, dimethyl thiourea (DMTU), hesperetin, tetrahydrocurcumin, tetrahydrodemethoxycurcumin, and Trolox (N-acetylcysteine, 6-hydroxy-2,5,7,8-tetramethylchroman-2-carboxylic acid). In a preferred embodiment, the antioxidant is BHA.

In exemplary embodiments, antioxidants may optionally be included in the compositions, for example, in concentrations of about 0.01 to about 2.0% (wt/wt), based upon total weight of the soft chewable dosage form, with about 0.05 to about 1.0% or about 0.1% to about 0.2% (wt/wt) being especially preferred. In a preferred embodiment, the composition comprises 0.01 to 0.2% (w/w) of an antioxidant. In a further embodiment, the composition comprises 0.01 to 0.2% (w/w) BHA, preferably about 0.1% BHA.

### Preservatives

Exemplary preservatives include, but are not limited to, the parabens (methylparaben and/or propylparaben), benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, ascorbic acid, calcium

In exemplary embodiments, preservatives may optionally be included in the compositions, for example, in concentrations of about 0.01 to about 5.0% (wt/wt), about 0.01 to about 2% (wt/wt) or about 0.05 to about 1.0% (wt/wt), based on total weight of the dosage form.

### Stabilizers

Exemplary stabilizers include, but are not limited to, magnesium stearate, citric acid, and sodium citrate. pH stabilizers (also referred to as buffers) include, for example, acetic acid/acetate, malic acid/malate, citric acid/citrate, tartaric acid/tartrate, lactic acid/lactate, phosphoric acid/phosphate, glycine/glycimate, tris, glutamic acid/glutamates and sodium carbonate.

In certain embodiments, the soft chewable dosage forms may include stabilizers and combinations of stabilizers to prevent or reduce macrocyclic lactone (e.g., moxidectin or doramectin) degradation. Suitable stabilizers include glycerol formal, amino sugars (e.g., glucosamine, tromethamine, meglumine, and the like), glycol ethers, modified starches, for example hydroxypropyl starch phosphate; cellulose derivatives (e.g., hydroxypropylmethyl cellulose (HPMC), ethyl cellulose, hydroxypropylmethyl cellulose acetyl succinate, carboxymethyl cellulose, and the like); and non-ionic triblock copolymers (e.g., Poloxamer-101 , -108, -124, -188, - 215, -284, -338, -407, and the like), all of which may be used to physically stabilize the macrocyclic lactone. In certain embodiments, a macrocyclic lactone (e.g., moxidectin) can be stabilized with a combination of stabilizing agents and optionally, at least one antioxidant. For example, a macrocyclic lactone such as moxidectin may be stabilized with hydroxypropylmethyl cellulose and meglumine and further stabilized with an antioxidant (e.g., BHT).

In exemplary embodiments, stabilizers may optionally be included in the compositions, for example, in concentrations of about 0.1 to about 5% (wt/wt), about 0.1 to about 3% (wt/wt) or about 0.1 to about 2% (wt/wt), based on total weight of the dosage form.

### Lubricants

Exemplary lubricants include, but are not limited to, polyethylene glycols of various molecular weight ranges including PEG 3350 (Dow Chemical) and PEG 4000, corn oil, mineral oil, hydrogenated vegetable oils (STEROTEX or LUBRITAB), peanut oil and/or castor oil. In certain embodiments, the lubricant is a neutral oil comprising a medium chain triglyceride (MCT) or propylene glycol fatty acid esters including caprylic/capric triglycerides. Non-limiting examples of neutral oils are known by the trademark MIGLYOL^{®} including MIGLYOL^{®} 810, MIGLYOL^{®} 812, MIGLYOL^{®} 818, MIGLYOL^{®} 829 and MIGLYOL^{®} 840. Further lubricants include vegetable oils termed long-chain triglycerides (LCT), magnesium stearate (MgSt), stearic acid, and sodium stearyl fumarate.

In exemplary embodiments, lubricants may optionally be included in the compositions, for example, in concentrations of about 1% to about 50% (wt/wt), about 1% to about 20% (wt/wt), about 1% to about 15% (wt/wt), about 1% to about 10% (wt/wt), or about 1% to about 5% (wt/wt), based on total weight of the dosage form.

### Glidants

Exemplary flow aids or glidants include, but are not limited to, silicon dioxide (CARBOSIL) or silica gel (SYLOID), talc, starch, calcium, stearate, magnesium stearate, and aluminum magnesium silicate (NEUSILIN).

In exemplary embodiments, glidants may optionally be included in the compositions, for example, in concentrations of about about 0.01 to about 25%, based upon weight of total composition.

### Palatibility Enhancers

The present chewable compositions may comprise one or more palatability enhancers. The present chewable compositions may comprise sodium chloride as a palatability enhancer. In a preferred embodiment, the composition comprises 1 to 2% (w/w) sodium chloride, preferably about 1.5% (w/w) sodium chloride.

### Sweeteners

The present chewable compositions may comprise a sweetener to further improve the palatability. Any natural sugar may be used including confectioners' sugar, maltitol, xylitol, sorbitol, mannitol, lactose, dextrose, saccharose, glucose or fructose, or any mixture thereof. Artificial sweeteners may also be used including saccharins, aspartame, acesulfame-k and Rebaten^{®}. In a preferred embodiment, the sweetener is Rebaten^{®}. In a preferred embodiment, the composition comprises 0.5 to 5% (w/w) sweetener, preferably 0.5% to 2% (w/w) sweetener, more preferably about 1% (w/w) of a sweetener. In a further embodiment, the composition comprises about 1% (w/w) Rebaten^{®}.

### Colorants

The present chewable compositions may comprise a coloring agent. The coloring agent may be selected from azo dyes, organic or inorganic pigments or coloring agents of natural origin. In a preferred embodiment, the composition comprises 0.05 to 0.25% (w/w) of a coloring agent.

### Plasticizers

The present chewable compositions may comprise a plasticizer. A "plasticizer" is a component which acts to increase the plasticity properties of the composition. Suitable plasticizers include, but are not limited to, glycerol, alcohols (such as chlorobutanol or sorbitol), glycols (such as polyethylene glycol or propylene glycol), phthalates (such as diethyl phthalate), lanolin, wool fat, liquid paraffin, petrolatum, benzyl phenyl formate, triacetin, poly(lactic-co-glycolic)acid (PLGA), methacrylates, acetyltributyl citrate, castor oil, dibutyl sebacate, tributyl citrate, triethyl citrate or Myvacet^{®} 9-45 K NF, or any combination thereof. In a preferred embodiment, the plasticizer is glycerol. In a preferred embodiment, the composition comprises 2 to 30% (w/w) plasticizer. In a further embodiment, the composition comprises 2 to 30% (w/w) glycerol, preferably 3 to 23% (w/w) glycerol, more preferably 6 to 20% (w/w) glycerol.

### Additional Disintegrants

Exemplary additional disintegrants include, but are not limited to, cellulose, carboxymethyl cellulose calcium, carboxymethyl cellulose sodium, polacrilin potassium, starch, hydroxypropyl starch, corn starch, bentonite, veegum, pregelatinized starch, modified starch, lactose monohydrate, croscarmellose sodium, microcrystalline cellulose, hydroxypropyl cellulose, glycine, crospovidone, magnesium aluminum silicate, sodium starch glycolate, guar gum, colloidal silicon dioxide, polyvinylpyrrolidone (Povidone), alginic acid, sodium alginate, calcium alginate, methylcellulose, and chitosan.

In exemplary embodiments, additional disintegrants may optionaly be included in the compositions, for example, in concentrations of 0% (wt/wt) to about 10% (wt/wt)about 1% (wt/wt) to about 10% (wt/wt), about 1% (wt/wt) to about 7% (wt/wt), about 1% (wt/wt) to about 5% (wt/wt), or about 2% (wt/wt) to about 4% (wt/wt) of one or more disintegrants, based on total weight of the dosage form.

In certain embodiments, the sole disintegrant in the soft chewable dosage form is sodium bicarbonate. In certain embodiments, the soft chewable dosage forms exclude inclusion of additional disintegrants beyond sodium bicarbonate.

### 3. Methods of Making

The compositions of the present disclosure can be manufactured according to methods known to those skilled in the art. For example, methods of dry blending, dry granulation, wet granulation, direct compression, and melt extrusion can be used to manufacture the disclosed compositions.

In an exemplary embodiment, compositions can be manufactured by preparing a powder blend of solid ingredients, adding liquid ingredients to the powder blend and granulating, and extruding the granulated powder blend to provide extrudates. In another exemplary embodiment, compositions can be manufactured by preparing a powder blend of solid ingredients, adding liquid ingredients to the powder blend and granulating, and extruding the granulated powder blend to provide extrudates.

In another exemplary embodiment, compositions can be manufactured by preparing a powder blend of solid ingredients [e.g., weighing powder materials, passing the powder materials through a sieve, mixing the powder materials (e.g., in a blender)]; preparing a liquid solution of liquid ingredients (e.g., weighing liquid materials, combining liquid ingredients); granulating (e.g., powder blend is poured into a Hobart planetary mixer, under stirring, the liquid is added to the powder blend for granulation); and extruding the granulate (e.g., final granulated blend is transferred to an extruder and extruded through a die).

In another exemplary embodiment, compositions can be manufactured according to the following procedure: (a) Prepare a powder blend: Powder materials are weighed and passed through a sieve (e.g., a 1000 µm sieve). The powders are poured into a bottle (e.g., a glass bottle) and mixed for a suitable time in a blender (e.g., 15 minutes with a Turbula blender). (b) Granulation: The powder blend is poured into the bowl of a mixer (e.g., a Hobart planetary mixer). Liquid ingredients are weighed and prepared into a liquid solution. Under stirring, the liquid solution is added to the powder blend for granulation. (c) Extrusion: The final granulated blend is transferred to an extruder (e.g., a Gabler Extruder DE40) and extruded through a die (e.g., a 13mm round die). Extrudates can be cut into single units, packed and stored at selected conditions. In certain embodiments, the extruder used for the production of the chewable compositions may be a co-rotating twin screw extruder type DE40/10D with a screw diameter of 40 mm and a screw length of L/D ratio of 10D from GABLER GmbH. In certain embodiments, a chiller/heater from Haake (subsequently ThermoFisher) can be used to maintain the double jacket of the extruder at 40°C.

In another exemplary embodiment, compositions can be manufactured according to the following procedure: Pre-blend active pharmaceutical ingredients and colorant with starch using a bin blender. Transfer preblend and remainder of solid materials into a drum of a bin blender. Blend, for example, for 15 minutes. Sieve powder blend and transfer into drum of a bin blender. Blend, for example, for 15 minutes. Sieve powder blend and transfer into K-Tron Powder feeder. Mix glycerin, optionally with an antioxidant solution and/or water, and transfer into tank of the liquid feeder. Set extrusion process parameters and start extruder. Liquid mixtures are pumped into the extruder. Powder blend is dosed into the extruder. Start automated cutting process. Cutting device of the extruder is adjusted to get the appropriate weights of the chewable dosage forms. Collect chewable dosage forms after extrusion and transfer to trays. Cure the chewable dosage forms for at least 24 hours at ambient conditions. After curing chewable dosage forms are packed.

In another exemplary embodiment, compositions can be manufactured by blending dry ingredients to provide a chew mixture; adding liquid components (e.g., oil, humectants or solvents) to the chew mixture, and blending the dry ingredient/liquid ingredient mixture to form a thoroughly blended mixture. After blending, the resultant soft chew mixture can be discharged from a port through a blender into a suitable container for processing into individual dosage units by hand or preferably with a forming machine. A variety of forming equipment may be utilized, but those particularly preferred for use are molding machines developed for use in producing molded food products. For example, suitable forming equipment includes the Formax F6^{™} molding machine made by the Formax Corporation. Each batch of chewable dosage forms may be packaged in bulk or, preferably, each soft chew is then individually packaged for storage. Examples of suitable packaging materials include HDPE bottles, blister or foil/foil packaging.

When active-coated pellets are included in the formulations, preferably such pellets are dosed with dry ingredients during manufacture.

In another exemplary embodiment, steps of blending, milling/sieving, extrusion, and mixing can be accomplished employing equipment listed in Table 1.

| Capability | Pilot Scale | Commercial Scale |
|---|---|---|
| Blending | Drum Blending | Bin Blender with Chopper - 1200 L |
| | • 1.5-150 L | |
| | | Drum Blender - 150-280 L |
| Milling/Sieving | Frewitt DM-MF-3 (oscillator) | Frewitt DM-MF-3 (oscillator) |
| | Bohle BTS100 (screening) | Bohle BTS100 (screening) |
| | | Quadro U20 (conical) |
| | | Fitzmill D6A (hammer) |
| | | Glatt TR120 (tumbler) |
| Extrusion | Buehler Extruder BCTL-42/28D | Buehler Extruder BCTL-62/28D |
| | K-Tron Feeder | K-Tron Feeder |
| Mixing | Planetary mixer (Hobart N50) | Drum granulation |

### 4. Formulation Properties

The disclosed compositions can be characterized by one or more properties. For example, the compositions can be characterized by acidity, adhesion, chewiness, cohesiveness, compression at break, compressive extrusion, disintegration, dissolution, lipophilic content, moisture content, palatability, pH, springiness, surface porosity, texture, water activity, or wettability.

### Disintegration

The present disclosure provides chewable compositions which have rapid disintegration profiles. Disintegration of the disclosed compositions can be assessed by according to disintegration method 2.9.1 (Test B) of the European Pharmacopoeia 6.0.

Preferred compositions of the present disclosure disintegrate in less than 50 minutes in water and/or less than 30 minutes in 0.1M HCl as determined by the disintegration method 2.9.1 (Test B) of the European Pharmacopoeia 8.0. More preferred compositions of the present disclosure disintegrate in less than 25 minutes in water and/or less than 20 minutes in 0.1M HCl as determined by the disintegration method 2.9.1 (Test B) of the European Pharmacopoeia 8.0. Yet more preferred compositions of the present disclosure disintegrate in less than 15 minutes in water and/or less than 15 minutes in 0.1M HCl as determined by the disintegration method 2.9.1 (Test B) of the European Pharmacopoeia.

The compositions may have a disintegration time of less than or equal to 60 minutes, less than or equal to 55 minutes, less than or equal to 50 minutes, less than or equal to 45 minutes, less than or equal to 40 minutes, less than or equal to 35 minutes, less than or equal to 30 minutes, less than or equal to 25 minutes, less than or equal to 20 minutes, less than or equal to 15 minutes, or less than or equal to 10 minutes, measured in water as determined by the disintegration method 2.9.1 (Test B) of the European Pharmacopoeia 6.0. The compositions may have a disintegration time of 60 minutes, 59 minutes, 58 minutes, 57 minutes, 56 minutes, 55 minutes, 54 minutes, 53 minutes, 52 minutes, 51 minutes, 50 minutes, 49 minutes, 48 minutes, 47 minutes, 46 minutes, 45 minutes, 44 minutes, 43 minutes, 42 minutes, 41 minutes, 40 minutes, 39 minutes, 38 minutes, 37 minutes, 36 minutes, 35 minutes, 34 minutes, 33 minutes, 32 minutes, 31 minutes, 30 minutes, 29 minutes, 28 minutes, 27 minutes, 26 minutes, 25 minutes, 24 minutes, 23 minutes, 22 minutes, 21 minutes, 20 minutes, 19 minutes, 18 minutes, 17 minutes, 16 minutes, 15 minutes, 14 minutes, 13 minutes, 12 minutes, 11 minutes, or 10 minutes, measured in water as determined by the disintegration method 2.9.1 (Test B) of the European Pharmacopoeia 6.0.

The compositions may have a disintegration time of less than or equal to 60 minutes, less than or equal to 55 minutes, less than or equal to 50 minutes, less than or equal to 45 minutes, less than or equal to 40 minutes, less than or equal to 35 minutes, less than or equal to 30 minutes, less than or equal to 25 minutes, less than or equal to 20 minutes, less than or equal to 15 minutes, or less than or equal to 10 minutes, measured in 0.1M HCl as determined by the disintegration method 2.9.1 (Test B) of the European Pharmacopoeia 6.0. The compositions may have a disintegration time of 60 minutes, 59 minutes, 58 minutes, 57 minutes, 56 minutes, 55 minutes, 54 minutes, 53 minutes, 52 minutes, 51 minutes, 50 minutes, 49 minutes, 48 minutes, 47 minutes, 46 minutes, 45 minutes, 44 minutes, 43 minutes, 42 minutes, 41 minutes, 40 minutes, 39 minutes, 38 minutes, 37 minutes, 36 minutes, 35 minutes, 34 minutes, 33 minutes, 32 minutes, 31 minutes, 30 minutes, 29 minutes, 28 minutes, 27 minutes, 26 minutes, 25 minutes, 24 minutes, 23 minutes, 22 minutes, 21 minutes, 20 minutes, 19 minutes, 18 minutes, 17 minutes, 16 minutes, 15 minutes, 14 minutes, 13 minutes, 12 minutes, 11 minutes, or 10 minutes, measured in 0.1M HCl as determined by the disintegration method 2.9.1 (Test B) of the European Pharmacopoeia 6.0.

In certain embodiments, the compositions may have a disintegration time less than or equal to 60 minutes, less than or equal to 55 minutes, less than or equal to 50 minutes, less than or equal to 45 minutes, less than or equal to 40 minutes, less than or equal to 35 minutes, less than or equal to 30 minutes, less than or equal to 25 minutes, less than or equal to 20 minutes, less than or equal to 15 minutes, or less than or equal to 10 minutes after storage at 40°C and atmospheric pressure in aluminum foil bags, aluminum blister packs, or similar storage receptacles for at least 5 days, at least 10 days, at least 15 days, at least 20 days, at least 25 days, at least 30 days, at least 35 days, at least 40 days, at least 45 days, or at least 50 days, measured in water as determined by the disintegration method 2.9.1 (Test B) of the European Pharmacopoeia 6.0. The compositions may have a disintegration time of 60 minutes, 59 minutes, 58 minutes, 57 minutes, 56 minutes, 55 minutes, 54 minutes, 53 minutes, 52 minutes, 51 minutes, 50 minutes, 49 minutes, 48 minutes, 47 minutes, 46 minutes, 45 minutes, 44 minutes, 43 minutes, 42 minutes, 41 minutes, 40 minutes, 39 minutes, 38 minutes, 37 minutes, 36 minutes, 35 minutes, 34 minutes, 33 minutes, 32 minutes, 31 minutes, 30 minutes, 29 minutes, 28 minutes, 27 minutes, 26 minutes, 25 minutes, 24 minutes, 23 minutes, 22 minutes, 21 minutes, 20 minutes, 19 minutes, 18 minutes, 17 minutes, 16 minutes, 15 minutes, 14 minutes, 13 minutes, 12 minutes, 11 minutes, or 10 minutes, after storage at 40°C and atmospheric pressure in aluminum foil bags, aluminum blister packs, or similar storage receptacles for at least 5 days, at least 10 days, at least 15 days, at least 20 days, at least 25 days, at least 30 days, at least 35 days, at least 40 days, at least 45 days, or at least 50 days, measured in water as determined by the disintegration method 2.9.1 (Test B) of the European Pharmacopoeia 6.0.

In certain embodiments, the compositions may have a disintegration time less than or equal to 60 minutes, less than or equal to 55 minutes, less than or equal to 50 minutes, less than or equal to 45 minutes, less than or equal to 40 minutes, less than or equal to 35 minutes, less than or equal to 30 minutes, less than or equal to 25 minutes, less than or equal to 20 minutes, less than or equal to 15 minutes, or less than or equal to 10 minutes after storage at 40°C and atmospheric pressure in aluminum foil bags, aluminum blister packs, or similar storage receptacles for at least 5 days, at least 10 days, at least 15 days, at least 20 days, at least 25 days, at least 30 days, at least 35 days, at least 40 days, at least 45 days, or at least 50 days, measured in 0.1M HCl as determined by the disintegration method 2.9.1 (Test B) of the European Pharmacopoeia 6.0. The compositions may have a disintegration time of 60 minutes, 59 minutes, 58 minutes, 57 minutes, 56 minutes, 55 minutes, 54 minutes, 53 minutes, 52 minutes, 51 minutes, 50 minutes, 49 minutes, 48 minutes, 47 minutes, 46 minutes, 45 minutes, 44 minutes, 43 minutes, 42 minutes, 41 minutes, 40 minutes, 39 minutes, 38 minutes, 37 minutes, 36 minutes, 35 minutes, 34 minutes, 33 minutes, 32 minutes, 31 minutes, 30 minutes, 29 minutes, 28 minutes, 27 minutes, 26 minutes, 25 minutes, 24 minutes, 23 minutes, 22 minutes, 21 minutes, 20 minutes, 19 minutes, 18 minutes, 17 minutes, 16 minutes, 15 minutes, 14 minutes, 13 minutes, 12 minutes, 11 minutes, or 10 minutes, after storage at 40°C and atmospheric pressure in aluminum foil bags, aluminum blister packs, or similar storage receptacles for at least 5 days, at least 10 days, at least 15 days, at least 20 days, at least 25 days, at least 30 days, at least 35 days, at least 40 days, at least 45 days, or at least 50 days, measured in 0.1M HCl as determined by the disintegration method 2.9.1 (Test B) of the European Pharmacopoeia 6.0.

### Dissolution

Dissolution of the disclosed compostions can be assessed according to Ph.Eur. 2.9.3. "Dissolution Test for Solid Dosage Forms." The disclosed compositions may release at least 30%, at least 35%, at least 40%, at least 45%, at least 50%, at least 55%, at least 60%, at least 65%, at least 70%, at least 75%, at least 80%, at least 85%, at least 90%, or at least 95% of active ingredient(s) within 60 minutes, as determined according to Ph.Eur. 2.9.3. The disclosed compositions may release at least 30%, at least 35%, at least 40%, at least 45%, at least 50%, at least 55%, at least 60%, at least 65%, at least 70%, at least 75%, at least 80%, at least 85%, at least 90%, or at least 95% of active ingredient(s) within 45 minutes, as determined according to Ph.Eur. 2.9.3. The disclosed compositions may release at least 30%, at least 35%, at least 40%, at least 45%, at least 50%, at least 55%, at least 60%, at least 65%, at least 70%, at least 75%, at least 80%, at least 85%, at least 90%, or at least 95% of active ingredient(s) within 30 minutes, as determined according to Ph.Eur. 2.9.3. The disclosed compositions may release at least 60%, at least 65%, at least 70%, at least 75%, at least 80%, at least 85%, or at least 90% of active ingredient(s) withinin 15 minutes, as determined according to Ph.Eur. 2.9.3. The relevant parameters of the Ph.Eur. 2.9.3 dissolution test for the disclosed compositions are provided in Table 2.

### Lipophilic Content

In certain embodiments, the compositions of the present disclosure include lipophilic content (e.g., contributed via fat powder and/or flavorant) to enhance palatability. While presence of lipophilic material (e.g., fat powder) can enhance palatability, it has been discovered that too high lipophilic content can impede suitable dosage form disintegration and active ingredient dissolution. Without wishing to be bound by theory, it is believed excessive lipophilic content in the dosage form may hinder rapid disintegration and dissolution by limiting access of the physiological media to the carbonate or bicarbonate compound within the dosage form. Accordingly, in certain embodiments, the present disclosure provides compositions which have an advantageous lipophilic content configured to balance palatability and disintegrant performance. In certain embodiments, the soft chewable dosage forms include 0%-15%, 0%-14%, 0%-13%, 0%-12%, 0%-11%, 0%-10%, 0%-9%, 0%-8%, 0%-7%, 0%-6%, 0%-5%, 0%-4%, 0%-3%, 0%-2%, 0%-1%, 1%-15%, 1%-14%, 1%-13%, 1%-12%, 1%-11%, 1%-10%, 1%-9%, 1%-8%, 1%-7%, 1%-6%, 1%-5%, 1%-4%, 1%-3%, 1%-2%, 2%-15%, 2%-14%, 2%-13%, 2%-12%, 2%-11%, 2%-10%, 2%-9%, 2%-8%, 2%-7%, 2%-6%, 2%-5%, 2%-4%, 2%-3%, 3%-15%, 3%-14%, 3%-13%, 3%-12%, 3%-11%, 3%-10%, 3%-9%, 3%-8%, 3%-7%, 3%-6%, 3%-5%, 3%-4%, 4%-15%, 4%-14%, 4%-13%, 4%-12%, 4%-11%, 4%-10%, 4%-9%, 4%-8%, 4%-7%, 4%-6%, 4%-5%, 5%-15%, 5%-14%, 5%-13%, 5%-12%, 5%-11%, 5%-10%, 5%-9%, 5%-8%, 5%-7%, 5%-6%, 6%-15%, 6%-14%, 6%-13%, 6%-12%, 6%-11%, 6%-10%, 6%-9%, 6%-8%, 6%-7%, 7%-15%, 7%-14%, 7%-13%, 7%-12%, 7%-11%, 7%-10%, 7%-9%, 7%-8%, 8%-15%, 8%-14%, 8%-13%, 8%-12%, 8%-11%, 8%-10%, 8%-9%, or 9%-10% fat powder content (wt/wt), based on total weight of the dosage form.

### Palatability

The present disclosure provides compositions which are palatable. The compositions can have a palatability of greater than or equal to 60%, greater than or equal to 65%, greater than or equal to 70%, greater than or equal to 75%, greater than or equal to 80%, greater than or equal to 85%, greater than or equal to 90%, or greater than or equal to 95%, as measured according to the Guideline on the demonstration of palatability of veterinary medicinal products, effective February 2015 [EMA/CVMP/EWP/206024/2011 (2014)], Committee for Medicinal Products for Veterinary Use (CVMP). The compositions can have a palatability of about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100%, as measured according to the Guideline on the demonstration of palatability of veterinary medicinal products, effective February 2015 [EMA/CVMP/EWP/206024/2011 (2014)], Committee for Medicinal Products for Veterinary Use (CVMP).

### Stability

The present disclosure provides compositions which are storage stable. The compositions may have acceptable physical and chemical stability, providing at least 12, 13, 14, 15, 16, 17, 18, 19, 20, 21, 22, 23, 24, 25, 26, 27, 28, 29, 30, 31, 32, 33, 34, 35, or 36 months shelf life.

### Texture

The present disclosure provides compositions which are chewable. Chewable means that the composition can be chewed by the animal, it should not be so brittle that it breaks or snaps when the animal bites the composition nor should it be so soft that there is complete loss of shape when the animal bites the composition. For preferred compositions of the present disclosure, the peak force necessary for a sphere with a diameter of 8mm with a velocity of 1mm/s to penetrate 2mm into the composition is between 5 and 40 N at room temperature at 1 hour post-extrusion, more preferably between 5 and 25 N and most preferably between 10 and 25 N.

The texture (softness/hardness) of the chewable formulation can be measured, for example, by using a TEXTURE ANALYSER type: TA-XT2 iHR/25 that is commercially available from Stable Micro Systems Ltd. (Headquater: Stable Micro Systems Ltd., Vienna Court, Lammas Road; UK). The peak force (in N) necessary to push a sphere with a velocity of 1 mm/s to penetrate 1 or 2 mm into the chewable composition is measured. The sphere has a diameter of 8 mm. The measurements can be taken at a selected time (e.g., 1 hour) post extrusion.

### Content Uniformity

The present disclosure provides compositions have content uniformity. Content uniformity can be assessed according to Ph.Eur. 2.9.40 (Uniformity of Dosage Units).

### 5. Methods of Use

The compositions of the present disclosure can be used to treat and/or control ectoparasite infections or infestations in an animal. The compositions can be used as an ectoparasiticide, in particular, as an acaricide and insecticide. They may, in particular, be used in the fields of veterinary medicine, livestock husbandry and the maintenance of public health: against acarids, insects, and copepods which are parasitic upon vertebrates, particularly warm-blooded vertebrates, including companion animals, livestock, and fowl and cold-blooded vertebrates like fish. Non-limiting examples of ectoparasites include: ticks (e.g., *Ixodes* spp., (e.g., *I. ricinus, I. hexagonus*), *Rhipicephalus* spp. (e.g., *R. sanguineus*), *Boophilus* spp., *Amblyomma* spp. (e.g., *A. americanum, A. maculatum, A. triste, A. parvum, A. cajennense, A. ovale, A. oblongoguttatum, A. aureolatum, A. cajennense*), *Hyalomma* spp., *Haemaphysalis* spp., *Dermacentor* spp. (e.g., *D. variabilis, D. andersoni, D. marginatus*), *Ornithodorus* spp., and the like); mites
(e.g., *Dermanyssus* spp., *Sarcoptes* spp. (e.g., *S. scabiei*), *Psoroptes* spp. (e.g., *P. bovis*), *Otodectes* spp., *Chorioptes* spp., *Demodex* spp., (e.g., *D. folliculorum, D. canis,* and *D. brevis*) and the like); chewing and sucking lice
(e.g., *Damalinia* spp., *Linognathus* spp., *Cheyletiella* spp., *Haematopinus* spp., *Solenoptes* sp *p., Trichodectes* spp., *Felicola* spp., and the like); fleas
(e.g., *Siphonaptera* spp., *Ctenocephalides* spp., and the like); biting flies, midges, and mosquitos
(e.g., *Tabanidae* spp., *Haematobia* spp., *Musca* spp., *Stomoxys* spp., *Dematobia* spp., *Cochlio myi*
*a* spp., *Simuliidae* spp., *Ceratopogonidae* spp., *Psychodidae* spp., *Aedes* spp., *Culex* spp., *Ano phles* spp., *Lucilia* spp., *Phlebotomus* spp., *Lutzomyia* spp., and the like); bed bugs (e.g., insects within the genus *Cimex* and family Cimicidae); and grubs (e.g., *Hypoderma bovis, H. lineatum*); and copepods (e.g., sea lice within the Order Siphonostomatoida, including genera *Lepeophtheirus and Caligus*)*.*

The compositions of the present disclosure can be used to treat and/or control endoparasites, for example, helminths (e.g., trematodes, cestodes, and nematodes) including heartworm, roundworm, hookworm, whipworm, fluke, and tapeworm. The gastrointestinal roundworms include, for example, *Ostertagia ostertagi* (including inhibited larvae), O. *lyrata, Haemonchus placei, H. similis, H. contortus, Toxocara canis, T. leonina, T. cati, Trichostrongylus axei, T. colubriformis, T. longispicularis, Cooperia oncophora, C. pectinata, C. punctata, C. surnabada* (syn. *mcmasteri*)*, C. spatula, Ascaris suum, Hyostrongylus rubidus, Bunostomum phlebotomum, Capillaria bovis, B. trigonocephalum, Strongyloides papillosus, S. ransomi, Oesophagostomum radiatum, O. dentatum, O. columbianum, O. quadrispinulatum, Trichuris* spp., and the like. Other parasites include: hookworms (e.g., *Ancylostoma caninum, A. tubaeforme, A. braziliense, Uncinaria stenocephala);* lungworms (e.g., *Dictyocaulus viviparus* and *Metastrongylus* spp); eyeworms (e.g., *Thelazia* spp.); parasitic stage grubs (e.g., *Hypoderma bovis, H. lineatum, Dermatobia hominis*); kidneyworms (e.g., *Stephanurus dentatus*); screw worm (e.g., *Cochliomyia hominivorax* (larvae); filarial nematodes of the super-family Filarioidea and the Onchocercidae Family. Non-limiting examples of filarial nematodes within the Onchocercidae Family include the genus *Brugia* spp. (i.e., *B. malayi, B. pahangi, B. timori,* and the like), *Wuchereria* spp. (i.e., *W. bancrofti,* and the like), *Dirofilaria* spp. (*D. immitis, D. repens, D. ursi, D. tenuis, D. spectans, D. lutrae,* and the like), *Dipetalonema* spp. (i.e., *D reconditum, D. repens,* and the like), *Onchocerca* spp. (i.e., *O. gibsoni, O. gutturosa, O. volvulus,* and the like), *Elaeophora* spp. (*E. bohmi, E. elaphi, E. poeli, E. sagitta, E. schneideri,* and the like), *Mansonella* spp. (i.e., *M. ozzardi, M. perstans,* and the like), and *Loa* spp. (i.e., *L. loa*)*.*

The compositions the present disclosure can be used for the treatment and/or control, in particular helminths, in which the endoparasitic nematodes and trematodes may be the cause of serious diseases of mammals and poultry. Typical nematodes of this indication are: *Filariidae, Setariidae, Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris,Ancylostoma, Uncinaria, Toxascaris* and *Parascaris.* The trematodes include, in particular, the family of *Fasciolideae,* especially *Fasciola hepatica.*

Certain parasites of the species *Nematodirus, Cooperia* and *Oesophagostonum* infest the intestinal tract of the host animal, while others of the species *Haemonchus* and *Ostertagia* are parasitic in the stomach and those of the species *Dictyocaulus* are parasitic in the lung tissue. Parasites of the families and may be found in the internal cell tissue and in the organs, *e.g.* the heart, the blood vessels, the lymph vessels and the subcutaneous tissue. A particularly notable parasite is the heartworm of the dog, *Dirofilaria iminitis.*

The parasites which may be treated and/or controlled by the compositions of the present disclosure include those from the class of *Cestoda* (tapeworms), *e.g.* the families *Mesocestoidae,* especially of the genus *Mesocestoides,* in particular *M. lineatus; Dipylidiidae,* especially *Dipylidium caninum, Joyeuxiella* spp., in particular *Joyeuxiella pasquali,* and *Diplopylidium* spp., and *Taeniidae,* especially *Taenia pisformis, Taenia cervi, Taenia ovis, Taeneia hydatigena, Taenia multiceps, Taenia taeniaeformis, Taenia serialis,* and *Echinococcus* spp., most particularly *Taneia hydatigena, Taenia ovis, Taenia multiceps, Taenia serialis; Echinococcus granulosus* and *Echinococcus multilocularis.*

Furthermore, the compositions of the present disclosure can be used for the treatment and/or control of human pathogenic parasites. Of these, typical representatives that appear in the digestive tract are those of the genus *Ancylostoma, Necator, Ascaris, Strongyloides, Trichinella, Capillaria, Trichuris* and *Enterobius.* The compositions of the present disclosure are also suitable against parasites of the genus *Wuchereria, Brugia, Onchocerca* and *Loa* from the family of *Dracunculus* and parasites of the genus *Strongyloides* and *Trichinella,* which infect the gastrointestinal tract in particular.

A particular parasite to be treated and/or and controlled by the compositions of the disclosure is the heartworm (*Dirofilaria immitis*). Particular subjects for such treatment are dogs and cats.

The compositions of the present disclosure can be used for the treatment and/or control of against tick infestations (*Ixodes hexagonus, Ixodes ricinus* and *Rhipicephalus sanguineus, Dermacentor reticulatus*)*,* for the treatment and/or control of flea infestations (*Ctenocephalides felis* and *Ctenocephalides canis*),

The compositions of the present disclosure can be used for the treatment and/or control of adult fleas and prevention of new flea infestations (*Ctenocephalides felis*): the treatment and/or control of tick infestations [*Dermacentor variabilis* (American dog tick), *Ixodes scapularis* (black-legged tick), *Rhipicephalus sanguineus* (brown dog tick), *Amblyomma americanum* (lone star tick), *Dermacentor reticulatus, Ixodes ricinus* and *Ixodes hexagonus];* the treatment and/or control nematodes: heartworm disease (*Dirofilaria immitis*), angiostrongylosis by reducing the level of infection with immature adult (L5) *Angiostrongylus vasorum,* adult hookworm (*Ancylostoma caninum*), adult roundworm (*Toxocara canis Toxascaris leonina*) and adult whipworm (*Trichuris vulpis*) infections, and cestodes (*Dipylidium caninum, Taenia pisiformis, Echinococcus multilocularis,* and *Echinococcus granulosus*) infections in dogs 8 weeks of age and older and 4.4 pounds (2 kg) of body weight or greater.

The present disclosure provides a method for treating parasites, comprising: administering to a subject in need thereof a chewable composition including an effective amount of an active ingredient and sodium bicarbonate, the method optionally further comprising an effective amount of at least one additional active compound.

The present disclosure provides a method for controlling parasites, comprising: administering to a subject in need thereof a chewable composition including an effective amount of an active ingredient and sodium bicarbonate, the method optionally further comprising an effective amount of at least one additional active compound.

The present disclosure provides the manufacture of a chewable composition for treating parasites, the compositiong including an active ingredient and sodium bicarbonate. In certain embodiments, the present disclosure provides the manufacture of a chewable composition for controlling parasites, the composition including an active ingredient and sodium bicarbonate.

The present disclosure provides a chewable composition of the invention for use in therapy. The present disclosure provides a chewable composition of the invention comprising an effective amount of one or more agents which are active against parasites, for use in controlling parasites in or on an animal. The present disclosure further provides a chewable composition of the invention comprising an effective amount of one or more agents which are active against parasites, for use in preventing and/or treating diseases transmitted by parasites.

The present disclosure provides the use of a chewable composition of the invention comprising an effective amount of one or more agents which are active against parasites for the manufacture of a medicament for controlling parasites in or on an animal. The present disclosure further provides the use of a chewable composition of the invention comprising an effective amount of one or more agents which are active against parasites for the manufacture of a medicament for preventing and/or treating diseases transmitted by parasites.

The present disclosure provides a method of controlling parasites in or on an animal in need thereof comprising administering a chewable composition of the disclosure comprising an effective amount of one or more agents which are active against parasites. The present disclosure further provides a method of preventing and/or treating diseases transmitted by parasites in an animal in need thereof comprising administering a chewable composition of the disclosure comprising an effective amount of one or more agents which are active against parasites.

The present disclosure provides the use of a chewable composition of the disclosure comprising an effective amount of one or more agents which are active against parasites, in controlling parasites in or on an animal.

The compositions of the disclosure may be combined with one or more other active compounds or therapies for the treatment of one or more disorders, diseases or conditions, including the treatment of parasites. The compositions of the present disclosure may be administered simultaneously, sequentially or separately in combination with one or more compounds or therapies for treating parasites or other disorders.

### ITEMS

The present invention will now be described with reference to the following Items.
1. A chewable veterinary composition, comprising:
   an active pharmaceutical ingredient; and
   a carbonate compound.
2. The composition of Item 1, wherein the carbonate compound is sodium bicarbonate.
3. The composition of Item 1 or 2, comprising 0.5% to 40.0% (wt/wt) sodium bicarbonate.
4. The composition of Items 2 or 3, comprising 1.0% to 4.0% (wt/wt) sodium bicarbonate.
5. The composition of any one of Items 1-4, comprising an isoxazoline class parasiticide as an active pharmaceutical ingredient.
6. The composition of any one of Items 1-5, comprising lotilaner, fluralaner, afoxalaner, or sarolaner.
7. The composition of any one of Items 1-6, comprising a macrocyclic lactone class parasiticide.
8. The composition of any one of Items 1-7, comprising moxidectin or milbemycin oxime.
9. The composition of any one of Items 1-8, comprising praziquantel.
10. The composition of any one of Items 1-9, comprising pyrantel pamoate.
11. The composition of any one of Items 1-10, comprising monepantel.
12. The composition of any one of Items 1-11, wherein the average particle size of the carbonate compound, preferably sodium bicarbonate, is 10-250 µm, 20-200 µm, 30-150 µm, or 40-100 µm.
13. The composition of any one of Items 1-12, wherein the average particle size of the carbonate compound, preferably sodium bicarbonate, is 30-150 µm.
14. The composition of any one of Items 1-13, wherein the average particle size of the carbonate compound, preferably sodium bicarbonate, is 40-100 µm.
15. The composition of any one of Items 9-14, wherein the praziquantel is incorporated into the dosage form as a praziquantel-coated pellet.
16. The composition of Item 15, wherein the the praziquantel-coated pellet comprises (i) a pellet substrate (e.g., comprised of microcrystalline cellulose), (ii) a first coating over at least a portion of the pellet, said first coating comprising praziquantel, and optionally (iii) a second coating over at least a portion of the first coating, said second coating comprising a physiologically acceptable polymer (e.g., dimethylaminoethyl methacrylate-copolymer).
17. A chewable veterinary composition, comprising:
   0.5% to 25% (wt/wt) of an isoxazoline (e.g., lotilaner, sarolaner, fluralaner, or afoxolaner);
   0.005% to 1% (wt/wt) moxidectin;
   1% to 10% (wt/wt) praziquantel;
   5% to 30% (wt/wt) pyrantel pamoate; and
   1.0% to 10.0% (wt/wt), preferably 1.0% to 5.0% (wt/wt), more preferably 4.0% (wt/wt) sodium bicarbonate.

### 6. Examples

The present invention has multiple aspects, illustrated by the following non-limiting examples.

### Example 1

Table 1 shows seven chewable formulations (LS7_01, 02, 04, 05, 06, 07, and 08) containing lotilaner, praziquantel, and milbemycine oxime as actives, and sodium bicarbonate as a disintegrant, and one reference formulation (LS_03) lacking sodium bicarbonate. Sodium bicarbonate was added at levels of 0.5% to 4.0%. The formulations were assessed for the effective level of sodium bicarbonate to affect suitable drug release out of the formulations.

**Table 1. Chewable Formulations with Sodium Bicarbonate**

| | *LS7_01* | *LS7_02* | *LS7_03* | *LS7_04* | *LS7_05* | *LS7_06* | *LS7_07* | *LS7_08* |
|---|---|---|---|---|---|---|---|---|
| *Ingredient* | *Conc % (wt*/*wt)* | *Conc % (wt*/*wt)* | *Conc % (wt*/*wt)* | *Conc % (wt*/*wt)* | *Conc % (wt*/*wt)* | *Conc % (wt*/*wt)* | *Conc % (wt*/*wt)* | *Conc % (wt*/*wt)* |
| *(Function)* | | | | | | | | |
| Lotilaner form G micron | 15.000% | 15.000% | 15.000% | 15.000% | 15.000% | 15.000% | 15.000% | 15.000% |
| (API) | | | | | | | | |
| Praziquantel micronized | 3.750% | 3.750% | 3.750% | 3.750% | 3.750% | 3.750% | 3.750% | 3.750% |
| (API) | | | | | | | | |
| Milbemycin oxime | 0.563% | 0.563% | 0.563% | 0.563% | 0.563% | 0.563% | 0.563% | 0.563% |
| (API) | | | | | | | | |
| Sodium Bicarbonate | 2.000% | 1.000% | 0.000% | 0.500% | 3.000% | 1.500% | 4.000% | 4.000% |
| (Disintegrant) | | | | | | | | |
| Sodium chloride Ph. Eur. | 1.500% | 1.500% | 1.500% | 1.500% | 1.500% | 1.500% | 1.500% | 1.500% |
| (Taste adjuvant) | | | | | | | | |
| Symtripal Liver type Flavor | 8.000% | 8.000% | 8.000% | 8.000% | 8.000% | 8.000% | 8.000% | 8.000% |
| (Flavor) | | | | | | | | |
| Pala Enhancer (D'TECH 8P0910748 VEGGIE) | 10.000% | 10.000% | 10.000% | 10.000% | 10.000% | 10.000% | 10.000% | 10.000% |
| (Flavor) | | | | | | | | |
| Pigment Blend PB-165001 | 0.100% | 0.100% | 0.100% | 0.100% | 0.100% | 0.100% | 0.100% | 0.100% |
| (Colorant) | | | | | | | | |
| Confectioners Sugar 10X NF | 5.000% | 5.000% | 5.000% | 5.000% | 5.000% | 5.000% | 5.000% | 5.000% |
| (Filler) | | | | | | | | |
| Sodium Lauryl Sulfate (SLS / SDS) | 1.000% | 1.000% | 1.000% | 1.000% | 1.000% | 1.000% | 1.000% | 1.000% |
| (Wetting agent) | | | | | | | | |
| Fat Powder (Vana-Grasa 75C) | 4.500% | 4.500% | 4.500% | 4.500% | 4.500% | 4.500% | 4.500% | 4.500% |
| (Taste adjuvant) | | | | | | | | |
| Waxy Maize Starch (Amioca Powder TF) | 26.423% | 27.019% | 27.615% | 27.317% | 25.827% | 26.721% | 25.231% | 24.040% |
| (Binder) | | | | | | | | |
| Waxy Pregelatinized Starch (Ultra Sperse A) | 3.800% | 3.800% | 3.800% | 3.800% | 3.800% | 3.800% | 3.800% | 3.800% |
| (Binder) | | | | | | | | |
| *Total Solids* | 81.636% | 81.232% | 80.828% | 81.030% | 82.040% | 81.434% | 82.444% | 81.253% |
| BHA | 0.100% | 0.100% | 0.100% | 0.100% | 0.100% | 0.100% | 0.100% | 0.100% |
| (Antioxidant) | | | | | | | | |
| Proylene Glycol | 0.345% | 0.345% | 0.345% | 0.345% | 0.345% | 0.345% | 0.345% | 0.345% |
| (Solvent) | | | | | | | | |
| *Total Antioxidants* | 0.445% | 0.445% | 0.445% | 0.445% | 0.445% | 0.445% | 0.445% | 0.445% |
| Glycerol Anhydrous > 98% | 17.919% | 18.323% | 18.727% | 18.525% | 17.515% | 18.121% | 17.111% | 16.302% |
| (Softener) | | | | | | | | |
| Purified Water | 17.919% | 18.323% | 18.727% | 18.525% | 17.515% | 18.121% | 17.111% | 16.302% |
| (Softener) | | | | | | | | |
| *Total Liquids* | 18.364% | 18.768% | 19.172% | 18.970% | 17.960% | 18.556% | 17.556% | 18.747% |
| *Total Solids + Liquids* | 100.000% | 100.000% | 100.000% | 100.000% | 100.000% | 100.000% | 100.000% | 100.000% |
| | | | | | | | | |
| Texture after 24h [N] | 88.46 | 89.85 | 87.09 | 73.51 | 84.75 | 78.79 | 92.11 | 51.96 |
| Texture after 1mo 25°C [N] | 118.11 | 84.28 | 93.71 | 71.28 | 84.12 | 109.36 | 93.82 | 42.52 |
| Texture after 1mo 30°C [N] | 83.29 | 82.61 | 81.73 | 77.02 | 74.25 | 103.84 | 89.70 | 44.11 |
| Texture after 1mo 40°C [N] | 102.17 | 83.38 | 83.97 | 70.62 | 70.78 | 89.42 | 93.14 | 48.70 |
| Texture after 3mo 25°C [N] | 118.25 | 119.67 | 99.88 | 88.46 | 96.91 | 91.08 | 95.22 | 48.72 |
| Texture after 3mo 40°C [N] | 92.16 | 103.53 | 98.59 | 84.37 | 90.50 | 112.33 | 90.56 | 46.52 |
| Texture after 6mo 25°C [N] | 100.46 | 91.54 | 87.29 | 77.06 | 84.03 | 93.81 | 75.53 | 46.02 |
| Disintegration after 24h [H2O] [min] | 14:21 | 12:26 | 14:43 | 14:26 | 13:24 | 15:10 | 15:18 | 13:30 |
| Disintegration after 1mo 25°C [H2O] [min] | 15:02 | 16:14 | 14:22 | 12:02 | 12:35 | 17:19 | 15:18 | 15:22 |
| Disintegration after 1mo 30°C [H2O] [min] | 17:37 | 17:40 | 14:51 | 15:49 | 12:48 | 16:29 | 14:14 | 17:02 |
| Disintegration after 1mo 40°C [H2O] [min] | 17:03 | 14:46 | 17:58 | 14:06 | 13:32 | 16:42 | 17:36 | 16:04 |
| Disintegration after 3mo 25°C [H2O] [min] | 18:36 | 17:43 | 15:18 | 14:38 | 14:22 | 13:06 | 15:27 | 15:47 |
| Disintegration after 3mo 40°C [H2O] [min] | 17:05 | 17:26 | 19:01 | 17:24 | 14:22 | 18:43 | 16:22 | 17:52 |
| Disintegration after 6mo 25°C [H2O] [min] | 15:02 | 21:36 | 18:13 | 15:08 | 18:55 | 18:49 | 16:32 | 16:04 |
| Disintegration after 6mo 40°C [H2O] [min] | 20:38 | 19:31 | 21:26 | 18:54 | 19:46 | 20:22 | 17:48 | 19:50 |
| pH after 24h | 7.43 | 7.19 | 6.89 | 7.18 | 7.73 | 7.38 | 7.73 | 8.03 |
| pH after 1mo 25°C | 7.80 | 7.47 | 6.84 | 7.15 | 7.99 | 7.61 | 8.03 | 8.34 |
| pH after 1mo 30°C | 7.87 | 7.44 | 6.77 | 7.14 | 8.10 | 7.63 | 8.10 | 8.25 |
| pH after 1mo 40°C | 8.09 | 7.35 | 6.56 | 6.94 | 8.36 | 7.61 | 8.31 | 8.42 |
| pH after 3mo 25°C | 7.71 | 7.34 | 6.70 | 7.12 | 7.98 | 7.59 | 8.00 | 8.08 |
| pH after 3mo 40°C | 8.22 | 7.34 | 6.40 | 6.87 | 8.38 | 7.81 | 8.40 | 8.70 |
| pH after 6mo 25°C | 7.77 | 7.34 | 6.61 | 7.02 | 7.99 | 7.62 | 8.06 | 8.16 |
| pH after 6mo 40°C | 8.27 | 7.04 | 6.16 | 6.65 | 8.49 | 7.61 | 8.40 | 8.80 |

All eight formulations were prepared according to the following procedure. Powder materials were weighed and passed through a 1000 µm sieve. The powders were poured into a glass bottle and mixed for 15 minutes with a Turbula blender. The powder blends were poured into the bowl of a Hobart planetary mixer. Liquid ingredients were weighed and prepared into a liquid solution. Under stirring, the liquid was added to the powder blend for granulation. The final granulated blend was transferred to a Gabler Extruder DE40 and extruded through a 13mm round die. Extrudates were cut into single units, packed and stored at defined conditions.

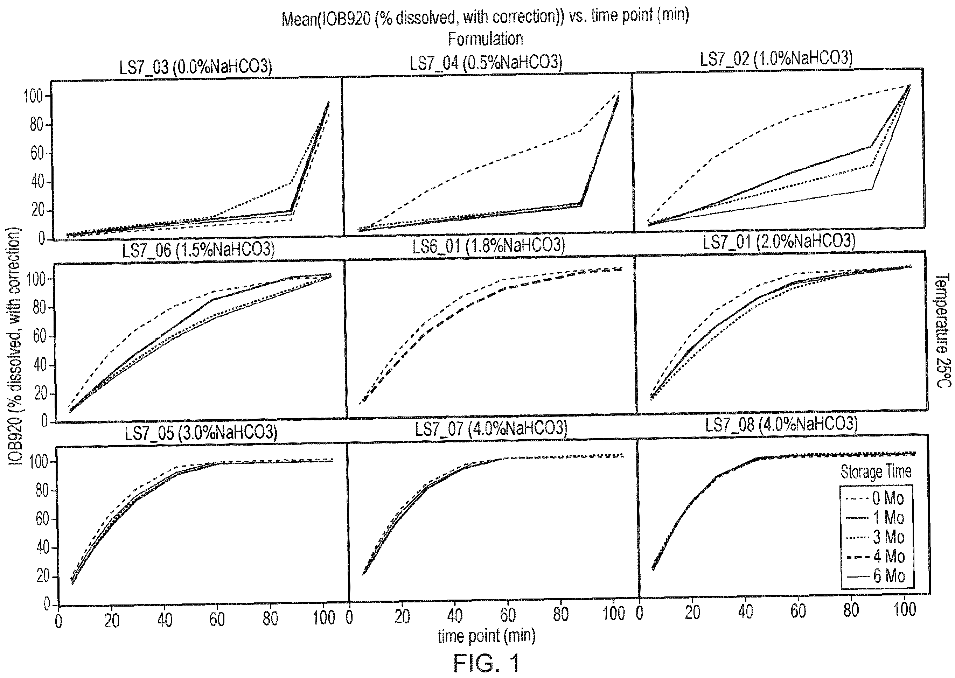

All eight formulations were evaluated according to the dissolution procedure described in Table 2. Drug release of all three active ingredients (lotilaner, praziquantel, milbemycin oxime) was significantly impacted by the level of sodium bicarbonate in the chewable formulation. As shown in FIG. 1, higher sodium bicarbonate content in the formulation led to faster drug release. Above 3.0%, no further increase was detectable. In the presence of 3% sodium bicarbonate, around 80% of the active ingredients were released after 30 minutes.

**Table 2. Dissolution Testing of Chewable Formulations**

| Apparatus | | Paddle method in accordance with USP <711>, "Dissolution" and Ph.Eur. 2.9.3, "Dissolution Test for solid Dosage Forms". | |
|---|---|---|---|
| Conditions | | | |
| | Temp of test medium | 37°C ± 0.5°C | |
| | Number of chewies tested | 1 chewable formulation per vessel | |
| | Volume of test medium | 1000 mL | |
| | Test medium | 1.0% CTAB in 0.1 N HCl | |
| | Sampling Time | Chewable formulation w NaHCO₃ | Chewable formulation w/o NaHCO₃ |
| | | 5, 10, 15, 20, 30, 45, 60, 90 min *+* 15 min Infinity (250 rpm) | 15, 30, 45, 60, 90, 120 min *+* 15 min Infinity (250 rpm) |
| | Speed of rotation | ω = 50 rpm | ω = 50 rpm |
| Test Solution | | | |
| | Sample volume | An aliquot of 5 mL from each vessel is withdrawn directly into a syringe. Remove the cannula and attach a syringe filter. Discard the first two millilitres of filtrate and use the following filtrate for injection. Dissolution medium will not be replaced with fresh medium after sampling. Medium loss is adapted by calculation if a dissolution profile is performed. | |
| | Filter type | Filter with a membrane PVDF and pore size 0.45 µm (e.g. Millex-HV). | |
| Dissolution Analysis | | Concentration of active ingredient in the test medium is determined by HPLC. | |

### Example 2

The formulations of Table 3 (LS_01-08) were assessed in a lab stability study. Samples were packed into sealed Aluminium pouches and were stored at controlled conditions at 25°C/60%rH and 40°C/75%rH. Dissolution profiles were evaluated according to the procedure of Table 2 at time points of 1, 3, 4, and 6 months. Formulations including 3% or 4% sodium bicarbonate showed stable, unchanged dissolution profiles over time at all storage conditions. The disintegration effect of sodium bicarbonate is effective in aged formulations, stored at accelerated conditions for 6 months at a sodium bicarbonate level of 3% or higher.

**Table 3. Chewable Formulations with Sodium Bicarbonate**

| | *LS8_01* | *LS8_02* | *LS8_03* | *LS8_04* |
|---|---|---|---|---|
| *Ingredient* | *Conc % (wt*/*wt)* | *Conc % (wt*/*wt)* | *Conc % (wt*/*wt)* | *Conc % (wt*/*wt)* |
| *(Function)* | | | | |
| Lotilaner (IOB920) form G micron | 15.000% | 15.000% | 15.000% | 15.000% |
| (API) | | | | |
| Praziquantel micronized | 3.750% | 3.750% | 3.750% | 3.750% |
| (API) | | | | |
| Milbemycin oxime | 0.563% | 0.563% | 0.563% | 0.563% |
| (API) | | | | |
| Sodium Bicarbonate | 4.000% | 3.000% | 1.000% | 0.000% |
| (Disintegrant) | | | | |
| Sodium chloride Ph. Eur. | 1.500% | 1.500% | 1.500% | 1.500% |
| (Taste adjuvant) | | | | |
| Symtripal Meat Chopped Flavor (Symrise 366202) | 15.000% | 15.000% | 15.000% | 15.000% |
| (Flavor) | | | | |
| Pigment Blend PB-165001 | 0.100% | 0.100% | 0.100% | 0.100% |
| (Colarant) | | | | |
| Confectioners Sugar 10X NF | 5.000% | 5.000% | 5.000% | 5.000% |
| (Filler) | | | | |
| Sodium Lauryl Sulfate (SLS / SDS) | 1.000% | 1.000% | 1.000% | 1.000% |
| (Wetting Agent) | | | | |
| Fat Powder (Vana-Grasa 75C) | 4.500% | 4.500% | 4.500% | 4.500% |
| (Taste adjuvant) | | | | |
| Waxy Maize Starch (Amioca Powder TF) | 27.019% | 27.615% | 28.807% | 29.403% |
| (Binder) | | | | |
| Waxy Pregelatinized Starch (Ultra-Sperse A) | 3.800% | 3.800% | 3.800% | 3.800% |
| (Binder) | | | | |
| *Total Solids* | 81.232% | 80.828% | 80.020% | 79.616% |
| BHA | 0.100% | 0.100% | 0.100% | 0.100% |
| (Antioxidant) | | | | |
| Proylene Glycol | 0.345% | 0.345% | 0.345% | 0.345% |
| (Solvent) | | | | |
| *Total Antioxidants* | 0.445% | 0.445% | 0.445% | 0.445% |
| Glycerol Anhydrous > 98% | 18.323% | 18.727% | 19.535% | 19.939% |
| (Softener) | | | | |
| Purified water | 0.000% | 0.000% | 0.000% | 0.000% |
| (Softener) | | | | |
| *Total Liquids* | 18.768% | 19.172% | 19.980% | 20.384% |
| *Total Solids + Liquids* | 100.000% | 100.000% | 100.000% | 100.000% |

| *Properties* | | | | |
|---|---|---|---|---|
| Texture after 24 hours (Newtons) | 9.21 | 10.30 | 9.09 | 9.74 |
| Disintegration after 24 hours [H2O] (Minutes) | 14:18 | 13:43 | 13:06 | 14:31 |
| pH after 24 hours | 8.05 | 7.93 | 7.21 | 4.15 |

All four formulations were prepared according to the following procedure. All powder materials were weighed and passed through a 1000 µm sieve. The powders were poured into a glass bottle and mixed for 15 minutes with a Turbula blender. The powder blends were poured into the bowl of a Hobart planetary mixer. Liquid ingredients were weighed and prepared into a liquid solution. Under stirring, the liquid was added to the powder blend for granulation. The final granulated blend was transferred to a Gabler Extruder DE40 and extruded through a 13mm round die. Extrudates were cut into single units, packed and stored at defined conditions.

As evaluated according to Table 2, drug release of all three active ingredients was significantly impacted by the level of sodium bicarbonate in the chewable formulation. Higher sodium bicarbonate content in the formulation led to faster drug release. Above 3.0% no further increase was detectable. In the presence of 3% of sodium bicarbonate, around 80% of the active ingredients was released after 30 minutes.

### Example 3

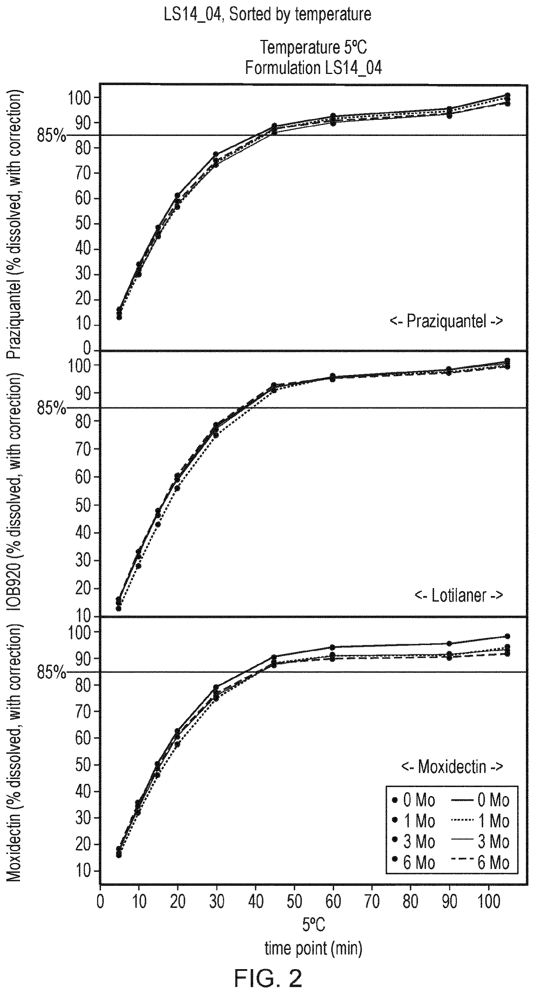

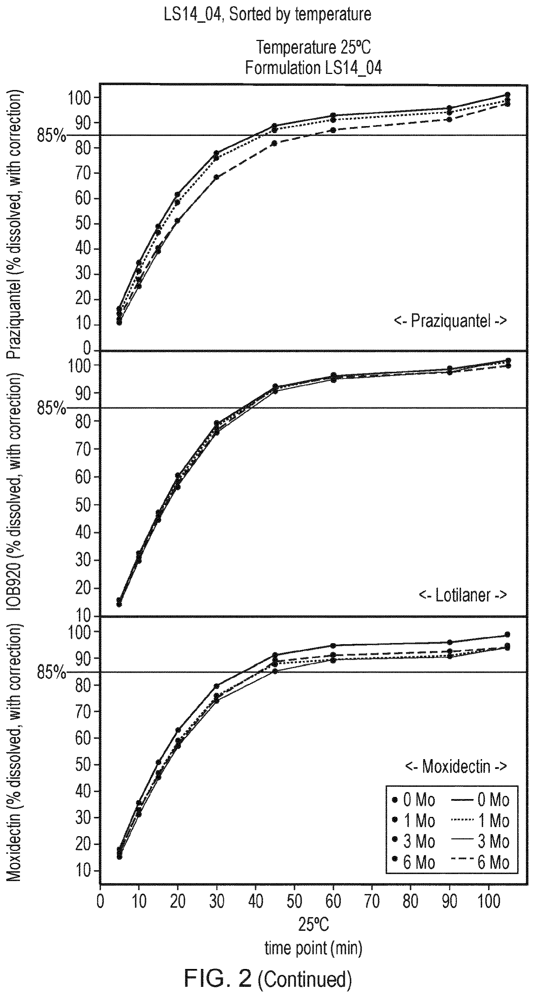

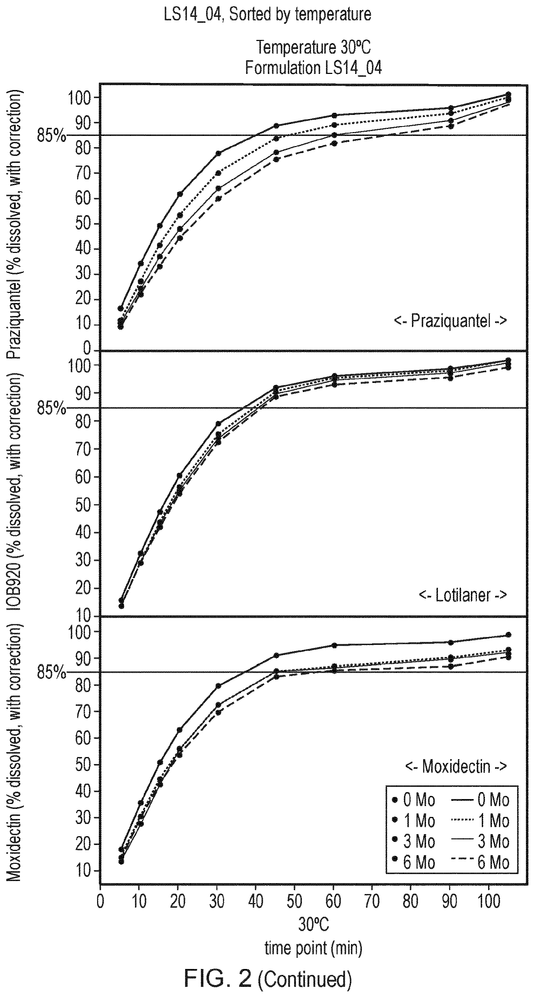

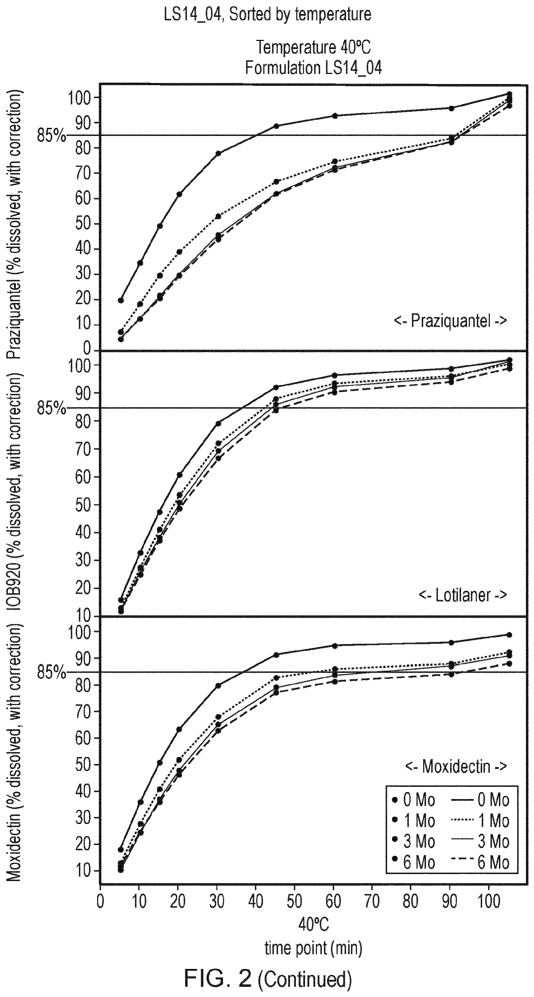

Table 4 shows chewable formulations containing lotilaner, moxidectin, and praziquantel as actives, and sodium bicarbonate as a disintegrant. Praziquantel is present in LS14_04 via a double-coated pellet (praziquantel-coated microcrystalline cellulose substrate, with a Eudragit^{®} EPO taste-mask secondary coating). Drug release of active ingredient was adversely impacted in LS14_03 including the sodium bicarbonate at the larger particle size fraction of 200-400 µm. FIG. 2 shows that LS14_04 exhibits rapid dissolution of all active ingredients.

**Table 4. Chewable Formulations with Sodium Bicarbonate**

| | *LS14_01* | *LS14_02* | *LS14_03* | *LS14_04* |
|---|---|---|---|---|
| | *NaHCO₃ particle size* | *NaHCO₃ particle size* | *NaHCO₃ particle size* | *NaHCO₃ particle size* |
| | *40-100µm* | *40-100µm* | *200-400µm* | *40-100µm* |
| *Ingredient* | *Conc % (wt*/*wt)* | *Conc % (wt*/*wt)* | *Conc % (wt*/*wt)* | 15.000% |
| Lotilaner | 15.000% | 15.000% | 15.000% | 0.000% |
| Praziquantel (CAS 55268-74-1) | 0.000% | 0.000% | 0.000% | 0.015% |
| Moxidectin (CAS 113507-06-5) | 0.015% | 0.015% | 0.015% | 4.000% |
| Sodium Bicarbonate (CS 144-55-8) | 4.000% | 4.000% | 4.000% | |
| Rebaten (91722-21-3) | 0.000% | 0.000% | 0.000% | 1.500% |
| Sodium chloride Ph. Eur. (CAS 7440-23-5) | 1.500% | 1.500% | 1.500% | 8.000% |
| Symtripal Liver Type | 8.000% | 8.000% | 8.000% | |
| Pala Enhancer (D'TECH 8P0910748 VEGGIE) | 0.000% | 0.000% | 0.000% | 0.100% |
| Pigment Blend PB-165001 | 0.100% | 0.100% | 0.100% | 5.000% |
| Confectioners Sugar 10X NF | 5.000% | 5.000% | 5.000% | 1.000% |
| Sodium Lauryl Sulfate (SLS / SDS) | 1.000% | 1.000% | 1.000% | 4.500% |
| Fat Powder (Vana_Grasa 75C) | 4.500% | 4.500% | 4.500% | 2.7500% |
| Cellets 175 or Cellet 200 | 16.000% | 16.000% | 16.000% | |
| Praziquantel TM Pellets containing ~ 30 wt% praziquantel | | | | 13.2500% |
| *Waxy Maize Starch (Amioca Powder TF)* | 23.640% | 23.640% | 23.640% | 23.640% |
| Waxy Pregelatinized Starch (Ultra-Sperse A) | 3.800% | 3.800% | 3.800% | 3.800% |
| *Total Solids* | *82.555%* | *82.555%* | *82.555%* | *82.555%* |
| BHA (CAS 121-00-6) | 0.100% | 0.100% | 0.100% | 0.100% |
| Propyleneglycol (CAS57-55-6) | 0.345% | 0.345% | 0.345% | 0.345% |
| *Total Antioxidants* | *0.445%* | *0.445%* | *0.445%* | *0.445%* |
| Glycerol Anhydrous > 98% (CAS 56-81-5) | 17.000% | 17.000% | 17.000% | *17.000%* |
| Purified water (CAS 7732-18-5) | 0.000% | 0.000% | 0.000% | |
| *Total Liquids* | *17.445%* | *17.445%* | *17.445%* | *17.445%* |
| *Total Solids + Liquids* | 100.000% | 100.000% | 100.000% | 100.000% |

### Example 4

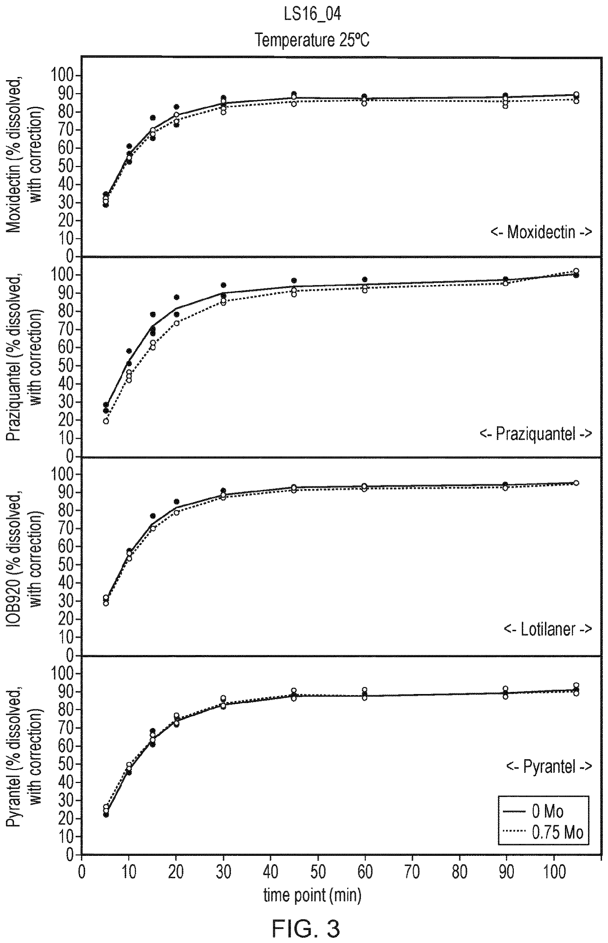

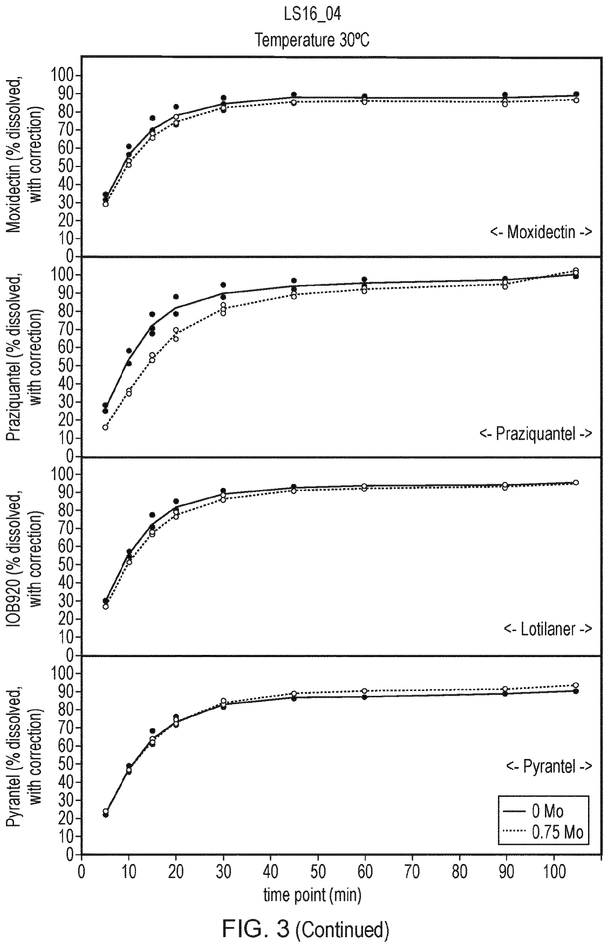

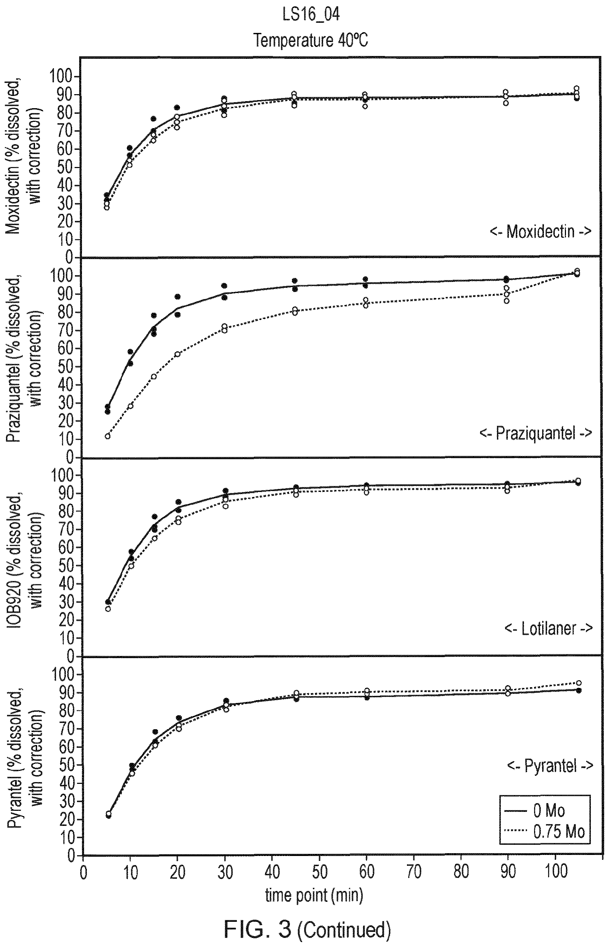

Table 5 shows a chewable formulation containing lotilaner, moxidectin, praziquantel taste-masked pellets, and pyrantel pamoate as actives, and sodium bicarbonate as a disintegrant. Sodium bicarbonate was added at a level of 4.0%. The formulation was assessed for disintegration and dissolution performance. FIG. 3 shows that LS16_04 exhibits rapid dissolution of all active ingredients.

**Table 5. Chewable Formulations with Sodium Bicarbonate**

| | *LS16_04* |
|---|---|
| *Ingredient* | *Conc % (wt*/*wt)* |
| Lotilaner | 15.00% |
| Moxidectin (CAS 113507-06-5) | 0.015% |
| Pyrantel pamoate (dose*: 5mg/kg) | 10.81% |
| Praziquantel (CAS 55268-74-1) | |
| Praziquantel TM Pellets containing ~ 30wt% praziquantel | 12.67% |
| Cellets 200 | 1.33% |
| Sodium Bicarbonate (CAS 144-55-8) | 4.00% |
| Sodium chloride Ph. Eur. (CAS 7440-23-5) | 1.50% |
| Symtripal Liver Type | 8.00% |
| Pala Enhancer (D'TECH 8P0910748 VEGGIE) | 2.00% |
| Pigment Blend PB-165001 | 0.10% |
| Confectioners Sugar 10X NF | 5.00% |
| Sodium Lauryl Sulfate (SLS / SDS) | 1.00% |
| Fat Powder (Vana_Grasa 75C) | 4.50% |
| Waxy Maize Starch (Amioca Powder TF) | 19.08% |
| *Total Solids* | 85.00% |
| BHA (CAS 121-00-6) | |
| Propyleneglycol (CAS57-55-6) | |
| *Total Antioxidants* | 0.00% |
| Glycerol Anhydrous > 98% (CAS 56-81-5) | 15.00% |
| Purified water (CAS 7732-18-5) | |
| *Total Liquids* | 15.00% |
| *Total Solids + Liquids* | 100.00% |

### Example 5

Table 6 shows a chewable formulation containing lotilaner, moxidectin, praziquantel taste-masked pellets, and monepantel as actives, and sodium bicarbonate as a disintegrant. Sodium bicarbonate was added at a level of 4.0%.

**Table 6. Chewable Formulations with Sodium Bicarbonate**

| | *LS18_01* |
|---|---|
| *Ingredient* | *Conc % (wt*/*wt)* |
| Lotilaner (IOB920) form G micron | 15.0000 % |
| Praziquantel TM Pellets | 13.1670 % |
| Moxidectin (CAS 113507-06-5) | 0.0150 % |
| Monepantel cristaline (Monepantel B) | 3.8000 % |
| Sodium Bicarbonate (CS 144-55-8) | 4.0000 % |
| Sodium chloride Ph. Eur. (CAS 7440-23-5) | 1.5000 % |
| Symtripal Liver Type | 8.0000 % |
| Pala Enhancer (D'TECH 8P0910748 VEGGIE) | 2.0000 % |
| Pigment Blend PB-165001 | 0.1000 % |
| Confectioners Sugar 10X NF | 5.0000 % |
| Sodium Lauryl Sulfate (SLS / SDS) | 1.0000 % |
| Fat Powder (Vana_Grasa 75C) | 4.5000 % |
| Cellets 200 | 0.8330 % |
| Waxy Maize Starch q.s. (Amioca Powder TF) | 26.0850% |
| *Total Solids* | 85.000% |
| BHA (CAS 121-00-6) | |
| Propyleneglycol (CAS57-55-6) | |
| *Total Antioxidants* | 0.0000% |
| Glycerol Anhydrous > 98% (CAS 56-81-5) | 15.0000% |
| Purified water (CAS 7732-18-5) | |
| *Total Liquids* | 15.0000% |
| *Total Solids + Liquids* | 100.0000% |

It is understood that the foregoing detailed description and accompanying examples are merely illustrative and are not to be taken as limitations upon the scope of the invention, which is defined solely by the appended claims and their equivalents.

Various changes and modifications to the disclosed embodiments will be apparent to those skilled in the art. Such changes and modifications, including without limitation those relating to the chemical structures, substituents, derivatives, intermediates, syntheses, compositions, formulations, or methods of use of the invention, may be made without departing from the spirit and scope thereof.

## Claims

1. A soft chewable veterinary dosage form, comprising:
0.5% to 25% (wt/wt) of an isoxazoline, such as lotilaner, sarolaner, fluralaner, or afoxolaner;
0.005% to 1% (wt/wt) of a macrocyclic lactone, such as moxidectin, milbemycin oxime, doramectin, or selamectin;
1% to 10% (wt/wt) of a pyrazinoisoquinoline, such as praziquantel; and
0.5% to 4.0% (wt/wt), preferably 1.0% to 4.0% (wt/wt), more preferably 2.0% to
4.0% (wt/wt) sodium bicarbonate.

2. The dosage form of claim 1, further comprising 5% to 30% (wt/wt) pyrantel pamoate.

3. The dosage form of claim 1 or 2, wherein the sodium bicarbonate has an average particle size of 10-250 µm, 20-200 µm, 30-150 µm, or 40-100 µm, as measured by sieves.

4. The dosage form of any one of claims 1-3, wherein the dosage form has a texture, measured as the peak force necessary for a sphere with a diameter of 8 mm with a velocity of 1 mm/s to penetrate 2mm into the composition, of between 5 and 40 N at room temperature at 1 hour post-extrusion.

5. The dosage form of any one of claims 1-4, wherein at least 70% of the isoxazoline, the macrocyclic lactone and the pyrazinoisoquinoline are released within 30 minutes of dissolution, as determined according to Ph.Eur. 2.9.3.

6. The dosage form of any one of claims 1-5, comprising
0.5% to 25% (wt/wt) of an isoxazoline, such as lotilaner, sarolaner, fluralaner, or afoxolaner;
0.005% to 1% (wt/wt) moxidectin;
1% to 10% (wt/wt) praziquantel; and
5% to 30% (wt/wt) pyrantel pamoate.

7. The dosage form of any one of claims 1-6, comprising
14-16% (wt/wt) lotilaner,
0.025% to 0.045% (wt/wt) moxidectin, and
3-5% (wt/wt) praziquantel.

8. The dosage form of any one of claims 1-5, comprising
0.5% to 25% (wt/wt) of an isoxazoline, such as lotilaner, sarolaner, fluralaner, or afoxolaner);
0.005% to 1% (wt/wt) milbemycin oxime;
1% to 10% (wt/wt) praziquantel; and
5% to 30% (wt/wt) pyrantel pamoate.

9. The dosage form of claim 8, comprising
15% (wt/wt) lotilaner;
0.563% (wt/wt) milbemycin oxime; and
3-5% (wt/wt) praziquantel.

10. The dosage form of any one of claims 1-9, comprising 1.0% to 4.0% (wt/wt) sodium bicarbonate.

11. The dosage form of any one of claims 1-10, wherein the praziquantel is incorporated into the dosage form as a praziquantel-coated pellet.

12. The dosage form of claim 11, wherein the praziquantel-coated pellet comprises (i) a pellet substrate, such as microcrystalline cellulose, (ii) a first coating over at least a portion of the pellet, said first coating comprising praziquantel, and optionally (iii) a second coating over at least a portion of the first coating, said second coating comprising a physiologically acceptable polymer, such as dimethylaminoethyl methacrylate-copolymer.

13. The dosage form of any one of claims 1-12, wherein the dosage form does not contain water.

14. The dosage form of any one of claims 1-13, wherein the sodium bicarbonate has an average particle size of 30-150 µm, as measured by sieves.

15. The dosage form of any one of claims 1-12, wherein the sodium bicarbonate has an average particle size of 40-100 µm, as measured by sieves.
